(19) Europäisches Patentamt / European Patent Office / Office européen des brevets

(11) **EP 2 862 596 A1**

(12) **EUROPEAN PATENT APPLICATION**

(43) Date of publication:
22.04.2015 Bulletin 2015/17

(51) Int Cl.:
*A61N 1/16* (2006.01)  *A61N 1/06* (2006.01)
*A61N 2/02* (2006.01)

(21) Application number: 14167813.6

(22) Date of filing: 10.05.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 14.05.2013  FR 1301105
19.06.2013  FR 1301426
06.09.2013  FR 1302077
02.10.2013  US 201361885659 P
02.10.2013  US 201361885790 P
10.10.2013  US 201361889065 P

(71) Applicant: **Lauer, Vincent**
**44240 La Chapelle sur Erdre (FR)**

(72) Inventor: **Lauer, Vincent**
**44240 La Chapelle sur Erdre (FR)**

Remarks:
Claims 16-177 are deemed to be abandoned due to
non-payment of the claims fees (Rule 45(3) EPC).

(54) **Device for treating diseases**

(57)    A method for telecommunicating without causing or worsening diseases, the method comprising administering a stay in a Faraday cage to a person exposed to electromagnetic waves and suffering from a disease.

Figure 1

EP 2 862 596 A1

**Description**

**Field of the invention**

The invention pertains to the field of devices for treating disease.

**prior art**

Georges Lakhovsky invented in 1923 a "radio cellulo oscillateur" emitting a single frequency with a wavelength between 2 and 10 m. He obtained some results in curing cancer by using this device, but also some failures. In 1928 he improved the device obtaining the "multiple waves oscillator". This was a spark gap transmitter equipped with an antenna having multiple resonant modes. He claimed that his device generated waves in the range of at least 750 kHz to 3 GHz. Submitting cancerous patients to these waves about twice a week, 15 minutes each time, he obtained recoveries. Georges Lakhovsky developed his own interpretation of why his devices could cure cancer, which was not in line with modern science. His multiple waves oscillator is described in patent number FR 732.276 and US 1,962,565 and his results are described in "Radiations and Waves, Sources of Our Life", published 1941, by G. Lakhovsky, editor Emile L Cabella, 288 East 45th Street, New York. The mainstream opinion is that there is uncertainty on whether his reported results in curing cancer were real and/or attributable to his multiple waves oscillator. This opinion is at least partly due to the absence of a reliable underlying explanation.

Roy Rife invented the "Beam Ray Machine" around 1930. This machine emitted light beam pulses and microwaves in the 20-35 MHz frequency range due to the frequencies supplied to the device to maintain the gas discharge in a "phanotron tube". There are reports that his machine could cure cancer. He also had his own understanding of how his machine worked, not in line with modern science.

ElectroHyperSensitivity (EHS) is a developing adverse health condition covering a number of different reactions to the presence of electromagnetic fields. A common health problem associated with electromagnetic fields is sleep disturbances. People affected with such sleep disturbances sometimes use Faraday cages to shield themselves from electromagnetic fields when sleeping. EHS is considered by health authorities as a purely psychological disease with no underlying physical cause.

**Description of the invention.**

Scientific knowledge underlying the present invention is described in:

US application number 61/889,065
HAL- 00877298 version 2 "A Quantum Theory of the Biological Effects of Radio-frequencies and its application to Cancer" by Vincent Lauer, November 2013, available on http://hal.archives-ouvertes.fr/hal-00877298.
HAL-009755963 "A model of the interaction of T lymphocytes with electromagnetic waves" by Vincent Lauer, April 9, 2014, available on http://hal.archives-ouvertes.fr/hal-00975963.

Aspects of the above documents which are essential for the present invention are described below. The invention is based on the finding that at low power, wideband electromagnetic waves cause inhibition of the recognition of a p-MHC (peptide and Major Histocompatibility Complex) presented by an APC (Antigen Presenting Cell) by a TCR (T Cell Receptor). Conditions for inhibition are roughly as follows:

- a bandwidth condition: the bandwidth of the artificial wave must be higher than a threshold which depends on the specific TCR-pMHC complex.
- a power condition: the power of the artificial wave must be higher than a threshold which depends on the specific TCR-pMHC complex.
- a frequency condition: the frequency of the artificial wave must be within a certain range which depends on the specific TCR-pMHC complex.

This inhibition is due to the following facts:

Recognition of an antigen by a TCR is characterized by the fact that the TCR-pMHC system makes a transition from a quantum well (a) to a quantum well (c) characterizing recognition of the antigen, through an intermediate quantum well (b). These transitions are normally stimulated by the thermal background of electromagnetic waves which determines the Rabi frequencies of the transitions. The presence of a wideband artificial electromagnetic wave at an appropriate frequency results in an increase of the Rabi frequency of the (a) to (b) transition whilst the Rabi frequency of the (b) to (c) transition remains un-modified. Basic quantum mechanical equations show that this results in a diminished probability of the (a) to (c) transition.

The bandwidth condition essentially results from the fact that all quantum states in well (a) must be affected by the inhibition otherwise the remaining quantum states still make transitions to well (c) strongly limiting the efficiency of the inhibitory effect. For all quantum states in well (a) to be affected, the bandwidth of the artificial electromagnetic wave must be at least the frequency step between adjacent states in well (b).

The frequency condition is due to the fact that the frequency of the artificial wave must be adapted to cause transitions between wells (a) and (b) taking into account the energies of quantum states in these wells.

If the spectral power of the artificial wave is below the power spectrum of the thermal background there is no significant inhibition because the artificial electromagnetic wave does not significantly affect the Rabi frequency of the (a) to (b) transition. The inhibition thus only becomes significant when the artificial wave has a sufficient power, corresponding to the power condition. The required power depends on the specific TCR-pMHC system considered.

The inhibition of the TCR-pMHC complex also occurs during negative selection in the thymus, except that during negative selection in the thymus the TCR-pMHC recognition yields elimination of the T cell by negative thymus selection rather than destruction of the APC. Thus, the inhibition of antigen recognition by an artificial electromagnetic wave also applies during the negative selection process. During the negative selection process, inhibition of antigen recognition can prevent elimination by negative selection (ENS) of a T cell which would not normally (i.e. absent the artificial wave) survive positive selection. This mechanism deeply modifies the overall effect of electromagnetic waves on the immune system. Various cases can be considered:

**[0013]** - Effect on cancer after a low to high exposure transition: After a low to high exposure transition, the capacity of T cells to recognize cancerous cells is diminished. But T cells newly produced by the thymus after the transition are selected in high exposure conditions and will thus have a normal capacity to recognize the self in high exposure conditions. These newly produced T cells will also have a normal capacity to recognize cancerous cells, because cancerous cells are little different from normal "self" cells. After a certain time newly selected T cells will dominate and a normal behaviour of the immune system relative to cancer will be reinstated. The pro-cancer effect of a low to high exposure transition is thus temporary.

**[0014]** - Effect on cancer of temporary high exposure or alternating high/low exposure: Due to the inhibition of the TCR-pMHC recognition, selection steps that occur in the thymus in high exposure conditions fail to eliminate T cells which would be eliminated in low exposure conditions. When low exposure conditions are re-instated, these T cells become abnormally aggressive towards the self, resulting in an anti-cancer effect.

**[0015]** - Effects on infectious diseases that do not mimic the self after a low to high exposure transition: After a low to high exposure transition, ongoing immune responses against pathogens are weakened, which may result in a loss of control of the pathogen by the immune system. After a certain time a new primary immune response may yield to a new immune response able to control the pathogen. However, antigens of pathogens which do not mimic the self are clearly different from self antigens. Thymus selection reinstates a normal behaviour of the immune system towards self antigens but is unlikely to reinstate a normal behaviour of the immune system towards non-self antigens. Therefore a normal behaviour of the immune system towards infectious diseases that do not mimic the self is not reinstated by thymus selection, and it is expected that there will be a long term pro-pathogen effect in high exposure conditions.

**[0016]** - Effects on infectious diseases of temporary or alternating high/low exposures: during high exposure periods there is a pro-pathogen effect but there is no appreciable modification of thymus selection with respect to non-self. Therefore during the low exposure periods the behaviour of the immune system relative to pathogens is essentially normal. The overall effect is pro-pathogen. If the high exposure periods represent a small fraction of the time, this pro-pathogen effect may however be negligible.

Auto-immune diseases, characterized by an excessive immune response against the self, are expected to react opposite to cancer, which is favoured by an insufficient immune response against self-like cancerous cells. Inflammatory diseases like pneumoconiosis, which are characterized by an excessive immune response against non-self, are expected to react opposite to infectious diseases, which are favoured by an insufficient immune response against non-self.

Infectious diseases that mimic the self can have more complex behaviors. On average they may be less favored by permanent exposure than other pathogens because their behavior in this respect is intermediate between "normal" pathogens and cancer.

The following table summarizes the effect of exposure to wideband, low-power electromagnetic waves on various diseases:

Table 1: Table of effects of electromagnetic waves

| | A | B | C | D | E |
|---|---|---|---|---|---|
| exposure<br><br><br>Immune threat | Permanent low exposure (thermal) | Permanent high exposure | Transition from Low to High exposure: period immediately after transition | Transition from High to Low exposure : period immediately after transition | Alternance between low and high exposure (at least half time low exposure) |
| Cancer | Standard | standard | Pro cancer | Anti cancer | Anti cancer |
| auto-immune disease | Standard | standard | Anti-autoimmune | Pro-autoimmune | Pro-autoimmune |
| pathogen (not self-mimicking) | Standard | Pro pathogen | Pro pathogen | standard (1) | Slightly Pro pathogen |
| inflammatory disease directed against non-self | Standard | Anti-inflammatory | Anti - inflammatory | standard (2) | Moderately Anti-inflammatory |
| Self-mimicking pathogen | Standard | Neutral to Pro-pathogen | Variable | Variable | Variable |

(1): standard with respect to permanent low exposure but anti-pathogen relative to high exposure.

(2): standard with respect to permanent low exposure but pro-inflamatory relative to high exposure

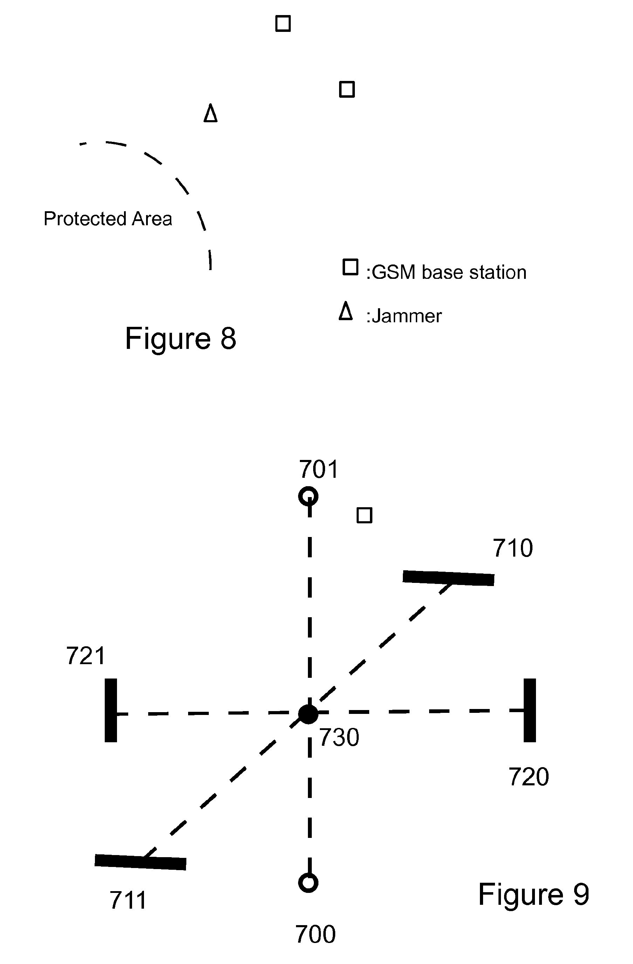

This table is in agreement with Table 2 of HAL- 00877298 , and with Table "INH+ENS" of figure 8 of HAL-009755963 and more detailed justifications for it can be found in HAL- 00877298 and in HAL-009755963, particularly sections 2.1 to 2.2.3 of HAL-009755963. This Table is also Table 2 of US provisional application 61889065. Further evidence confirming the correctness of this table can be found inter alia in section 9 of HAL-00877298 version 2, in sections 3.1.7 to 3.1.10 of in HAL-009755963 and in section 4.12 of US provisional application 61889065. This evidence is summarized hereafter.

In the cities of Pontchateau, Saumur, Segré in France there occurred a significant maximum of the yearly death rate in the 35-54 years age class relative to all-ages death rate in the year starting on day 450 after the onset of Digital Video Broadcasting (DVB) and abandonment of analog TV in 2005, this maximum being common to these cities and non-existent in the comparable cities of Chateaubriant, Cholet, Ancenis, Challans, La Fleche which did not have a DVB onset. The number of deaths for these cities for different ages and times was obtained from death notices on www.avis-de-deces.net. This maximum was attributable at least in part to cancer in view of the fact that in Paris similar death rate disturbances attributable to DVB onset occurred in the age category 35-45, the maximum being attributable essentially to cancer according to cause of death statistics from CEPIDC. Since the power in Pontchateau was measured and was everywhere below 5 $\mu$W/m2 (5.10$^{-6}$ W/m2), the power threshold for the pro-cancer effect is below 5 $\mu$W/m2.

In Paris cancer death rate increased significantly in 2005 following the onset of DVB in the less than 35 years old and it increased only in 2006 in the 35-44 age category. This delay for the increase in the 35-44 age category is comparable to the delay for the increase in Pontchateau, Saumur, Segré and both delays are attributable to the delay from the start-up of cancer until death, which is higher in the 35-44 age category than in the less than 35 age categories.

In Paris in 2005 in the 35-44 age category there was also a statistically significant temporary decrease of the death rate by heart diseases, showing that heart diseases probably have an auto-immune aspect so as to decrease in this situation pursuant to Table 1. There was also a statistically significant decrease in chronic diseases of the liver, which lasted longer than the decrease of heart diseases, so that it is unclear whether chronic diseases of the liver behave like auto-immune diseases or inflammatory diseases directed against non-self.

Cancer death rate increases also after onset of GSM base stations as results from ("Increased Incidence of Cancer near a Cell-Phone Transmitter Station", International Journal of Cancer Prevention 2004 vol 1 no 2, by R Wolf and D Wolf). In this case there is both an anti-cancer effect as per Table 1 column E due to day/night power variations, and a pro-cancer effect as per Table 1 column C due to the low to high exposure transition at onset of the base station. For heavily loaded base-stations emitting day and night and during the period immediately following onset of the base station, the pro-cancer effect dominates for permanent residents of the base station area. In the above-mentioned publication, most victims were women, probably because they did not work and thus remained permanently exposed to the base station emissions, favoring the pro-cancer effect.

Concerning pneumonia, a detailed examination of death statistics for France, as published in the databases of the INED (Institut National des Etudes Demographiques) and CEPIDC-Inserm shows that the variations of the age and sex-normalized pneumonia death rates ( with the 1999 population as a reference) were +32% in 1981, corresponding to the introduction of tolerated of FM radio (which caused an immediate nation-wide boom in FM radio broadcasting), +23% in 1983 corresponding to the official authorization of FM radio, triggering nationwide power increases in emitted power, +46% in 1985 corresponding to the start-up of "Canal+" commercial analog TV broadcasting, +17% in 1986 corresponding to the start-up of the 5th channel, a commercial TV, and +16% in 2005 corresponding to the start-up of digital television. The 20-year maximum value before 1981 was +11% and the maximum interim value between 1987 and 2004 was +9%. Thus there is a correlation between short-term increases of the death rate by pneumonia, and the increases in emitted power for waves causing essentially permanent exposure, particularly FM radio (88MHz to 108 MHz) and analog and digital TV (up to about 790 MHz). This correlation seems to be stronger for lower frequency waves as the most obvious increases are for FM radio and Canal+, which emitted mostly around 200 MHz. These temporary increases of pneumonia were due in each case to a sudden increase of the occupied bandwidth and power of artificial waves. T cell lines which were controlling existing infections lost control brutally due to the inhibition of the TCR-pMHC by artificial waves and patients who did not timely have a novel primary immune response died.

Comparison of the pneumonia death rates in France and other countries show that the death rate in France relative to a panel of other countries weighted according to their population (Austria, Belgium, Denmark, Finland, Greece, Ireland, Italy, Netherlands, Norway, Portugal, Sweden, Switzerland, Germany) increased from 28% to 77% between 1980 and 1987 based on the OECD statistics and German Statistisches Jahrbuch. If French figures from the INED "causes of death from 1925 to 1999" database are used the increase is from 30% to 50% during the same period. In either case there is a long term increase of the relative death rate confirming the existence of an effect on infectious diseases of a permanent wave as per Table 1 column B, which is not limited to the transient period immediately following the change in exposure conditions.

The long-term increase of pneumonia death rate in France was accompanied by a long-term decrease in death by inflammatory respiratory diseases directed against non-self, i.e. pneumoconiosis, emphysema, non-obstructive chronic bronchitis. Death rates by these diseases increased by 2% in the 7 years period ending in 1978, but decreased by 33% in the 7 years period ending in 1987 and by 35 % n the 7-years period ending in 1994. This marked change of tendency, occurring simultaneously with the increase in pneumonia death rate, confirms the anti-inflammatory effects of permanent exposure as per Table 1 column B.

Based on INED and CEPIDC figures, asthma death rate also increased by roughly 30% between 1981 and 1986, which was an exception in a generally descending curve. The level of asthma deaths stabilized after 1990 at a level superior to the 1977-1980 level, showing that asthma death rate increases with permanent exposure as per Table 1 column B. The mechanisms behind this increase are not well understood, because asthma is not a well-understood disease.

Globally, death rate by respiratory diseases was not strongly affected during the 1981-1987 period but the equilibrium between infectious respiratory diseases (mostly pneumonia), inflammatory respiratory diseases, and asthma was strongly modified as predicted by Table 1 (for pneumonia and inflammatory diseases).

Multiple sclerosis death rate (from CEPIDC) in France for women in the 55-64 and in the 65-74 age ranges, as well as the overall multiple sclerosis death rate for women, show correlated peaks in 1994, corresponding to a multiplication by 7 of GSM traffic in this year. These death rates peaked again in 2002, probably corresponding to the spreading of wifi. They were significantly higher in the post-1994 period than in the pre-1994 period. These facts show that multiple sclerosis, which is an auto-immune disease, increased due to GSM and other time-variable waves, as predicted by Table 1 column E. This is to be compared to the global decrease of cancer death rate in France during the post-1994 period, also in agreement with Table 1 column E.

Parkinson's disease, which is believed to have both an infectious and an auto-immune aspect, correspondingly increased in France both during the 1981-1986 period and during the post-1994 period, which were respectively pro-infectious and pro-auto-immune, confirming the dual aspect of the disease. These changes in Parkinson's disease death rate, as compared to a general tendency, separately affected each of the 65-74 and 75-85 age categories for men (based on INED and CEPIDC death rates), showing that they were not due to changing age structure of the population.

Creutzfeldt-Jacob disease (CJD) is known to be caused by a disease-causing form PrP-Sc of a prion. The variations of the death rate by CJD can be understood by assuming that:

- the disease-causing form PrP-Sc causes a reaction of the immune system, probably including T cells attacking APCs that present PrP-Sc peptides and/or B cells producing antibodies directed against PrP-Sc.
- PrP-c being either a strict isoform (sporadic CJD) or nearly an isoform (infectious CJD) is attacked by the immune system as a side effect of the attack directed against PrP-Sc.

- The attack by the immune system of the PrP-c proteins disorganizes them in an effect analog to known denaturation processes, causing the formation of PrP-Sc
- the newly formed PrP-Sc participate in causing an increased response of the immune system and an exponential growth of the proportion of PrP-Sc ensues.
- The reason why PrP-Sc causes a reaction of the immune system whilst PrP-c does not may be due to PrP-Sc being treated as foreign inside a cell due to its abnormal folding and associated disturbing effects, so that PrP-Sc peptides get displayed on MHCs by a cell hosting PrP-Sc whilst for equivalent concentrations the normal PrP-c peptides would not be displayed.

Thus, in this model CJD essentially appears as an exponentially-growing auto-immune reaction. Because CJD develops fast, as opposed to most auto-immune diseases which are long-term diseases, its reactions to changes in electromagnetic waves are more immediate and thus easier to correlate with the changes, as compared to other auto-immune diseases.

In France, based on CEPIDC death rates, the number of deaths by CJD dropped momentarily in 1981 for men in age categories over 65 (shortly missing the significance limit with P_=0.059 calculated as per section 9.1.1. of HAL-00877298) and in 2005 (significant with P_=0.034) corresponding to the transient response of CJD, an auto-immune disease, as per Table 1 column C. There was no other significant or near-significant decrease between 1979 and 2010. In 1994, corresponding to the multiplication by 7 of GSM customer basis, there was a near significant increase (P+=0.07) which was followed by other significant or non-significant yearly increases. Overall, the number of yearly deaths for men in the age categories over 65 increased about 3-fold between the pre-GSM years and 2010, corresponding to the effect of GSM, Wifi and other time-variable waves as per Table 1 column E on auto-immune diseases.

The number of yearly CJD deaths for men aged less than 34 years, each year from 1992 to 2008, was significantly higher than during the 1979-1991 period. The number of deaths increased roughly 15-fold between pre-1991 and post-1994. It increased earlier in younger age categories. This is interpretable as follows: When the auto-immune reaction was started by GSM exposure, there was only a limited reservoir of persons carrying the disease-causing prion. Once this reservoir was exhausted the number of deaths dropped. Prior to 1991 there were no deaths in the 15-24 age class but there were prion-carrying subjects. After start of GSM exposure all pre-existing prion carriers died within a few years. After that the reservoir was exhausted and newly infected persons were dying as soon as infected, yielding the low (but higher than pre-GSM) post-1998 level in the 15-24 years age category. In the older age categories 65+ years the "reservoir effect" was lower as the increase in the number of deaths was only about 3x instead of 15x.

From the above considerations, it appears that as of today a majority of the CJD deaths are linked to the existence of GSM and other time-varying waves.

Depressive disorder can be monitored by examining death rates by suicide. The number of deaths by suicide in Paris for women aged 55-84 years increased strongly in 2005, most particularly in the 75-84 age category. It also increased significantly for men in the 55-74 age class, but not as much as for women. But for men aged 24-44 there was a significant decrease of the number of deaths by suicide in 2005. The difference between age categories can be explained as follows: the thickness of the myelin sheath in known to increases with age. There is probably an ideal thickness of the myelin sheath for which conduction of the nervous influx is optimal. In the transient period following the onset of DVB, which was anti-autoimmune, the thickness of myelin sheaths increased. Older persons have myelin sheaths which are already too thick and this further increase braught them farther from optimal equilibrium, causing mental problems including suicide. Younger persons have myelin sheaths which are too thin, and the increase of the myelin sheath thickness braught them nearer to optimal thickness, reducing the incidence of mental problems including suicide.

The effect of GSM is opposite to the effect of the DVB transition period so GSM is expected to increase mental problems including suicide in young men. Correspondingly, a peak of suicide deaths is identifiable in 1993-1994 for men of younger age categories alone. For the 15-44 years age range, 1993 is the only statistically significant year-to-year increases between 1986 and 2010. There was no corresponding statistically significant increase in the 55-84 age category between 1986 and 2002. The fact that this peak affected selectively the younger age categories, confirms that it was caused by GSM, since the younger age categories are the only ones which based on the reaction to DVB can be predicted to react to GSM by an increased suicide rate. The fact that the increase was already significant in 1993 shows that the sensitivity threshold is low.

The body mass index (BMI) of French men was stable between 1981 and 1992 but increased by about 4% between 1992 and 2003 (source: INSEE). The BMI of French women was already increasing between 1981 and 1992 but at a fairly low rate and this increase came to about 5% between 1992 and 2003. This increase is to be compared to the corresponding disturbances of feeding behavior and corresponding weight increases caused by GSM around 900 MHz which were found in mice at 0.4 W/kg ("No effects of GSM-modulated 900 MHz electromagnetic fields on survival rates and spontaneous development of lymphoma in female AKR/J mice", BMJ cancer 2004, by A M Sommer and J Streckert and A K Bitz and V W Hansen and A Lerchl) then in Djungarian hamsters at 0.08 W/kg ("Effects of mobile phone electromagnetic fields at nonthermal SAR values on melatonin and body weight of Djungarian hamsters", Journal of Pineal Research 2008, by A. Lerchl; and "Einfluss hochfrequenter Felder des Mobilfunks auf die metabolische Umsatzrate in Tiermodell (Labornager)", published by Jacobs-University Bremen) then on mice at 900 MHz cw at 1 V/m ("effects of chronic exposure to radiofrequency electromagnetic fields on energy balance in developing rats", Environmental Science and Pollution Research 2013, by A Pelletier and S Delanaud and P Decima and G Thuroczy and R de Seze and M Cerri and V Bach and J P Libert and N Loos). Thus the BMI increase of the French population between 1992 and 2003 is due to the same cause as the experimentally observed BMI and disturbed feeding behavior in rodents, i.e. GSM-900. However this is probably unrelated to the immune system and it could be related to effects on a receptor implied in controlling the stomach, possibly on interstitial cells of Cajal which are known to react to electromagnetic waves (J. Kaszuba-Zwoinska, loss of interstitial cells of Cajal after pulsating electromagnetic fields (pemf) in gastrointestinal tract of the rats, Journal of Physiology and pharmacology, 56(3):421-432 www.kpp.krakow.pl). The effect exists at low or high bandwidth (for example the work by De Seze used a cw wave) and it is stronger around 900 MHz as in the work by Lerchl other frequencies were tried that did not have as strong an effect. The effect is applicable to the human being in view of the statistics by the INSEE. However whilst on small rodents the effect was considerably stronger in males than in females, in the human being the effect seems to essentially affect both sexes.

In addition to the above-mentioned evidence, further evidence and reasoning (for example in section 4.14 of provisional application number US 61889065) shows how artificial electromagnetic waves influenced emerging diseases. These are summarized hereafter.

Lyme's disease is due to Borrelia Burgdorferi which is a cyst-forming spirochaete also capable of molecular mimicry. By forming cysts, it can escape the immune system during periods of low exposure, but replicate and cause health disturbances during periods of high exposure which are pro-pathogen according to Table 1 column B. Further, being capable of molecular mimicry, it can take advantage of pro-auto-immune conditions as per Table 1 column E which can cause a strengthening of regulatory systems which can itself cause the elimination of T cell lines which would otherwise have eliminated Borrelia Burgdorferi. For these reasons, Lyme's disease is favoured by both permanent and time-variable waves and, most importantly, it can survive periods of low exposure. This explains the observed increased of Lyme disease incidence.

Alpine ibex in the area "Vanoise" died in abnormal numbers starting in the winter 2006/2007 in the Champagny area. Many dead animals had keratoconjunctivitis, which can be auto-immune and/or linked to a Borrelia infection. This is probably attributable to the arrival of UMTS in the area which increased the dangerousness of Borrelia. The high death rate may be due to the lack of genetic variability of these ibex, with possibly a large number of animals having the same MHC type being particularly sensitive to the effect of UMTS on Borrelia..

The proportion of influenza deaths in the 15-54 years age category in France (number of deaths in this age category divided by total number of deaths) increased during the influenza pandemic of 1918, increased again in the pandemics of 1957 and 1969, then remained lower until 1984, then from 1987 onward this proportion started to vary unusually strongly, regularly reaching the values of 1957 and 1969 (but not the 1918 values). In 2009-2010 this proportion increased to an historical high, higher than any value since 1925 at least, and comparable to the 1918 value. There was no pandemic in France in 2009-2010, probably because most pandemic deaths actually occurred through opportunistic pneumonias in 1918, and pneumonia was controlled by antibiotics in 2009-2010. Influenza has some self-mimicry capacities, which is confirmed by the association of the vaccine with Guillain-Barré syndrome, which is auto-immune (Immunization Safety Review consensus report: influenza vaccines and neurological complications. Technical Report. Institute of Medicine of the National Academies, Washington DC, October 2003) and by the high proportion of pregnant women who died or lost their child during the 1918 pandemic (The great influenza, John M. Barry, penguin books 2004). Like is the case for Lyme's disease, this self-mimicry capability causes influenza to be particularly favored by time-varying waves like GSM, with the pro-influenza effect of GSM being stronger in age categories which have a more active thymus, which explains the pandemics of 1918 which coincided with the brutal expansion of the power and bandwidth of emitted waves when the United States entered the first world war, corresponding to military radio-telephony systems and other radio emissions favoring both influenza and pneumonia, and the increasing values of the proportion of deaths in the 15-54 years age class after 1987, corresponding to the spreading of modern radiotelephony (analog then digital) and culminating with the near-pandemic of 2009-2010.

AIDS is a viral disease which the immune system is often unable to control. There is strong evidence that HIV was already present in Africa before the 1960s but was not deadly. First cases of AIDS were all cases in which the HIV virus contaminated the victim in Africa in a region which did not have television, but developed AIDS only in a place which had television (Arvid Noe, Grethe Rask, and a number of similar cases). There seems to be no record of any case which developed AIDS in a country which did not have Television. In view of these facts, it appears that exposure to analog television and possibly FM radio increased the dangerousness of HIV pursuant to Table 1 column B and caused it to become unusually dangerous whilst HIV was not per se unusually dangerous.

Heart diseases, particularly heart attacks, diminished in Paris in men of the 35-44 years age category in 2006 following the onset of DVB in 2005, which makes them interpretable as auto-immune diseases reacting as per Table 1 column C to the change.

Electro-Hyper-Sensitivity (EHS) is an emerging syndrome having multiple aspects including sleep disturbances and tingling and numbness, which appear to be linked to a disturbance of the immune system as discussed in section 4.15 of US provisional application number 61889065. Basic considerations about these aspects of the EHS syndrome are discussed hereafter.

Sleep disturbances usually have a 2-day time lag (table 7.6, low exposure group, of "Study of health effects of the shortwave transmitter station of Schwarzenburg", Technical Report, university of Berne, Switzerland, 1995) also explaining why in ("EMF protection sleep study near mobile phone base stations", by Leitgeb, Somnologie 12:234-243, 2008) where participants slept three non-consecutive nights in a Faraday cage, the effect of the Faraday cage resulted in a worsening rather than an improvement of sleep quality. These data are consistent with the sleep disturbances being caused by a pathogen which is favored by electromagnetic waves. This pathogen is most likely a cyst-forming spirochaete because in the absence of electromagnetic waves it is capable to survive as cysts, and be reactivated later upon exposure. When exposure brutally ceases, it takes 2 days for a secondary immune response to develop, forcing the spirochaetes to form cysts, and thus ending the sleep disturbances. The cyst-forming spirochaete reacts, inter alia, to FM and GSM exposures which were dominant in the Leitgeb study.

Pro-auto-immune effects of time-varying waves as per Table 1 column E have demyelinization as a direct result. Demyelinization causes tingling and numbness, which are common symptoms of multiple sclerosis and other demyelinating diseases. Most EHS victims having tingling and numbness feelings in the presence of electromagnetic waves believe that they "feel" the waves, because of the nature of these feelings and because these feelings cease after a sufficiently long non-exposure period. But the feeling of tingling and numbness does not occur instantaneously upon exposure because it requires some time for the demyelinization to reach a sufficient level to cause these feelings. This explains why these persons usually cannot detect any electromagnetic wave in short-term provocation studies.

The theory presented herein yields a rational understanding of many effects of electromagnetic waves on the human being. Theoretical predictions are confirmed by statistics. Threshold levels for some of these effects are several orders of magnitude lower than has ever been contemplated in previous publications. The proven and suspected health consequences of the use of electromagnetic waves in communication systems are described in the following paragraphs.

Negative consequences of permanent exposure to permanent waves (TV, radio) in permanent regime comprise the increase of death rate by Parkinson's disease and the increase of death rate by infectious diseases generally, and most likely the emergence of AIDS. Negative consequences of exposure to permanent waves (TV, radio) in a transient periods after a low to high transition comprise cancer outbreak and increased depressive disorders (suicide outbreaks) in older age categories. Negative consequences of exposure to time-varying waves (GSM, Wifi, UMTS, wireless telegraphy) include the increase of multiple sclerosis death rate, the increase of Creutzfeldt-Jacob disease death rate, the increase of depressive disorder (suicide) in younger age categories, the EHS syndrome, the increase of Parkinson's disease death rate, the EHS syndrome and the increase of body mass index, and most likely the increase of incidence of Lyme's disease and the flu pandemic of 1918.

Positive consequences of exposure to permanent waves (TV, radio) in a transient periods after a low to high transition comprise diminished death rates for heart and liver diseases in certain age categories, diminished death rates for Creutzfeldt-Jacob disease in older age categories, and diminished depressive disorders (suicides) in younger age categories. Positive consequences of exposure to time-varying waves (GSM, Wifi, UMTS, wireless telegraphy) include diminished death rate by cancer and decrease of depressive disorders (suicides) in older age categories.

This invention is based on the above-discussed background. According to this background, levels of irradiation by artificial electromagnetic waves that are commonly present in today's crowded electromagnetic environment do:

- cause or worsen severe diseases by themselves (multiple sclerosis, cancer, depression) or in association with pathogens (infectious diseases, including AIDS, Lyme disease, Creutzfeldt-Jacob disease).
- prevent or cure certain diseases (cancer, cardiomyopathy, heart attacks, disease of the liver, depression)
- cause abnormal weight increase (in at least the case of GSM), which may be viewed as a disease.

The objective of this invention is to improve health of patients generally. This objective is achieved, according to this invention, by suitable use of a Faraday cage and/or an emitter of medical electromagnetic waves. The Faraday cage allows the reduction of the power of the artificial electromagnetic waves which are present in the environment, and the emitter of medical electromagnetic waves allows the generation of controlled medical electromagnetic waves to which the patient is exposed purposely. The term "medical electromagnetic wave" is used to distinguish medical electromagnetic waves purposely emitted to improve health from other artificial electromagnetic waves which are present in the environment for other reasons. Depending on the case the Faraday cage can be used alone, or the emitter can be used alone, or they can be used jointly. The Faraday cage and/or emitter of medical electromagnetic waves make it possible to submit patients to an electromagnetic environment which is purposely and individually adjusted to their medical needs.

**Faraday cage**

An aspect of this invention is a treatment of disease and/or the reduction of body weight by use of a Faraday cage.

However diseases which can be cured by use of a Faraday cage are caused or worsened by the presence of electromagnetic fields used for telecommunication purposes, including but not limited to FM radio, analog and digital TV, GSM, UMTS, Wifi. This aspect of the invention is therefore also a method for telecommunicating comprising a step of emitting an artificial electromagnetic wave reaching an exposed area exposed to said artificial electromagnetic wave, wherein a person living in said exposed area suffers of a disease, and wherein said method is characterized by the fact that it comprises administering to said person a stay in a Faraday cage. This method for telecommunicating is useful whenever the artificial electromagnetic wave used for telecommunicating causes or worsens the disease affecting the person living in the exposed area and/or whenever suppressing this artificial electromagnetic wave results in an improvement of the immune system's capability to overcome the disease from which the person suffers.

Diseases to be treated vary widely. Electromagnetic waves affect the immune system and therefore plays a role in all forms of disease. The use of a Faraday cage makes it possible to control this environment. In some cases the Faraday cage may be used alone because the best environment is a natural environment free of artificial electromagnetic waves. In other cases the Faraday cage may be used jointly with an emitter of medical electromagnetic waves because the best treatment for the disease requires some (not necessarily permanent) electromagnetic waves. Diseases which can be treated in view of the theory and statistics include, non-limitatively, infectious diseases (including pneumonia, AIDS, diseases caused by cyst-forming spirochaete), cancer, asthma.

The treatment may be limited to the use of a Faraday cage with or without an emitter of electromagnetic waves, or it may additionally comprise the use of an orally or intravenously administered substance which effects complements those of the immune system. The treatment by use of a Faraday cage may also be complemented by other known treatments generally.

Diseases to be treated include, but are not limited to, cancer, autoimmune diseases, infectious diseases, body weight problems, depressive disorders. The treatment comprises repeated stays of a patient to be treated in the Faraday cage or a permanent presence of the person to be treated in the Faraday cage.

Concerning autoimmune diseases the Faraday cage if used alone can improve the situation in the long term, essentially by providing a stable environment.

However the person to be treated should preferably stay full time in the cage for a time which counts in weeks if the person is young and in years after involution of the thymus. In the interim period, the autoimmune disease tends to worsen. For this reason, the treatment of autoimmune diseases in a Faraday cage preferably comprises exposure to a controlled medical electromagnetic wave, which can be generated by an emitter of electromagnetic waves inside the Faraday cage, and which is described in more detail in the following sections.

Concerning cancer, the Faraday cage improves the situation in the very short term by allowing T cells to bind cancerous cells which they would not bind otherwise. As shown in Table 1, the high exposure to low exposure transition shifts the cancer/autoimmune balance towards autoimmunity, so that capabilities to recover from cancer are enhanced, at the cost of an increased risk of autoimmune disease. The efficiency of the Faraday cage is high in this use, but for young people under 25 the likelihood of the immune system activating an appropriate line of T cells against the cancer decreases over time due to new naive T cells produced by the thymus during the stay in the Faraday cage being less able to bind the self than T cells produced by the thymus outside the Faraday cage. This inconvenience can be partly avoided by using treatment by alternate stays in the Faraday cage and outside the Faraday cage to produce a pro-autoimmune situation according to Table 1. After thymus involution the high exposure to low exposure transition lasts longer and alternate stays are less necessary. Alternate stays are however not an ideal solution because the outside environment in not properly controlled and its effect on any particular cancer in uncertain. A better solution is to use the Faraday cage jointly with an emitter of medical electromagnetic waves as will be described in more detail in the next section.

Use of a Faraday cage is expected to improve the immune system's position with respect to infectious diseases "on average" but medical follow-up is necessary, because auto-immune problems may arise in certain situations, because modified inflammatory responses may occasionally cause problems, because toxins released by dying bacterias may reach dangerous levels due to the sudden character of an immune reaction, and/or because self-mimicking pathogens may in certain cases be temporarily favoured after a transition from high exposure (outside the Faraday cage) to low exposure (inside the Faraday cage). The importance of the Faraday cage is expected to be highly dependent on the exact disease. Infectious diseases are caused by pathogens, including but not limited to bacteriae, fungi, viruses.

Fever increases the efficiency of the immune system and, inter alia, accelerates the process of primary and secondary immune responses. Staying in a Faraday cage has the same effect. When the immune system cannot overcome a threat, fever tends to increase unreasonably. When a patient is in a Faraday cage, his immune system is more efficient against pathogens and therefore fever needs not increase as much as outside a Faraday cage. According to a version of the invention, a treatment for fever comprises a stay or a series of repeated stays in a Faraday cage. For example a treatment for fever comprises measuring the body temperature from time to time and starting a stay or a series of repeated stays in the Faraday cage when the body temperature increases.

Generally, infectious diseases which are not quickly controlled by the immune system in the crowded electro-magnetic environment of every-day's life will be controlled faster inside a Faraday cage. According to an aspect of the invention, the treatment for infectious diseases comprises repeated stays of a patient to be treated in the Faraday cage or a permanent presence of the patient to be treated in the Faraday cage. The treatment preferably lasts at least 2 days, most preferably it lasts for more than one week. Generally the duration of treatment is dependent on the exact illness being treated, and treatment should preferably last until the immune system finds an immune response that will work even outside a Faraday cage or until the pathogen is entirely eliminated. Typically, the patient should sleep in the Faraday cage but in not too severe cases can be allowed his normal activities when awake (outside the Faraday cage). In more severe cases it is necessary for the patient to stay permanently in the Faraday cage during the treatment. In many cases the Faraday cage method avoids the exaggerate use of antibiotics. Treating diseases with a Faraday cage is a more natural treatment than antibiotics and is believed to cause less development of allergies in the long term.

However, in difficult cases or when a particularly fast result is required, the Faraday cage may advantageously be used in conjunction with antibiotics, anti-fungals, anti-viral or other orally or intravenously administered products, as the case may be.

Some specific diseases are highly sensitive to the radiofrequency environment. AIDS is one of them, and accordingly an aspect of the invention is the use of a Faraday cage for the purpose of treating AIDS, the treatment comprising repeated stays of a patient to be treated in the Faraday cage and/or a permanent presence of a patient to be treated in the Faraday cage.

In the initial period of treatment after entering the Faraday cage, in case a tritherapy has already been started, it is wise to continue it simultaneously with the use of the Faraday cage to avoid a potentially damageable loss of immune control. Even if tritherapy has not yet been started, using it will facilitate the initial immune response by giving more time to the immune system to develop its immune response. The more advanced the disease is at startup of the treatment, the more useful a tritherapy is, to help an already weakened immune system. In a second period of the treatment, once an immune response has shown capability to fight the disease in the low exposure conditions, it may be preferable to abandon tritherapy - under regular medical control - and let the immune system make its job. Even in a non-controlled environment a small percentage of people treated by tritherapy manage to control the virus after the end of tritherapy. Use of a Faraday cage by patients will increase this percentage. After abandonment of tritherapy the patient should preferably undergo regular tests to check that the virus is remaining under control.

The Faraday cage may also be used against AIDS without tritherapy. This option is particularly interesting for patients who cannot afford tritherapy but may afford a Faraday cage. A Faraday cage can be built for much cheaper than the cost of a tritherapy, and hospitals may build collective Faraday cages for hosting AIDS patients at minimal cost. It is also interesting for patients who wish to avoid the difficult experience of tritherapy. A Faraday cage is minimally invasive as compared to tritherapy despite the constraint of preferably staying in full-time. It is expected that a higher number of patients of patients will be capable of controlling AIDS without further medication when staying full time in a Faraday cage, than outside a Faraday cage. It is also expected that a higher number of patients will be able to control AIDS when staying part time in a Faraday cage (typically, at night) than when staying full time outside a Faraday cage.

Preferably, the patient should initially stay full time in the Faraday cage. Once a proper immune response is established, an adaptation process can preferably be followed to progressively allow the patient to stay out of a Faraday cage. Under such process, the patient will initially stay out of the Faraday cage for a short time each day, and the time out of the Faraday cage will be progressively increased, with the patient being regularly tested to confirm continued control of the virus, until the patient can be content with using the Faraday cage at night only. Some patients may will even be able to survive without a Faraday cage as happens to some tritherapy patients. But for a majority of patients it is not advisable to fully cease using a Faraday cage. In case of strong autoimmune problems, unless the patient manages to control HIV without a Faraday cage, it may be preferable to relocate the patient to an area less exposed to electro-magnetic waves, whilst still using the Faraday cage part time.

Alternatively, the patient may at all times stay only part-time in the Faraday cage. In this version of the treatment, the Faraday cage encloses a bed and the patient sleeps inside the Faraday cage each night. This version is however not preferred; a full-time stay in the Faraday cage is expected to be much more efficient, because it allows the immune system to fight AIDS full-time rather than part-time and because it avoids creating a pro-auto-immune effect which may indirectly favour HIV.

Some patients complaining of sleep disturbances are already treating themselves using a Faraday cage. Such sleep disturbances may be caused by certain spirochaete which have the ability to agglomerate into cysts. When agglomerated into cysts they are largely protected from the immune system. By exception to the above, use of a Faraday cage for the treatment of sleep disturbances caused by cyst-forming spirochaetes does not fall within the scope of this invention unless complemented either by the use of antibiotics or the use of a medical emitter of electromagnetic waves or unless a low-pass Faraday cage is used. But use of a Faraday cage in combination with an emitter of electromagnetic waves is within the scope of the invention, even when used to treat sleep disturbances. Use of a Faraday cage in combination with antibiotics for the treatment of sleep disturbances is also within the scope of this invention. Use of a low-pass Faraday cage to treat sleep disturbances is also within the scope of this invention.

Cyst-forming spirochaete which cause recurrent fevers cause discomfort generally and can be life-threatening. Therefore it is preferable to eliminate them. Cyst-forming spirochaete which cause exacerbated electrosensitivity in the form of sleep disturbances must also be eliminated in the present context in which electromagnetic waves are widely present at high levels. It may also be desireable to diagnose and eliminate cyst-forming spirochaete preventively so as to avoid later diseases. Antibiotic treatments against cyst-forming spirochaete are little efficient.

It is a purpose of this invention to treat diseases caused by cyst-forming spirochaete, by elimination of the spirochaete from the organism or reduction of their numbers.

In a version of the invention, the treatment comprises administering to the patient a cyst-inhibiting antibiotic, such as tetracycline, for reducing the number of cyst-forming spirochaete in a patient's body, to treat a disease caused by cyst-forming spirochaete. Jointly using the Faraday cage and such an antibiotic results in the immune system being in optimal conditions for eliminating the spirochaete and in the spirochaete being prevented from escaping the immune system by forming cysts. The antibiotic is preferably administered so as to be active when the patient is staying in the Faraday cage.

Other infectious diseases that can be treated by a stay or a series of stays in a Faraday cage include:

- influenza, particularly any dangerous existing (like H1N1) and future types of influenza.
- keratoconjunctivictis of the ibex and other animals, in veterinary practice.
- pneumonia, based on the statistics.

In the case of diseases which have or may have an interaction with auto-immune problems, like keratoconjunctivictis, some influenza viruses or Lyme, a permanent stay in the Faraday cage is preferred so as to avoid creating a permanent pro-autoimmune situation which may favour the pathogen, and there may be a high risk period immediately after entering the Faraday cage during which, due to the pro-auto-immune transitional effect, a worsening of the disease cannot be fully excluded.

Other diseases that can be treated by use of a Faraday cage include asthma and most likely allergies, based the fact that asthma increased due to permanent electromagnetic waves. In this case the Faraday cage should ideally be used full time but part time stays in the Faraday cage should also contribute in decreasing asthma and allergies. As the risk of asthma and allergies in general is believed to be linked to a difficulty of the immune system in overcoming infectious diseases, it is expected that using the Faraday cage for treating infectious diseases in replacement of antibiotics should strongly contribute in reducing the incidence of asthma and allergies, especially amongst children.

The Faraday cage can theoretically be used for treatment of depressive disorders, essentially in persons younger than 45 years, as it is shown herein that in this age category certain time-varying waves cause an increase of suicide death rate, which is an indicator of depressive disorder. In individual cases the Faraday cage may also be efficient in older age categories, however on average, depression is expected to increase rather than decrease when persons of older age categories stay in Faraday cages. Another problem is that when starting a stay in a Faraday cage the situation is transient pro-auto-immune causing a worsening of depressive disorders expected to last about one year. Therefore the stay in a Faraday cage should preferably be a long-term permanent stay and in most cases may become efficient against depressive disorder only after about a year.

The levels of attenuation of the external electromagnetic field which are needed for a Faraday cage to be efficient in treating diseases depend on which disease is being considered, but generally if the spectral power of the artificial electromagnetic waves inside the Faraday cage is at all times below or comparable to thermal spectral power, this is a good guarantee of optimal effects. Accordingly, an aspect of the invention is a building comprising a Faraday cage, for use in the treatment of disease, the Faraday cage and the place where it is installed being adapted so that the spectral power of the electric field due to artificial waves inside the Faraday cage is lower than the thermal spectral power, on at least the 10 MHz to 1 GHz frequency range. According to aspects of the invention, spectral powers of the electric field due to artificial waves inside the Faraday cage can be up to 10 times or even up to 100 or 1000 times thermal spectral power, at the cost of only moderately diminished efficiency in many diseases.

The "thermal spectral power" means the spectral power of the electromagnetic field at equilibrium at the ambient temperature inside the Faraday cage, i.e. about 300K, as given by Planck's law , integrated on a solid angle corresponding to a half space, absent any artificial waves, which can be calculated using Planck's law.

"moderately diminished efficiency" implies that treatment efficiency may be compromised for certain diseases. For example, the threshold spectral power (12h daytime average) for triggering of Creutzfeldt-Jacob disease by GSM is in the order of 50 times thermal spectral power for 3 years average survival time of youngest prion carriers which means roughly 10 times average power for 15 years survival time. Therefore for any attempted treatment of this particular disease the spectral power of the time-varying artificial electromagnetic wave inside the Faraday cage should preferably be brought below 10 times thermal power. The threshold spectral power for the causation of increased suicide death rate by GSM is within the same range as for Creutzfeldt-Jacob disease so that for treatment of depression the spectral power of the time-varying artificial electromagnetic wave inside the Faraday cage should preferably be brought below 10 times thermal power for best treatment efficiency.

For cancer, any increase of the destruction probability Wdest of a cancerous cell by a corresponding T cell determines an increase in the size threshold of controllable neoplasms. If the Wdest of the pre-existing activated T cell lines (with inhibitory receptors) recognizing the neoplasm is near 1, the mere use of a Faraday cage cannot improve the binding capabilities of these existing activated T cell lines, and may even yield to suppression of these existing T cell lines by regulatory mechanisms, which can actually worsen the cancer. But the use of a Faraday cage in this situation can cause new T cells within the existing T cell library (which were thymus-generated under high exposure conditions and are thus abnormally aggressive against the self and the neoplasm under low-exposure conditions inside the Faraday cage) to be activated and to control the cancer. This second effect is expected to be the dominant effect as it implies the entire library of T cell lines. There is not generally a precise threshold for curing cancer and any decrease of the value of permanent fields by a Faraday cage is expected to improve the chances of controlling cancer.

To diminish the risk of a counter-productive excessive reaction of the regulatory mechanisms of the immune system, in one version of the invention the treatment implies a progressive, rather than sudden, diminishing of the external fields. This can be realized with a single Faraday cage which attenuation is adjustable, or with a series of Faraday cages of decreasing attenuation. In this version of the invention, the treatment comprises a series of stays in Faraday cages having different attenuations or in a Faraday cage having a modifiable attenuation, the attenuation of the external field being increased between each two consecutive treatment steps. This treatment can be used for cancer but also for other diseases including AIDS.

Faraday cages having much more limited attenuations than discussed above can however be efficient depending on the precise situation. Persons who become cancerous due to a nearby DVB emitter are likely to recover when using a Faraday cage of more limited attenuation if such Faraday cage is used early enough. However, since cancer is always quite advanced when it is discovered, it is wise to use a Faraday cage having sufficient attenuation to bring down the level of electromagnetic waves to thermal level. Attempting to use a non-optimal Faraday cage may yield to loss of life.

In the case of AIDS, the limit value is expected to strongly vary with the HLA system of the infected patient. In most cases however, the limit value is expected to be one which is rarely obtained in open air in developed countries. In view of the fact that AIDS emergence essentially coincided with changes in Television broadcasting in affected African countries and Haiti, AIDS is expected to be favoured by at least television, most likely television and FM radio. A quick survey in France shows that Television and FM radio field levels rarely go below 1 mV/m or about 1 nW/m2 ($10^{-9}$ W/m2). It is thus likely that the threshold value for AIDS control is below that value. The thermal level for a 16 MHz dual-multiplex DVB signal it is 1 pW/m2 ($10^{-12}$ W/m2) (it is roughly the same for a dual analog TV broadcast). The threshold value for AIDS is thus probably between thermal level and 1000 times thermal level although it may reach higher levels. It is therefore expected to be somewhere between thermal level and 40 dB above thermal level, most likely between thermal level and 30 dB above thermal level. The corresponding power attenuation of a Faraday cage in the FM bandwidth and television bandwidth in an environment where FM radio and TV signals peaks at 1 $\mu$W/m2 ($10^{-6}$ W/m2) is somewhere between 20 dB and 60 dB, probably below 40 dB. Recovery is slown down if there is a reasonable margin between the limit value and the actual field value, so it is preferable to go down to thermal or near thermal level (for example below 10x thermal power) to obtain best recovery chances for all HLA systems. However if a limit value allowing control of AIDS by the immune system alone is not reached, use of a Faraday cage is still expected to improve the chances of recovery or delay death.

The Faraday cage can be used to treat mental disease. Modifying the thickness of the myelin sheath has a direct effect on suicide rates and this implies that it has an effect on mental health generally. The Faraday cage, by protecting the patient from the external electromagnetic field, protects him from mental health problems due to this field and makes it possible to submit the patient to a medical electromagnetic field best suited to his disease.

Generally, in a preferred version of the invention the attenuation of the electric field in the Faraday cage is at least 30 dB in the 10 MHz to 2 GHz frequency range, most preferably 40 dB, even more preferably 50 dB. This ensures a strong contrast with the outside environment which in many cases will yield good results as compared to staying in the outside environment.

More preferably, the attenuation of the electric field in the Faraday cage is superior to 120 dB in FM band and superior to 80 dB in the 110 MHz to 2 GHz band. This ensures below-thermal operation in most commonly encountered operating environments.

A version of the invention is a building which either is entirely enclosed in a Faraday cage and comprises a plurality of rooms, or alternatively comprises a plurality of rooms with each room forming a Faraday cage, preferably with a bed in each room, for use as a place where the patients can make prolonged stays at or near to a hospital. These rooms can be used for treatments as described above, but they can also be used independent of any specific treatment, to reduce the risks associated with infections contracted in the hospital. For example death by pneumonia may often result from hospital-acquired infections. Generally, nosocomial infections are believed to represent a large share of deaths by bacterial diseases. According to the invention, such infections can be largely avoided by allowing the patients to stay in Faraday cages when at the hospital, and preferably allowing them to stay in a Faraday cage, whether part time or full time, for a sufficient time to eliminate infections after any surgical or other invasive treatment.

A version of the invention is a surgery room for practising surgery on patients, which is fully enclosed in a Faraday cage. In surgery operations, the inner parts of the body are left in open air and infection is frequent despite the precautions taken. Using surgery rooms enclosed in Faraday cages will strongly reduce the consequences of such infection, as the immune system will be in better position to react quickly and efficiently. Since electronic apparatuses are often used in surgery rooms, the surgery room preferably comprises an absorber of electromagnetic waves (enclosed in the same Faraday cage as the surgery room) which is adapted to absorb any electromagnetic waves emitted by medical apparatuses.

Preferably, the medical apparatuses used in the surgery room is designed to avoid any un-controlled emissions of electromagnetic waves.

A problem with the use of Faraday cages is that persons using Faraday cages experience an increase of the feelings of tingling and numbness commonly associated with EHS. It is a further objective of this invention to provide a Faraday cage in which a patient may stay part-time, for example at night, whilst at the same time minimizing any increase of the feelings of tingling and numbness associated with EHS.

According to a version of the invention, this problem is resolved by a Faraday cage which as far as possible attenuates higher frequency electric fields (including, for example, GSM900, GSM1800, Wifi, DECT, UMTS) more strongly than lower frequency electric fields (including, for example, FM radio or TV). Preferably it attenuates electromagnetic fields having frequencies corresponding to time-varying artificial electromagnetic waves (including GSM900, GSM1800, Wifi, DECT, UMTS) more strongly than electromagnetic fields having frequencies corresponding to permanent artificial electromagnetic waves (including FM radio or TV). However, since effects on the immune system are essentially due to the electric field, it is not necessary to control the attenuation of the magnetic field as efficiently as the attenuation of the electric field. This low-pass Faraday cage largely suppresses the additional auto-immune effects which result from using the Faraday cage.

In the present situation, most time-varying electromagnetic fields have higher frequencies than permanent electromagnetic fields. Therefore a low-pass Faraday cage can be used which is adapted to attenuate frequencies higher than a high limit more strongly than frequencies which are lower than a low limit. The high limit is preferably below the frequencies of GSM communication networks, Wi-fi and DECT. The low limit is preferably higher than the frequencies of Hertzian Television. For use in France and other countries having similar frequency allocation plans, the low limit can be 790 MHz and the high limit can be 870 MHz. The low pass Faraday preferably attenuates the electric field above the high limit by at least 20 dB more than the electric field below the low limit. Preferably, the electric field below the low limit is attenuated by less than 10 dB, so as to minimize any pro-auto-immune effect. Ideally, the electric field below the low limit should not be attenuated at all. This low-pass Faraday cage preferably encloses a bed and/or is adapted to enclose an adult human being, so as to be usable by human beings.

For example, the Faraday cage comprises at least a first and a second conducting elements, wherein a first surface of the first conducting element is situated in front of a second surface of the second conductive element to form a capacitance between the first and a second surface, to allow some low frequency electric field to enter the Faraday cage. There may be electrical no contact at all between the first and second conducting elements, or there may be some electrical contact. Preferably the low-pass Faraday cage is adapted so that the resistance measured between any two points of the first conductive element and the resistance measured between any two points of the second conducting element are lower than the resistance measured between any point of the first conducting element and any point of the second conductive element.

There are a variety of methods for obtaining such a low-pass Faraday cage. For example, according to a version of the invention, the walls of the Faraday cage comprise a plurality of conducting plates with at least one first conducting plate being in mechanical contact with at least another second conducting plate, the first conducting plate being electrically isolated from the second conducting plate, the contact area and the distance between the two plates being adapted to attenuate high frequencies corresponding to time-varying artificial electromagnetic waves more strongly than lower frequencies corresponding to permanent artificial electromagnetic waves.

This low-pass Faraday cage can be used in all treatments based on the sole use of a Faraday cage, whenever high-frequency fields have abnormally strong powers. It is expected to be particularly useful to persons living near cellular phone base stations and working far from such base stations, because it attenuates the base station signal whilst avoiding any additional pro-auto-immune effect related to attenuation of lower frequency signals, thus minimizing the risk of appearance of a complete EHS syndrom. Similarly, it is expected to be useful to persons whose neighbours use Wifi or DECT, especially persons living in small apartments where distance to neighbour's emitters is small. This low pass Faraday cage is also particularly useful in the treatment of weight excess which is specifically related to GSM. This low pass Faraday cage is expected to be often used at night-time and thus may comprise a bed enclosed in the Faraday cage. The drawbacks of using this low-pass Faraday cage is a diminished efficacy of most treatments, particularly when low frequency waves have a strong spectral power. For example when FM waves have a strong power, the low-pass Faraday cage is little efficient against an infectious disease.

**Emitter of medical electromagnetic waves**

Although the mere protection from electromagnetic waves can help recovery in most cases by suppressing damageable artificial waves, electromagnetic waves can also be used to fine-tune the immune response and certain diseases are better treated using appropriate electromagnetic waves. Therefore, an aspect of the invention is an emitter adapted to generate a medical electromagnetic wave, for use in the treatment of disease. This emitter is preferentially used in an area as little exposed to other artificial electromagnetic waves as is reasonably possible, so as to avoid any un-controlled waves which could affect treatment results. Such area may be the inside of an appropriate Faraday cage, or a geographical area which for other reasons is little affected by electromagnetic waves.

Diseases to be treated using this emitter comprise inflammatory states (whether or not caused by a pathogen, and including both localized inflammations and generalized inflammations), autoimmune diseases, and/or cancers. They also include heart attacks and chronic disease of the liver, at least in certain age and sex categories. I use the words "medical electromagnetic wave" to differentiate the "medical electromagnetic wave" purposedly emitted in order to treat a disease, from the general case of artificial electromagnetic waves emitted for other reasons. Diseases caused by pathogens are not usually curable by using an electromagnetic wave, but inflammation associated to such pathogens can be treated by the medical electromagnetic wave according to the invention, any auto-immune effect associated with a Faraday cage treatment of such pathogen can be treated by the medical electromagnetic wave of the invention, and a medical electromagnetic wave can also be part of a treatment for specific classes of pathogens like cyst-forming spirochaetes. So often an emitter of medical electromagnetic wave is indirectly useful for a treatment against pathogens.

There is no lower bandwidth limit for the medical electromagnetic wave to have some effects. A continuous wave with zero bandwidth will have some effects. A larger bandwidth electromagnetic wave will usually be more efficient because its threshold power is lower than for a cw or low-bandwidth wave.

According to a preferred version of the invention, the medical electromagnetic wave comprises a first part extending in the frequency domain from a first lower frequency limit to a first upper frequency limit and having a non-zero spectral power between the first lower and first upper frequency limits. Preferably the first bandwidth extending between the first lower frequency limit and the first upper frequency limit is larger than 10 MHz, but as previously discussed lower bandwidth, down to cw exposure, are also efficient.

In a preferred version of the invention, the attenuation of any frequency in the first part of the medical electromagnetic wave, relative to any lower frequency within the first part of the medical electromagnetic wave, does not exceed 30 dB. In a version of the invention, the medical electromagnetic wave has a substantially constant first spectral power between the first and second frequency limits. In another version of the invention, the spectral power is increasing with frequency for frequencies lower than the frequency of a maximal spectral power and decreasing with frequency for frequencies higher than the frequency of the maximal spectral power.

However these preferred versions of the invention can be heavily modified whilst essentially keeping the same functionality. For example a medical electromagnetic wave which has a "comb" shape in the frequency domain, including a number of equally spaced carriers, separated by empty parts with zero spectral power, can perform well insofar as the Rabi integration bandwidth of each carrier extends it enough to touch the other carriers. Many signal shapes can be imagined which result in reasonable performance.

In a version of the invention the medical electromagnetic wave is substantially zero below the first lower frequency and above the second frequency, however in alternative versions of the invention these bandwidths can be used for signals that have different and/or complementary effects with respect to the first part of the medical electromagnetic wave. Therefore, in an alternative version of the invention, the medical electromagnetic wave also comprises a second part extending in the frequency domain from a second lower frequency limit to a second upper frequency limit and having a non-zero second spectral power between the first and second frequency limits, the second spectral power being different from the first spectral power. This alternative version of the invention can be applied for example to generate a medical electromagnetic wave which power spectrum mimics the power spectrum of electromagnetic waves in the normal environment of a patient, for use in a treatment against auto-immune diseases.

Preferably, at least the first part of the medical electromagnetic wave is a random or pseudo-random gaussian noise. Emitters of large band gaussian noise which are programmable to apply a filter on the gaussian noise to select or attenuate certain bandwidths are known per se.

In practical applications, it is thus reasonable to use a medical electromagnetic wave which is a flat gaussian noise over the bandwidth extending between the first lower frequency limit and the first upper frequency limit. However this is not the only option. For example Lakhovsky's emitter (the Multiple Waves Oscillator) emitted a wave did not have a constant spectral power and which was not a random gaussian noise, yet this emitter did cure cancers.

Preferably, the emitter is adapted to generate an electromagnetic wave which has non-zero mutually independent electric field components along three mutually orthogonal directions. This is because the Rabi frequency is proportional to the interaction hamiltonian of equation so that if the electric field is directed along a constant direction, the Rabi frequencies will vary with the orientation of the TCR-pMHC relative to the field. Such variations may result in the TCR-pMHC being little affected by the medical electromagnetic wave when having specific orientations. Such variations are limited due to the fact that the TCR-pMHC system has some degrees of rotational liberty. Yet any such variation results in a degradation of the treatment efficacy. This degradation is to be avoided, particularly for treatment of auto-immune disease because in the treatment of auto-immune diseases it is of some importance that all TCR-pMHCs of a disease-causing kind be affected by the electromagnetic wave. Most preferably, these E-field components of the medical electromagnetic wave along each of the three mutually orthogonal direction are mutually independent gaussian noises. Preferably, the components of the electric field on three mutually orthogonal directions in a place where the patient is allowed to stay having the same time-averaged spectral power. Preferably, the components of the electric field on three mutually orthogonal directions being simultaneously turned on and off.

Applying the medical electromagnetic wave to a patient in case of an auto-immune disease results in rendering inefficient the T cell lines causing the disease, thus stopping disease progression. However the disease can be restarted by other T cell lines at a later point in time. To avoid this, in a version of the invention the power of the medical electromagnetic wave is further increased during the treatment, so as to render inefficient any new line of T cells which would otherwise re-start the disease. The power increase may concern the whole frequency spectrum or be localized on certain limited bandwidths.

Experimentally, the consequences of the DVB-T signal show that more significant effects on cancer (and correspondingly on autoimmune diseases) are obtained using larger bandwidth.

To obtain best results in treating cancer and autoimmune diseases, a higher bandwidth of the medical electromagnetic wave is thus required. Therefore, in a further improved version of the invention, the said first bandwidth is at least 10 MHz.

A bandwidth higher than 60 MHz is expected to have an effect on at least certain cancers for men aged less than 45, since there was a pro-cancer effect in Paris for this age category , and analysis of the signal changes in Paris shows that a single bandwidth of 60 MHz should be sufficient to obtain the same results. But lower bandwidths do impact some cancers as well and higher bandwidths may increase the number of responsive cancers.

An overall power of less than 5 μW/m2 (5.10$^{-6}$ W/m2) had a proven effect on at least certain patients in the 35-44 years age range in Pontchâteau and the overall power was probably lower in Paris. Therefore a power above 5 μW/m2 is expected to have some pro-cancer effect on at least certain cancers. Whilst applying this signal permanently would kill certain patients, applying it part time will shift the autoimmune/cancer balance towards autoimmune, according to Table 1. However uncertainties as to the level of efficiency of a treatment using this signal exist. The efficiency of this signal may be limited to a relatively limited number of patients and the obtaining of really good results may require a long treatment and/or long exposure periods. Since the above signal is far from acceptable norms, it is possible to apply a much stronger power, which will from the start ensure better and more generalized results on cancer treatment and allow shorter exposure times.

According to the invention, the overall power of the medical electromagnetic wave is preferably superior to 1 W/m2. However the strongest results may be obtained by using powers which are stronger, insofar as the power does not cause excessive heating of the body. The limit for heating is typically about 10 W/m2. Therefore, according to the invention, the emitter of electromagnetic wave is preferably able to generate a medical electromagnetic wave having an overall power superior to 0.1 W/m2. On the other hand, it may also be possible, particularly on patients living in little-exposed areas, to have an efficient anti-cancer effect for overall powers lower than 1 μW/m2.

the power threshold is roughly proportional to the square root of the bandwidth. Also, some power which it emitted below or above certain frequency limits may be lost if the T cells to be generated have frequency conditions excluding such frequency ranges. Therefore increasing the bandwidth does not systematically yield an increase of anti-cancer efficacy. It can diminish the ability to induce the thymus in generating agressive T cells with low Δb or limited frequency conditions, and thus be counter-productive. Additionally it can yield unnecessary side effects. Preferably, the emitter of electromagnetic wave is thus programmable to modify the first lower frequency limit and the first upper frequency limit.

Exposing patients to uselessly strong waves yields potential side effects in the form of triggering auto-immune illness. Therefore, the emitter of electromagnetic waves is preferably programmable to modify the power of the medical electromagnetic wave.

For treatment of cancer, preferably before full involution the thymus, the emitter can be used as specified in Table 1 to provoke a pro-autoimmune effect by alternating periods of exposure with periods of non-exposure. In a version of the invention, a single period of exposure can be sufficient, preferably lasting less than 24 hours. In a preferred version of the invention, the emitter is adapted to turn on or off the medical electromagnetic wave according to a predefined time sequence. Preferably it is programmable, to adapt the predefined time sequence to specific patients. Preferably, the time sequence lasts at least one week. Preferably, the time sequence comprises daily exposures of 10 minutes to 4 hours each. Using a time sequence allows more time for the immune system to deal with any T cells having autoimmune effects and to activate T cells having anti-cancer efficiency, and minimizes the risk of "overflow" of the immune system generally.

Over a long period of time, the alternance of exposure periods and non-exposure periods results in the medical electromagnetic wave to appear as a slowly pulsed waveform, with pulse length in the order of a few minutes and repetition rates in the order of a few days typically. The use of shorter pulses with higher repetition rate may be advantageous for certain cancers as it allows higher peak powers to be used without any heating effects. During a power peak, negative selection in the thymus is more fully prevented - but it is also prevented less often due to the shortness of the pulse. For example a pulsed wave can be tried when a cancer does not respond to the normal treatment with a constant gaussian noise. Such pulsed wave can be a periodically or non-periodically repeated pulse. During each pulse, the medical electromagnetic wave is preferably a randomor pseudo-random gaussian noise. Pulse length should preferably be longer than a typical TCR-pMHC interaction time in the thymus, to allow the medical electromagnetic wave to cancel negative selection steps during each pulse. When short pulses are used for a treatment, they may be repeated during an overall exposure period of typically less than one hour, with this overall exposure period being itself repeated a few times, for example once a day or once a week until positive effects of the treatment become detectable.

The power of the medical electromagnetic wave is limited in order to not cause any excessive direct heating of the patient. More generally, excessive powers may trigger unexpected side effects. For a constant power of the medical electromagnetic wave, the spectral power is lower if the bandwidth is higher, potentially reducing the effects on cancers when the $\Delta b$ of implied TCR-pMHCs is low. On the other hand, an insufficient bandwidth reduces the impact on cancers when the $\Delta b$ of implied TCR-pMHCs is high. To overcome this limitation and maximize the impact on all cancers, in a version of the invention the emitter is programmable to generate a time sequence of exposures comprising periods of exposure with a lower first bandwidth of the medical electromagnetic wave and periods of exposure with a higher first bandwidth of the medical electromagnetic wave. Preferably, the spectral power is increased during the periods of exposures that use the lower bandwidth relative to the spectral power used for periods of exposure that use a higher bandwidth. Preferably the overall power of the medical electromagnetic wave is maintained substantially constant as the bandwidth is modified. This method should further increase the number of responsive cancers.

For treatment of autoimmune diseases, the emitter can be used to create a stable electromagnetic environment in which the autoimmune disease is stopped. Since the autoimmune disease is likely to have been triggered due to alternance between strong and low electromagnetic field in the daily life of the patient, a strategy for suppressing this autoimmune disease is to use a medical electromagnetic wave which has a spectral power reproducing the maximum spectral power encountered by the patient in daily life. Therefore, according to a version of the inversion particularly adapted to this treatment of autoimmune diseases, the emitter is programmable to generate a medical electromagnetic wave having a predefined spectral power profile. Preferably this predefined spectral power profile mimics the spectral power of waves encountered in the normal environment of a patient, but is substantially constant over time. Preferably the spectral power on each frequency is the maximum value of the spectral power on this frequency which the patient encounters in a typical day in his normal environment. In case this constant spectral power does not impact the auto-immune disease, it can be increased within the limits of reasonableness. If a relatively short term response is obtained, it is then possible to modify this spectral profile by suppressing certain types of emissions like FM, DVB, or GSM and observe modifications in the patient's response to the treatment, so as to identify which artificial wave or combination of artificial waves caused the disease and should be used to treat it. Ideally the power spectrum should be reduced as much as possible in both occupied frequencies and power level whilst still maintaining the efficiency of the cure, to the point of having only the first part of the electromagnetic wave, extending over a first bandwidth which is not uselessly extended. It is useless and even potentially dangerous as in the long term it may worsen the illness and cause cancer, to use a power spectrum which is wider and/or stronger than is strictly necessary. For treatment of an autoimmune disease it is sometimes necessary for the patient to undergo treatment during the whole of his remaining lifetime. However, in some cases, once the T cell line causing the disease ceases to bind elements of the self due to the application of the medical electromagnetic wave, then after some time this T cell line may be suppressed by positive selection mechanisms elsewhere than in the thymus, for example in lymph nodes, for example implyig regulatory T cells. In this case the treatment can be stopped. The difficulty in determining whether the treatment can be stopped or not is that stopping the treatment too early can re-activate the T cell line in question which proliferates again, so that any final recovery is strongly delayed. Therefore the treatment should preferably not be stopped or attempted to be stopped before a typical time for such positive selection to take effect.

For treatment of inflammations or autoimmune disease, it can be useful to irradiate specifically an area of interest within the human body. In a version of the invention adapted to local treatment of inflammations or autoimmune diseases, the emitter comprises a signal generator connected to an antenna or to electrodes for emitting the medical electromagnetic wave in free space or directly in the human body. In this version of the invention, the antenna or electrodes are adapted to be placed near to the human body and, in the case of electrodes, in electrical contact with the human body. They are further adapted to emit a medical electromagnetic wave having an electric field which is stronger in the area of interest than in the remainder of the human body. For example, the area of interest may be the vertebral column, for treating pain in the vertebral column; any joints, for treating joint pain generally; the stomach, for treating certain inflammatory states of the stomach area; the head, for treating Creutzfeldt-Jacob disease. Accordingly, an aspect of the invention is an emitter and a mechanism for fixing this emitter on the head, to selectively expose the head to electromagnetic waves. If this emitter and mechanism are to be used outside a Faraday cage, then preferably the mechanism for fixing the emitter on the head also includes conductive elements adapted to protect the neighbourhood from the waves emitted by the emitter - although such protection is necessarily imperfect.

For treatment of depression or other mental problems certain patients need a pro-auto-immune environment in order to diminish the thickness of their myelin sheaths. These patients can be submitted to a time varying electromagnetic wave inside the Faraday cage. Other patients need an anti-auto-immune environment in order to thicken their myelin sheaths. These patients can in principle stay permanently in a Faraday cage without other electromagnetic waves. However in order to facilitate the initial period immediately after entering the Faraday cage, the patient can be submitted to a permanent field mimicking the constant fields that are present in the outside environment. This avoid the transient pro-autoimmune effect immediately after entering the Faraday cage. Generally, patients aged more than 50 years are more likely to need of an auto-immune effect whilst patients aged less than 50 years are more likely to need an anti-auto-immune effect, but this is not systematic.

Generally and irrespective of the specific disease being treated, there is a tradeoff between efficiency of the anticancer treatment and increased risk of autoimmune diseases (when treating cancer) and between increased cancer risk and efficiency of the treatment (when treating autoimmune diseases or inflammations). Therefore, the power of the medical electromagnetic wave should not be increased unnecessarily. The medical electromagnetic wave should be a reasonable compromise between positive and negative effects in view of the disease to be treated. In a version of the invention the first bandwidth and/or the first spectral power and/or the predefined time sequence of the medical electromagnetic wave are optimized by tests on animals before being applied on humans. In a version of the invention, the first bandwidth and/or the first spectral power and/or the predefined time sequence of the medical electromagnetic wave are further optimized by medical practice. An aspect of the invention is a database comprising results of applying the treatment to humans and including for each case the details of the medical electromagnetic waves applied to the patient, any useful details of the treatment and the medical condition of the patient, and treatment results. Such database is expected to allow practitioners to use the best characteristics of the medical electromagnetic wave as applied to each specific case. Such database may be provided on a computer via a suitable visual interface.

The effects of a medical electromagnetic wave depend on:

a) the patient's HLA system, because it determines the characteristics of the TCR-pMHC bound,
b) the patient's age, because it determines a rate of output of naive T cells by the thymus,
c) the specific disease to be treated, because it determines whether a new line of T cells must be produced (in the case of cancer) or an existing line of T cells must be rendered inoperative (in the case of autoimmune disease) and further it determines which specific line of T cells and thus TCR-pMHC bound needs to be produced (in the case of cancer) or rendered inoperative (in the case of autoimmune disease).

All these elements in turn influence the optimal frequency, bandwidth, power and time sequence of the medical electromagnetic wave as well as the requirement for a local or generalized application of the medical electromagnetic wave. Therefore, although a generic medical electromagnetic wave is efficient, the parameters of the medical electromagnetic wave should ideally be specifically adapted to each set of values of the parameters (a) (b) (c) so as to optimize the treatment and minimize any adverse effects.

Accordingly, in a version of the invention, the treatment comprises a diagnostics step to identify the disease to be treated (including type and location of cancer, autoimmune disease or inflammation) and/or the HLA system of the patient and/or the age of the patient, and a treatment step in which the medical electromagnetic wave is emitted towards the patient to be treated, wherein the first lower frequency limit and/or first upper frequency limit and/or first spectral power and/or time sequence depend on the identified disease and/or on the HLA system and/or the age of the patient.

In a version of the invention, the emitter is connected to a computer adapted for allowing the entry of the indication of the HLA system and/or type of disease and/or age of the patient by an operator and for controlling the emitter so as to emit a medical electromagnetic wave having optimized first lower frequency limit and/or first upper frequency limit and/or first spectral power and/or time sequence corresponding to the specific HLA system and/or autoimmune disease and/or age of the patient entered by the operator.

A medical electromagnetic wave can be dangerous. In case the treatment implies a sequence of exposure and non-exposure, there is a risk of causing an autoimmune disease. Therefore, according to a version of the invention in which the treatment comprises a sequence of exposure or non-exposure, the treatment further comprises regular diagnosis of autoimmune diseases. If any such autoimmune disease is diagnosed, the treatment should preferably be modified to diminish the power of the medical electromagnetic wave and/or to diminish the duration of each exposure period; or the treatment may be interrupted.

In case the treatment implies a permanent exposure, there is an increased risk of cancer during the treatment and an increased risk of autoimmune disease immediately after the treatment. Therefore, according to a version of the invention in which the treatment comprises a permanent or near-permanent exposure, the treatment further comprises regular cancer diagnosis. If any cancer is diagnosed, the permanent exposure should preferably be interrupted and the cancer should preferably be treated in priority whether by stay in a Faraday cage or by application of a suitable electromagnetic wave or both, or by use of other known treatments against cancer.

All forms of use of a medical electromagnetic wave can cause an uncontrolled multiplication of lymphocytes at least in a transient period. Therefore in a preferred version of the invention the treatment comprises a follow-up of the number of T and B lymphocytes in blood. Treatment can be continued or modified or interrupted depending on the importance of the excess of lymphocytes in blood.

In the specific case of autoimmune symptoms, these can be eased very quickly by using the emitter to generate a permanent background level at a power well which is substantially less than the power used during the cancer-treating exposure. This is not however a recommended solution in the point of view of overcoming cancer as it may have the effect of allowing a cancer to develop again. It is better to reduce the daily cancer-treating exposure power, but if quick relief from an autoimmune or inflammatory problem is needed, using a background level is the only practical solution.

One risk of the treatment - which also exists with exposure to non-medical artificial electromagnetic waves - is to provoke a situation in which both an autoimmune illness and a cancer co-exist. In such case the treatment by medical electromagnetic wave should be carefully adjusted and generally it should be chosen to fight cancer as a priority until full disappearance of such cancer, and the autoimmune illness may be fought against by other means. This sort of situation is to be managed on a case by case basis.

Other diseases which can be treated with the emitter of electromagnetic waves comprise acute myocardial infarction, cardiomyopathy, alcoholic liver disease. Generally, statistics of the reaction of a population to a modification of exposure conditions make it possible to determine which diseases and patients (i.e., non-limitatively, age/racial/sex groups) respond to a change of exposure conditions (whether by an increase or a decrease of death rate). These diseases and age groups are most likely to respond well to an appropriate treatment by an appropriate medical electromagnetic wave. The treatment should be reasonable in view of the response as determined by the statistics, but generally it is necessary to refine treatment conditions by trials on animals and clinical trials on humans.

Preferably the medical electromagnetic wave is phase and/or amplitude modulated according to a random or a pseudo random sequence of bits. Most preferably the medical electromagnetic wave is a gaussian or pseudo gaussian noise, as this creates ideal conditions for an efficient and reproducible effect of the medical electromagnetic wave.

The antenna through which the medical electromagnetic wave is emitted by the emitter can be of any known type. However, it can also be made of two plates disposed in a manner adapted so that the patient or any specific area of the patient's body can be placed between the two plates, a voltage difference being applied between the two plates by the signal generator assembly of the emitter. This configuration ensures a better uniformity of the irradiation power together with less losses. This configuration is most efficient in this respect when the wavelength of the medical electromagnetic wave is large relative to the separation between the plates.

Frequencies below 10 MHz do not propagate well in the human body but can be efficiently applied through electrodes in direct contact with the body, to provide sufficient electrical currents to maintain the current in the body. Thus, according to a version of the invention, the first upper frequency is below 50 MHz, preferably below 10 MHz, and the emitter comprises a signal generator connected to electrodes adapted for being in electrical contact with the human body.

Frequencies higher than about 80 MHz propagate well in the human body. Thus, according to a version of the invention, the first lower frequency is higher than 40 MHz, preferably higher than 80 MHz, and the emitter comprises a signal generator connected to an antenna adapted to radiate the electromagnetic wave in free space.

An improved emitter of electromagnetic wave for creating an anti-auto-immune effect is adapted so as to increase the power of the medical electromagnetic wave during the treatment. The anti-auto-immune effect of Table 1 column C is transient, and increasing the power of the medical electromagnetic wave during the treatment allows a longer anti-auto-immune effect. Such improved emitter can be used to cure auto-immune diseases, to delay death in auto-immune diseases, or for providing relief in certain mental diseases by increasing the thickness of the myelin sheath.

The emitter of medical electromagnetic waves can also be used in organ transplantation to minimize transplant rejection. Preferably, the emitter should in this case be installed so as to maximize exposure of the transplanted organ whilst at the same time minimizing exposure of other organs and more specifically of the thymus, so as to make T cells unable to recognize the transplanted organ (which is highly exposed) whilst still allowing them to function normally in other organs, and so as to not cause cancer by thymus exposure.

Known emitters of electromagnetic waves usually emit the electromagnetic wave from a single point of emission which is typically a dipole antenna. The Lakhovsky emitter has two antennas which are placed on each side of a patient to be treated. However these two antennas receive essentially the same signal, resulting in the formation of an interference pattern between the two antennas, which reduces the homogeneousness of exposure. The treatment using the medical electromagnetic wave, in case of a whole-body exposure, would be more reliable and predictable with a more homogeneous exposure. Using two electromagnetic waves having distinct propagation directions when reaching the patient can improve homogeneousness of the patient's exposure but should be done so as to avoid the formation of a stable interference pattern between the waves. Thus in a version of the invention adapted for whole-body exposure, the emitter of medical electromagnetic waves is adapted to emit a first and a second electromagnetic waves that have different propagation directions when reaching the person to be treated, and the first and second electromagnetic waves are adapted to not form a stable interference pattern. For example the emitter may comprise two sub-emitters emitting mutually decorrelated noises. More than two electromagnetic waves can advantageously be used and the emitter is thus preferably adapted to also emit a third electromagnetic wave adapted to not form a stable interference pattern with the first and/or second electromagnetic waves.

## Jammer

The present section is concerned with the prevention and treatment of diseases caused by time-varying waves when it is not physically possible to use a Faraday cage. For example, it may not be practical to use a Faraday cage because a large geographical area must be protected or because the persons who benefit the treatment are unwilling to spend their life inside a Faraday cage.

According to the invention, this problem is solved by using at least one jammer to reduce the incidence or seriousness of diseases caused by a time-varying artificial electromagnetic wave in a Protected Area. The diseases which incidence or seriousness can be reduced in this manner are auto-immune diseases or diseases which have an auto-immune aspect. When the thermal power can be neglected as compared to the power of the artificial wave, the ability of a time-varying artificial wave to cause auto-immune diseases depends on the value Pmax/Pmin wherein Pmax is the maximum average power of the signal and Pmin is the minimum average power of the signal, both averages being over a reasonable time which corresponds to the duration of at least some TCR-pMHC interactions. Abnormally aggressive T cells selected by the thymus under a power Pmax of the artificial wave cause auto-immune damage when the power goes down to Pmin. The jammer acts by creating a constant background signal so that the Pmax/Pmin of the artificial wave (now including both the time-variable artificial wave and the jamming signal occupying the same frequencies) is reduced. Ideally when Pmax/Pmin=1 there is no more pro-auto-immune effect. This ideal situation would require a very strong jamming signal which is undesirable since it would cause other problems such as cancer for people arriving in the Protected Area.

Preferably, the jammer is adapted to generate a jamming signal having a power spectrum which is constant over time. Preferably, the jamming signal occupies the same frequencies as the existing time-variable signal, so as to affect the same TCR-pMHC systems which are affected by the time-variable signal. The power of the jamming signal, as measured on each frequency occupied by the time-variable signal in W/m2 in a Protected Area which is to be protected from the pro-auto-immune effects of the time-varying signal, is preferably higher than 1/10th the peak power of the time-variable signal on the same frequency, so as to obtain a Pmax/Pmin which is less than 10.

Preferably, the power spectrum value of the jamming signal in the Protected Area on any frequency occupied by the time-variable signal is at least equal to the peak power spectrum value of the time-variable signal on the same frequency, so as to obtain a Pmax/Pmin of less than 2. For example the GSM signal typically occupies 125 uplink frequency sub-carriers and 125 downlink frequency sub-carriers. The power of the jamming signal on each of the sub-carriers is preferably equal to at least 1/10th the peak power of the GSM signal on the same sub-carrier, and most preferaby at least the power of the GSM signal on the same sub-carrier. The jammer or jammers should preferably be arranged to generate signals which when reaching the Protected Area have substantially the same directions as the signals due to the time-variable emitters from which protection is attempted.

Diseases to be prevented or treated by stays in the Protected Area or preferably by permanently living in the Protected Area include Creutzfeldt-Jacob disease, multiple sclerosis, depressive disorders, and other auto-immune problems. Such diseases may also include certain influenza epidemics having an auto-immune aspect or Lyme disease. They may further include other diseases or health problems. They may further include other diseases caused or favoured by time-varying signals.

The signal emitted by the jammer (jamming signal) is a particular medical electromagnetic wave aimed at curing the health problems caused by the time-variable artificial wave. The presence of the jamming signals increases the risk of dying from infectious diseases which do not mimic the self. A cancer outbreak may be produced in the Protected Area when initially setting up the jamming signal. The presence of the jamming signal also results in an increased cancer risk for persons migrating into the Protected Area. These risks are minimized by avoiding any uselessly high values of the jamming signal. Therefore, according to the invention the power of the jamming signal as measured on each frequency occupied by the time-variable signal in W/m2 in a Protected Area which is to be protected from the pro-auto-immune effects of the time-varying signal, is preferably less than 10 times the peak power of the time-variable signal on the same frequency.

In the case of depressive disorders the at least one jammer is expected to have a positive effect on essentially younger patients as it is shown herein that in younger patients suicide rate (an indicator of depressive disorder) is increased by time-varying waves. In older patients the effect is uncertain and there may even be a negative effect, i.e. an increase of depressive disorders, because it is shown herein that at least certain transient anti-auto-immune situations do favour depressive disorder in older patients. Therefore, concerning the treatment of depressive disorder, where a mixed population is present in the Protected Area, it is expected that the younger age classes (typically less than 45 years) will be benefit from a reduction in incidence of depressive disorders but the older age classes will not on average benefit from a reduction in incidence of depressive disorders. If the Protected Area is a specialized institution for the treatment of depressive disorders, that institution should preferably be specialized in treating younger patients. Therefore, a version of the invention is a Protected Area adapted to receive patients, preferably below 45 years of age, for treatment of depressive disorder, the Protected Area being protected from the pro-auto-immune effects of time-variable waves by jammers.

The inconveniency that the depressive disorders may increase for the older population can be eliminated by submitting older residents of the Protected Area from time to time to a medical electromagnetic wave having a higher power than the permanent electromagnetic waves present in the Protected Area. In order to not induce health problems in the other residents, this should preferably be done inside a Faraday cage. Accordingly, a version of the invention comprises jammers for reducing auto-immune problems in a Protected Area and an emitter of medical electromagnetic waves enclosed in a Faraday cage for treating depressive disorders in older residents of the Protected Area.

Preferably, the Protected Area also has a generally low power of artificial electromagnetic waves as this maximizes the chances of the immune system to overcome infectious diseases, including diseases that cause death and diseases that cause less major inconveniences.

However a Protected Area with a power of artificial electromagnetic waves which is comparable to the normal environment of patients to be treated may be desirable in order to avoid the transient pro-auto-immune effect of a transfer to a Protected Area having unusually low power of electromagnetic waves. This avoidance may be generally desirable in the case of serious auto-immune problems. Thus, whilst a low-power Protected Area is generally a good way to simultaneously prevent auto-immune diseases and infectious diseases, where a serious auto-immune disease is already existent it is ideally preferable to use an electromagnetic field specifically adapted to each patient being treated.

**Faraday cage in combination with an Emitter of electromagnetic waves.**

Use of a Faraday cage and use of an Emitter of electromagnetic waves can be efficient against a number of diseases. However, to take a simple example, it is unlikely that Lakhovsky's "multiple waves oscillator" would work as efficiently today as it did in the 1920s. This is because the device relied not only on the medical electromagnetic waves which it generated, but also on the long period of near thermal conditions which separated each exposure to the medical electromagnetic wave. These conditions do not exist anymore so it is preferable today to use a Faraday cage for an efficient and optimal treatment of cancer using an Emitter of electromagnetic waves.

The same is true for treatment of autoimmune illnesses which needs a stable electromagnetic environment which cannot be reached in the presence of numerous time-dependent artificial waves. The same is true for treatment of inflammatory states, since non-controlled artificial waves can increase pathogen proliferation beyond what can be tolerated for the treatment.

For treatment of pathogen-induced diseases, if an autoimmune reaction or inflammation independent of the pathogen is triggered by using the Faraday cage, it can also be necessary to control such reaction by using a controlled artificial wave inside the Faraday cage.

Generally, it is desirable to fully control the electromagnetic environment of a patient for an optimized treatment of disease, and accordingly an aspect of the invention is an installation for use in the treatment of diseases, comprising a main Faraday cage and an emitter adapted to generate electromagnetic waves.

In this installation, appropriate exposure or non-exposure can be applied to optimize treatment. Preferably, the emitter is enclosed in the main Faraday cage. This is because it is desirable to control the exposure rather than superimpose a medical exposure on a perturbating exposure due to a crowded electromagnetic spectrum in open air. If the emitter is outside a Faraday cage, the patient may stay for relatively long time periods in the Faraday cage and come to the emitter area of the installation from time to time to undergo exposure sequences. If the emitter is inside a main Faraday cage, the patient may make stay in a separate Faraday cage for relatively long time periods and come into the main Faraday cage from time to time to undergo exposure sequences. Accordingly, the installation may comprise at least one secondary Faraday cage, adapted for prolonged stays of the patients between exposure sequences. However the emitter may also be installed in a Faraday cage in which the patient stays for long time periods. In this case the patient stays in the Faraday cage whilst he is continuously exposed or alternatively, as he undergoes alternating sequences of exposed and non-exposed periods.

Enclosing the emitter into a Faraday cage is important for improving the treatment by suitably controlling the electromagnetic environment generally. However it is also important in order to avoid submitting persons other than the patient to the medical electromagnetic waves. Medical electromagnetic waves can have unintended side effects on persons who do not need them (including triggering autoimmune diseases like multiple sclerosis). In particular, doctors using an emitter of medical electromagnetic waves should preferably be protected from its emitted waves. Enclosing the emitter of medical electromagnetic waves in a Faraday cage ensures protection of the doctors who control it. If used at home, enclosing the emitter of electromagnetic waves in a Faraday cage ensure protection of the family and neighbours of the person being treated. Further, a non-enclosed emitter of medical electromagnetic waves will have pro-autoimmue effects (if not permanently working) or pro-cancer effects (if permanently on) resulting in an effect on death causes and mortality which is far reaching geographically and potentially strong, like the effects of DVB on cancer or the effects of GSM on multiple sclerosis. Enclosing an emitter of electromagnetic waves in a Faraday cage is thus a matter of public health going beyond the sole relation between a patient and his doctor.

Therefore, in a preferred version of the invention the emitter and the Faraday cage are arranged to that the medical electromagnetic wave is present inside the Faraday cage, and the Faraday cage is adapted to shield the patient to be treated from at least certain artificial electromagnetic waves not emitted by the emitter, and/or to shield the outside of the Faraday cage from the medical electromagnetic wave. Said shielding need not be complete: insofar as for at least some frequencies the electromagnetic waves not emitted by the emitter are prevented to be as strong inside the Faraday cage as outside, there is some shielding and the Faraday cage contributes to a better treatment. Insofar as for at least some frequencies the medical electromagnetic wave is attenuated before reaching the outside of the Faraday cage, the Faraday cage improves the protection of the doctors and the general public.

Protection of the general public from the medical electromagnetic wave is largely characterized by how much the power of the medical electromagnetic wave in W/m2 (Watts per square meter) is attenuated outside the Faraday cage, as compared to inside the Faraday cage. Preferably, the power of the medical electromagnetic wave is at least 20 dB weaker outside the Faraday cage than in a place adapted to receive the patient to be treated inside the Faraday cage. But a better shielding is desirable and ideally the Faraday cage should attenuate the medical electromagnetic wave to such point that its spectral power outside the Faraday cage is less than thermal power.

Any disease which causes a reaction of the immune system, or which would cause such a reaction in the absence of artificial electromagnetic waves, or which is caused by artificial electromagnetic waves, or which is caused by the immune system, can be treated by the combination of an emitter of medical electromagnetic waves and a Faraday cage as described herein. This includes virtually all diseases since all diseases imply the immune system at some point. Even diseases that do not imply the immune system directly, like some hereditary diseases, generally imply it indirectly as the functioning of the immune system somehow impacts the evolution of the disease. The treatment comprises modifying the electromagnetic environment dependent of the exact disease to create anti-autoimmune conditions, anti-cancer conditions, or anti-pathogen conditions or other appropriate conditions as the case may be, based on the effects of electromagnetic waves on the immune system as described in Table 1 and occasionally on other effects of electromagnetic waves. A suitable mix of these conditions may also by used, for example to treat an infectious disease whilst minimizing any pro-autoimmune effects of the treatment. Diseases independent of the immune system can also be treated by creating an electromagnetic environment which has a therapeutic effect through other biological pathes than the immune system. An example of such disease is the excess of weight problem which can be treated by suppressing at least the GSM artificial waves.

The emitter can be any emitter capable of emitting an electromagnetic wave adapted to interact with the immune system. This encompasses previously known emitters like the Lakhovsky multiple wavelength oscillator. It also encompasses emitters having any of the characteristics discussed in the above section "Emitter of medical electromagnetic waves". The Faraday cage can be any enclosure adapted to shield the inside of the enclosure from electromagnetic waves present outside the enclosure, and/or to shield the outside of the enclosure from electromagnetic waves present inside the enclosure. It may for example be made up of metal sheet and/or wire mesh. Optionally the Faraday cage can have any of the characteristics defined in the above section "Faraday cage".

Treatments for specific diseases using the combined Faraday cage and Emitter of Electromagnetic waves of the present section can be as described in the above sections "Faraday cage" and "Emitter of medical electromagnetic waves"

In one version of the invention, the installation is adapted to obtain a substantially constant power of irradiation by the electromagnetic wave generated by the emitter in a treatment area adapted to receive a patient. This is because the power of the electromagnetic waves is an essential parameter in the success of a treatment, and inhomogeneous irradiation may result in aberrant behaviour of the immune system in overexposed or underexposed parts of the body.

In another version of the invention, the installation is adapted to specifically irradiate a specific part of the body.

For example in the treatment of inflammation or localized auto-immune diseases, it may be desirable to reduce the immune response in the diseased part of the body without endangering the body globally by reducing the immune response globally. This result is obtained by selectively irradiating target areas of the body.

In both cases it is preferable to avoid reflections on the walls of the main Faraday cage and the appearance of stationary wave patterns that create inhomogeneous and/or un-controlled irradiation zones. Therefore the installation preferably comprises absorbers of the electromagnetic waves emitted by the emitter, said absorbers being enclosed in the main Faraday cage, to avoid excessive buildup of electromagnetic fields inside the main Faraday cage enclosing the emitter. Amongst other, buildup of electromagnetic fields inside the Faraday cage yields to electromagnetic fields at discrete resonant frequencies of the Faraday cage being amplified by constructive interference, whilst electromagnetic fields at other frequencies are attenuated by destructive interference, thus causing the electromagnetic field inside the cage to have a spectral power curve forming of a spectrum of lines. However, theory shows that an electromagnetic wave having a broadband spectrum is more efficient in affecting the TCR-pMHC recognition than an electromagnetic wave having a line spectrum. Such line spectrum should be avoided, which implies that the excessive buildup of fields inside the Faraday cage must be avoided, which can be done by using absorbers enclosed inside the Faraday cage.

Preferably the emitter is enclosed into an absorbing cage, the absorbing cage being itself enclosed in the main Faraday cage, to best avoid field inhomogeneities inside the main Faraday cage. This double enclosure system avoids any direct reflections on the walls of the Faraday cage. Ideally, the absorbing cage together with the main Faraday cage forms an anechoic chamber.

In a version of the invention the emitter comprises an antenna for emitting the electromagnetic wave and a signal generator of appropriate frequency and bandwidth, connected to the antenna. The electrical power for powering the emitter can be taken from a battery, to avoid power connections between the inside and outside of the cage. Power can also be provided by alternative means, like laser light impinging on a solar cell for example. In a version of the invention,the signal generator, the battery if there is one, and any other electrical apparatus except the antenna, are separately enclosed inside an intermediate Faraday cage. The walls of the intermediate Faraday cage can be connected to one of the battery connections if there is a battery, and to the outer conductor of a coaxial cable connecting the signal generator to the antenna. Alternatively the antenna may protrude directly from the intermediate Faraday cage without an intermediate cable connection. The intermediate Faraday cage should preferably comprise an absorber of electro-magnetic waves enclosed in it. The intermediate Faraday cage is itself enclosed in the main Faraday cage. Alternatively, it can be partly or entirely part of the main Faraday cage, insofar as the inner part of the main Faraday cage remains isolated from the outer electromagnetic fields and from any spurious fields generated by the signal generator. The connection between the antenna and the signal generator may comprise an analog filter suitably placed to filter out unwanted frequencies and ovoid leaks of unwanted frequencies into the main Faraday cage. The grounds of all electrical apparatuses enclosed in the intermediate Faraday cage are preferably connected to the intermediate Faraday cage. These precautions minimize the spurious, unwanted emissions from the emitter, and favour better control of the electromagnetic environment. The intermediate Faraday cage, and generally any Faraday cage in this invention can be of any shape, including shapes in which the battery is separate from the signal generator and connected to it by coaxial cables the outer conductor of which can be considered as a part of a Faraday cage.

The exposure near to the antenna is much stronger than the exposure far from the antenna. It is generally desirable to avoid excessive exposure of the patient. In particular, if the treatment is against autoimmune disease, any change of the field power reaching the patient is damageable. Therefore the patient should be prevented from accidentally coming near to the antenna and should even be restrained to such part of the Faraday cage which is far from the antenna. For this reason the Faraday cage can comprise an internal wall (not shown) separating the patient from the emitter of electromagnetic waves. This internal wall must be permeable to the electromagnetic waves emitted by the emitter. Instead of a wall, any physical barrier preventing the patient to come near to the antenna can be used. The Faraday cage should preferably be sufficiently large so that the medical electromagnetic wave has a reasonably constant power in the area where the patient is allowed to stay.

The Faraday cage and emitter of medical electromagnetic waves can be used to treat depressive disorders of patients younger than 45 years, or auto-immune diseases like multiple sclerosis, by a permanent stay into the Faraday cage in the presence of a medical electromagnetic wave. The medical electromagnetic wave is used to avoid any transient pro-auto-immune situation after entering the Faraday cage, which would cause a worsening of depressive disorders or auto-immune diseases. Preferably, the emitter is used to generate a medical electromagnetic wave which is an "outdoor simulation signal" mimicking the time-averaged spectral power of the artificial electromagnetic waves present in the patient's normal environment. The spectral power of the outdoor simulation signal is preferably kept constant. The outdoor simulation signal can be a gaussian noise of suitable spectral power. When it is necessary to adapt the patient to a novel stable electromagnetic environment in which he will stay after initial treatment, the outdoor simulation signal can be modified progressively from an initial outdoor simulation signal corresponding to the patient's normal environment to a final outdoor simulation signal corresponding to the novel stable electromagnetic environment. If the novel stable electromagnetic environment is a purely thermal environment without artificial waves, this modification can be made by progressively attenuating the outdoor simulation signal over its entire frequency range. In the case of auto-immune diseases or depressive disorders of persons aged less than 45 years, it is also possible to increase the spectral power of the medical electromagnetic wave above the outdoor simulation signal, so as to create an initial anti-auto-immune situation which favours an initial improvement of the patient's health.

The Faraday cage and emitter of medical electromagnetic waves can be used to treat cancer by progressive field cancellation. In this case, the emitter is used to generate a medical electromagnetic wave which is the above-defined "outdoor simulation signal" mimicking the time-averaged spectral power of the electromagnetic waves present in the patient's normal environment. Preferably, the medical electromagnetic wave is already attenuated with respect to the outdoor simulation signal when the patient starts his stay inside the Faraday cage. The power of the medical electromagnetic wave is then reduced during the stay of the patient inside the Faraday cage. Preferably this reduction is progressive and/or is made step by step with multiple steps. At least in certain cases, use of progressive field cancellation may make the treatment more efficient in the long term because it avoids triggering an excessive reaction of immune regulatory mechanisms and because the period of time during which new T cell lines become activated which are aggressive against the cancer is lengthened.

Progressive field cancellation can be combined with a treatment with a cancer-treating electromagnetic wave. In this case, a cancer-treating electromagnetic wave can be emitted from time to time whilst the outdoor simulation signal is permanently present. For example the cancer-treating electromagnetic wave can be emitted 15 minutes per day.

Progressive field cancellation can also apply to AIDS patients to avoid the initial disturbance of a brutally applied absence of fields, which in certain individual cases may cause the elimination by the regulatory system of T cell lines which in exposed conditions and with tritherapies contribute to disease control, or to an excessive aggressiveness of these T cell lines yielding to destruction of the remaining healthy lymphocytes. In the case of AIDS the aim of progressive field cancellation is to reach a thermal environment without any artificial waves, without causing death in a weakened subject due a brutal change and any excessive reaction of the immune system caused by this change.

**Thymus protection combined with an emitter of electromagnetic waves.**

Negative selection of T cells takes place in the thymus. Thymus-selected T cells then migrate to other areas where they can cause damage if too aggressive. The thymus is near the external surface of the human being. An aspect of the invention is an emitter of medical electromagnetic waves combined with a conductive plate adapted to protect the thymus from the waves emitted by the emitter. For treatment of an auto-immune disease, the patient may be submitted to electromagnetic waves whilst his thymus is protected by the conductive plate. The parts of the body which are far from the thymus are exposed, which renders the disease-causing TCR-pMHC generally unable to bind and thus cures the disease in the short term, whilst the generation of abnormally aggressive T cells in the thymus is avoided by not submitting the thymus to the medical electromagnetic wave. This method has its own drawbacks, including imperfect thymus protection, possible development of the auto-immune disease in tissues surrounding the thymus, possible existence of extra-thymic T cell selection processes which are affected by the electromagnetic wave.

**Brief description of the figures:**

Figure 1 represents a Faraday cage.
Figure 2 represents a Faraday cage enclosing an emitter adapted to emit a medical electromagnetic wave of low frequency.
Figure 3 represents a Faraday cage enclosing enclosing an emitter adapted to emit a medical electromagnetic wave and connected to an external control system.
Figure 4 represents a Faraday cage enclosing enclosing an emitter adapted to emit a medical electromagnetic wave and connected to an external control system, wherein the emitter comprises three sub-emitters.
Figure 5 represents a Faraday cage enclosing enclosing an emitter adapted to emit a medical electromagnetic wave and connected to an external control system, wherein the emitter is a Lakhovsky emitter.
Figure 6 represents an exposure setup for exposing the body to an electromagnetic wave but protecting the thymus from the electromagnetic wave.
Figure 7 shows a plurality of jammers protecting a Protected Area
Figure 8 shows a jammer protecting a Protected Area

**Exemplary embodiments:**

**Faraday cage**

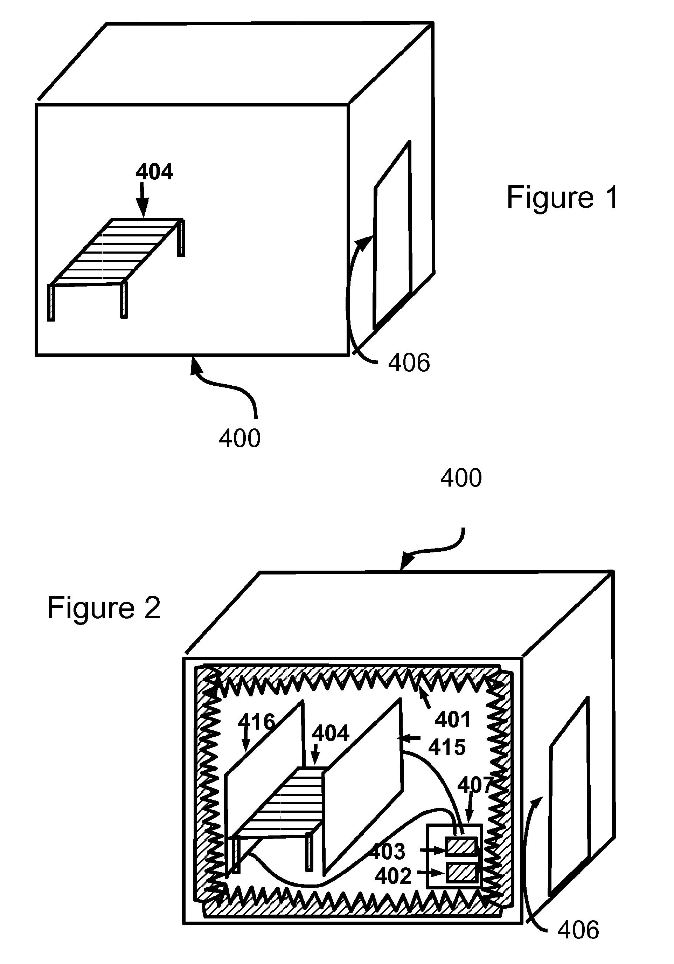

An exemplary embodiment of a main Faraday cage according to the invention is shown on figure 1. It comprises a place where a patient can stay, preferably a bed 404 where he can rest, fully enclosed in a metallic enclosure 400 having a door 406 which when closed is electrically connected to the enclosure and constitutes a barrier to the electromagnetic waves. It has an attenuation of better than 100 dB for electric and magnetic field and is capable of bringing down the field to thermal level in most practical situations.

Faraday cages for use in this invention can however be of any known type, including light Faraday cages made of conductive fabric, and/or fully metallic enclosures made of plain metal or thin metal sheet. Fully metallic enclosures are generally preferred as they provide a better attenuation. Double enclosures as is known in the art are also usable to further improve attenuation of the external electromagnetic fields.

This Faraday cage can be used to protect persons having infectious diseases or cancers from artificial electromagnetic waves.

**Faraday cage comprising an emitter:**

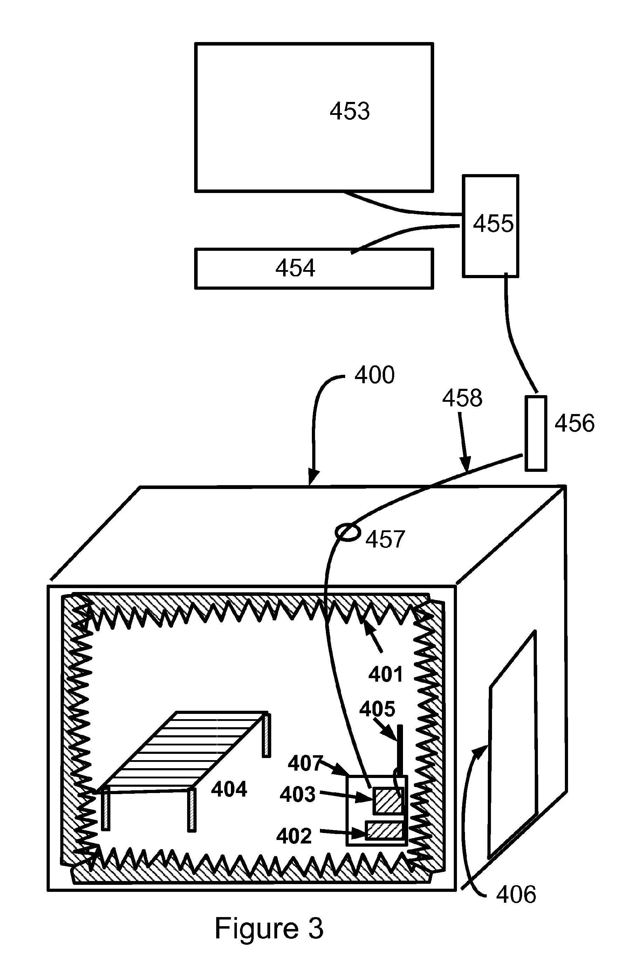

An exemplary embodiment of a main Faraday cage enclosing an emitter, according to the invention, is shown on figure 3. The emitter comprises an antenna 405 connected to a signal generator 403 powered by the battery 402. The grounds of the signal generator and battery are connected to an intermediate Faraday cage 407 which fully encloses the signal generator and battery. The antenna is protruding out. The intermediate Faraday cage further encloses an absorber of electromagnetic radiations. The emitter and its intermediate Faraday cage are enclosed in an absorbing cage made up of absorbing foam 401 which may be shaped with protruding pyramids and absorbs electromagnetic fields at the frequencies generated by the emitter. The absorbing cage 401 also encloses a bed 404 for allowing a patient to rest. The absorbing cage 401 is fully enclosed in the Faraday cage 400. The door 406 is covered on its inner side with absorbing foam elements so as to avoid spurious reflections of electromagnetic fields on the door. The Faraday cage 407 together with the absorbing cage 401 ideally form an anechoic chamber.

The signal generator may be a broadband gaussian noise generator. Broadband gaussian noise generators generating a flat gaussian noise between 10 MHz and 3 GHz are available off the shelf and are programmable to generate any band limited noise or even more complex filtering. Noise generators with a finer programming allowing detailed programming of the attenuation by frequency steps can be made. Such noise generators may be used as the signal generator 403. As for the antenna, it is possible to use a small antenna which is quarter wave at the highest frequency used. For example if a 100 MHz bandwidth from 500 to 600 MHz is used this solution is fully satisfying. If the emitted frequencies are for example between 100 MHz and a few GHz, then due to the wide frequency bandwidth it is preferable to use a log periodic antenna, which is broadband, or the "circular antenna" of patent number FR 732.276 which is also broadband, or any broadband antenna generally.

Optionally, the signal generator 403 is adapted to be controlled via an optical fibre 458. It can then be connected to a computer 455 via an optical fibre adaptor 456. The optical fibre 458 leaves the Faraday cage 400 through a hole 457. The computer 455 is connected to a screen 453 and a keyboard 454 to facilitate controlling or programming the signal generator 403. If the signal generator 403 is not connected to the outside of the Faraday cage, it may simply be programmable, so that a pre-set time sequence (for example a single 15 minutes exposure) can be applied to the patient (with door closed) and the door can be re-opened after termination of the time sequence.

Optionally, the device can also be adapted to automatically turn off the signal generator when the door is open. For example, the connection between the battery and the signal generator can be modified so that the circuit is closed by closing the door, so that when the door is open the signal generator is not powered and thus not emitting.

The exposure near to the antenna is much stronger than the exposure far from the antenna. It is generally desirable to avoid excessive exposure of the patient. In particular, if the treatment is against autoimmune disease, any change of the field power reaching the patient is damageable. Therefore the patient should preferably be prevented from accidentally coming near to the antenna and should even preferably be restrained to such part of the Faraday cage which is far from the antenna. For this reason the Faraday cage can comprise an internal wall (not shown) separating the patient from the emitter of electromagnetic waves. This internal wall must be permeable to the electromagnetic waves emitted by the emitter. Instead of a wall, any physical barrier preventing the patient to come near to the antenna can be used.

A modified embodiment, best adapted to lower frequencies, is shown on figure 2. In this embodiment the antenna is replaced by two plates 415, 416 with a place for a patient to stay being disposed between the plates (here a bed 404). The signal generator is connected to the plates so as to generate an alternating electric field between the plates. For example the ground of the signal generator may be connected to one plate and its signal connector may be connected to the other plate. The optional connection of the signal generator to the outside was not represented in this case.

**Faraday cage comprising three or more sub-emitters**

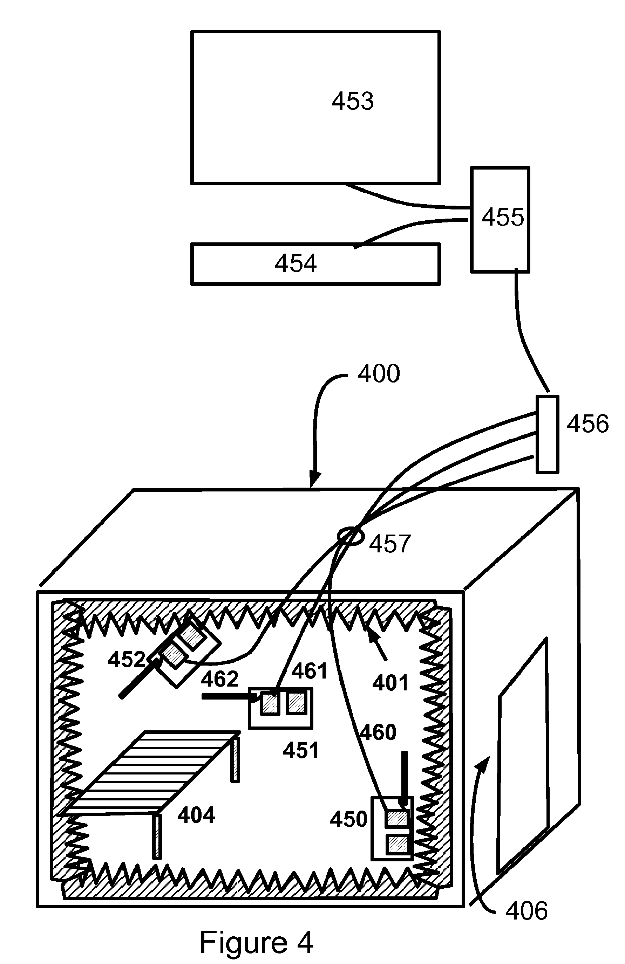

In order to obtain E-field components on three orthogonal directions, the emitter can be replaced by an emitter comprising of a plurality of sub-emitters, yielding the device of figure 4 which comprises three independent sub-emitters 450, 451, 452, each sub-emitter comprising an antenna, a signal generator and a battery.

Each sub-emitter is connected to a computer 455 having a screen 453 and a keyboard 454. The connection is via optical fibers 460, 461, 462 leaving the Faraday cage 400 through a small hole 457. The optical fibers reach an adaptator 456 which is itself connected to the computer 455. The sub-emitters, optical fibers, adaptator, and computer are adapted so that an operator can use the computer to program and/or control the emissions of each sub-emitter.

These three sub-emitters are normally used to generate mutually independent gaussian noises. If the noise is pseudo-gaussian, the pseudo gaussian noise sequences of the sub-emitters preferably differ from each other. The emitted power of each sub-emitter is preferably adapted so that the patient receives about the same spectral power from each sub-emitter. The antennas of the sub-emitters are positioned so that the polarizations of the signals received from each sub-emitter by the patient lying on the bed 404 are orthogonal to each other. In operation, the three sub-emitters are always switched on and off simultaneously, actually behaving like a single sub-emitter of spatially homogeneous random gaussian electric field. This device allows a more homogeneous exposure of the body. The sub-emitters may be individually protected by absorbing foam so as to avoid spurious reflections of the waves on each sub-emitter, or the batteries and signal generators may be grouped together and globally protected by absorbing foam, with the antennas being positioned as appropriate and connected by longer coaxial cables to the signal generators, which is also expected to diminish spurious reflections.

It is possible to use more than three sub-emitters in order to improve the homogeneity of the patient's exposure. Figure 9 shows an exemplary positioning of 6 dipole antennas each of which is emitting an electric field which is a gaussian noise independent of each of the 5 other gaussian noises emitted by the other antennas. The Faraday cage, the signal generators feeding the antennas, and other details, have not been represented. On figure 9 the dipole antennas 700, 701 are parallel to each other and orthogonal to all other dipole antennas; the dipole antennas 710,711 are parallel to each other and orthogonal to all other dipole antennas; the dipole antennas 720,721 are parallel to each other and orthogonal to all other dipole antennas; and the patient is expected to be installed at the centre, where dotted lines cross each other, so as to obtain an exposure as homogeneous as possible.

**Faraday cage enclosing a Lakhovsky emitter**

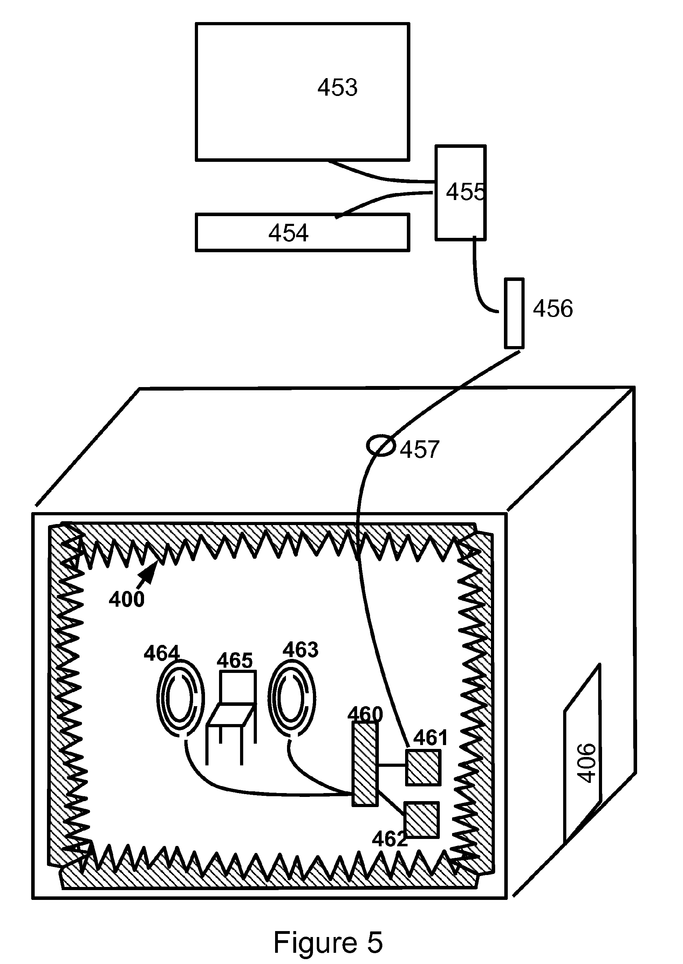

A further modified embodiment (figure 5) uses the emitter and antenna described originally by Georges Lakhovsky in French patent number 732276 ("multiple waves oscillator"). Some working exemplaries of this multiple waves oscillator have been preserved until today and can be used in this embodiment. The Lakhovsky emitter 460 is connected to the antennas 463, 464 and a chair 465 is provided for the patient to sit there. The emitter is powered by a battery-powered autonomous alternative current source 462 and the manual commands are replaced by adaptation circuitry 461 allowing the emitter to be controlled via optical fiber by the computer 455.

The Lakhovsky emitter is usable for treatment of cancer but is not recommended for treatment of autoimmune diseases due to the time-varying nature of the emitted signal.

The Lakhovsky multiple wavelengths oscillator used two antennas which were fed with essentially the same signal comprising both high and low frequencies. Exactly in the middle between the two antennas, the low frequency fields corresponding to wavelengths significantly higher than the distance between the antennas (which distance was 1 to 2 m) were near zero because the quasi-static field between two equally charged antennas is zero. The patient sat there and thus benefited essentially of the high frequency fields. However, low frequency fields existed in other regions due to the potential difference between the antenna and ground, which could be in the order of 100,000 Volts in the low frequencies. The Faraday cage will modify these low frequency fields. In order to minimize disturbances due to the Faraday cage, the Faraday cage should preferably be large enough and the oscillator should preferably be placed far enough from the walls of the cage. For example, the Faraday cage 460 may be cubic, measuring 10x10x10m with the Lakhovsky oscillator's antennas being placed in the center of the Faraday cage about 4-5 m from each of the 6 walls.

**Faraday cage which blocks mostly high frequencies**

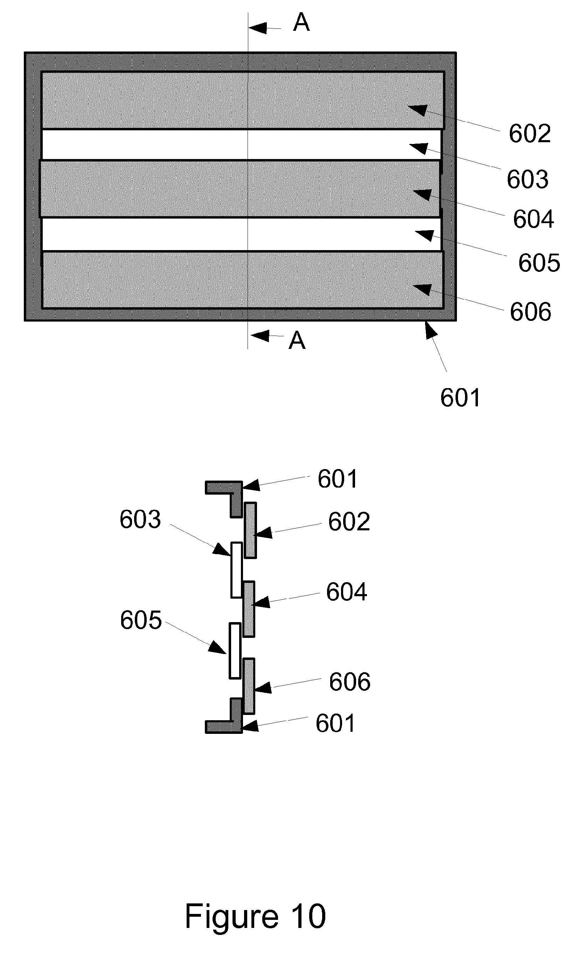

A Faraday cage as shown on figure 1 can have walls adapted to block high frequencies more efficiently than lower frequencies. This Faraday cage is for some specific uses but is not of general use like a Faraday cage which blocks all frequencies. The wall of this Faraday cage is shown on figure 10. This wall is made of plates 602,603,604,605 mounted on a frame 601. The frame and the plates are metallic and covered with a thin wallpaper or any thin non-conductive layer. They may be glued together, for example. The connection between two plates or between a plate and the frame forms a capacitor which blocks low frequencies. Therefore the wall and the Faraday cage as a whole appear as conductive for the high frequencies but not for the low frequencies. As a consequence the Faraday cage blocks the high frequencies and lets the low frequencies pass through. The inside of the Faraday cage is protected from the high frequencies only. As a variation of this figure, instead of large plates it is possible to use square tiles. The surface of the contact area between the plates and the thickness of the non-conductive layer covering the plates determines the cut-off frequency and should preferably be adapted so that the cut-off frequency separates time-varying signals (GSM, wifi, DECT..) from permanent signals (radio, television).

unless otherwise specified this Faraday cage is not the preferred Faraday cage for the treatments described herein as letting the low frequencies pass through is disadvantageous in most cases. This Faraday cage which lets low frequencies pass trough is particularly advantageous when the following conditions are fulfilled:

(a) blocking only the higher frequencies yields an acceptable treatment,
(b) the patient stays only part-time inside the Faraday cage,
(c) it is additionally desirable to minimize the auto-immune effects associated to the alternance between strong artificial electromagnetic waves outside the Faraday cage and weak artificial electromagnetic waves inside the Faraday cage.

Essentially, this low-pass Faraday cage can be used to deal with any health problem specifically related to GSM or higher frequencies. Persons living near GSM base stations or otherwise disturbed by high frequency signals can have their corresponding health problems reduced by sleeping in the low-pass Faraday cage at night, because the high-frequency signals are removed inside the low-pass Faraday cage. By eliminating only the high frequency signals which cause the health problem, it avoids the pro-auto-immune effect which would otherwise result from the alternance between low exposure to permanent signals inside the Faraday cage and high exposure to permanent signals outside the Faraday cage. Such pro-auto-immune effect can cause an auto-immune disease or worsen or trigger an EHS syndrome, so the low-pass Faraday cage is particularly useful to avoid it.

This Faraday cage can also be adapted to lightly attenuate the low frequencies (for example by 3 to 10 dB), so as to maintain a slight anti-cancer, pro-autoimmune effect which is insufficient to cause strong auto-immune effects but is helpful to avoid cancer outbreaks when the lower frequency signals are modified. In this case the higher frequency should preferably remain more strongly attenuated, preferably by 30 dB or more. This adaptation can be made, for example, by using partly conductive layers instead of the wholly non-conductive layers separating the plates. In this case the attenuation in the low frequencies range is adjusted by modifying the resistivity of the partly conductive layer. Instead of using partly conductive layers, it is also possible to electrically connect adjacent plates to one another through resistors. Adjustment of the resistors allows modifying the attenuation of the low frequencies by the Faraday cage.

**Jammers**

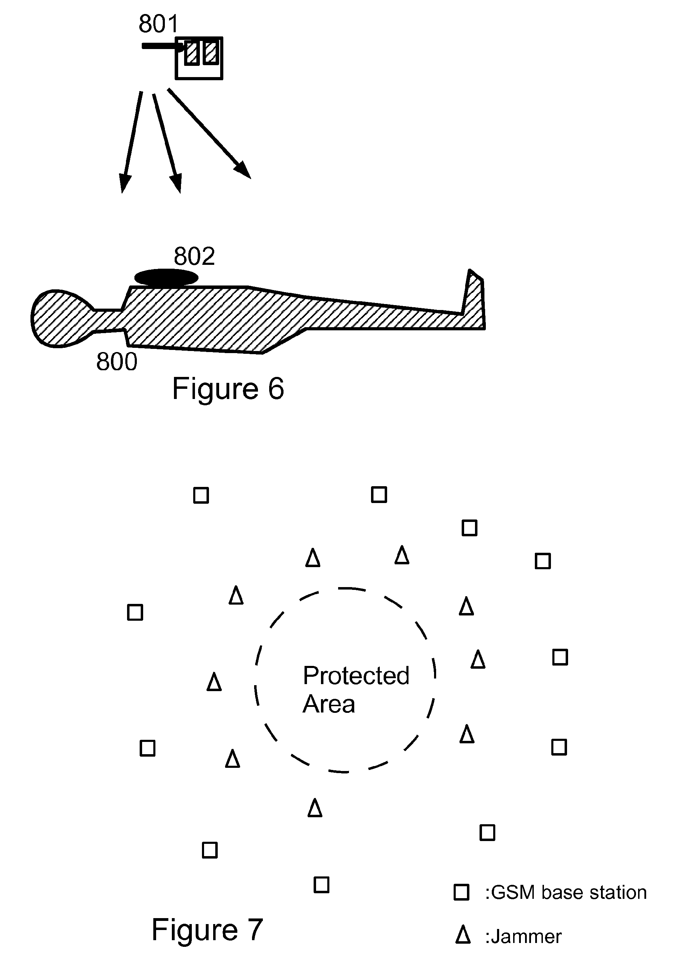

The jammers of figure 7 protect a protected area from the auto-immune effects of neighbouring GSM emitters. In this case the Protected Area is surrounded with jammers. Each jammer emits a white noise signal covering all frequencies used by GSM, both uplink and downlink. The power of each jammer is adjusted so that at the center of the Protected Area the resulting jamming signal resulting from summing up the powers coming from all jammers has a power comparable to the peak GSM power in the same place when all available GSM channels are being used by the GSM transmitters and the corresponding mobile phones outside the protected area. The jamming signals emitted by different jammers are non-correlated. If using pseudo-gaussian noise generators, the pseudo-gaussian sequences of each jammer should preferably be different or sufficiently shifted in time relative to one another. The emission power of each jammer is kept constant.

The same effect can be realized with a single jammer when the GSM power comes from essentially a single direction as shown on figure 8.

The jammers can be adapted to jam any other time-varying signal, by emitting a white noise signal covering all frequencies occupied by the other time-varying signal, with a power comparable to the peak power of the time-variable signal.

**outdoor simulation signal**

For the treatment of certain diseases, particularly autoimmune diseases, an "outdoor simulation signal" is necessary. This signal reproduces the signal levels encountered by the patient in his normal environment, but suppresses all signal variations.

For example, it is possible to make a complete measurement of the maximum spectral power of all radiofrequency signals which the patient encounters in his normal environment. This includes measuring the spectral power of the signals received in the place where the patient lives, at various times including daytime and nighttime, and in the place where the patient works, and then calculating the maximum of these measured spectral powers on each frequency. Then the "outdoor simulation signal" will be a gaussian noise having the calculated maximum spectral power.

By exception to the above, certain signals present in the patient's environment need not be taken into account in the "outdoor simulation signal" if there is a good reason for that. In particular, high power signals from a single GSM phone need not be taken into account because they may not disturb the immune system very strongly and because taking them into account would yield unreasonably high power values. But time-averaged signals from nearby GSM base stations or resulting from a time-averaging of many GSM phones operating from reasonably far should preferably be taken into account because such signals do disturb the imune system.

**treatment of cancer by medical electromagnetic waves**

Treatment of cancer by a medical electromagnetic wave can be performed outside a Faraday cage on a patient living in a daily environment which comprises relatively high power electromagnetic waves. However this is far from ideal. Ideally, a treatment for cancer should be performed using an emitter enclosed in a Faraday cage, for example as shown on figure 3 or figure 4, so as to avoid triggering pro-auto-immune diseases on the operating physician and on the neighbourhood generally, and so as to improve predictability and reproducibility of the results since the medical electromagnetic wave may interfere with other waves. Ideally, the patient should stay full time in a Faraday cage from the beginning of the treatment to the complete elimination of the cancer, or alternatively stay in an area which generally has very low powers of artificial electromagnetic waves, so as to stop any ongoing pro-cancer effect due to a low to high exposure transition, create an anti-cancer effect due to the high to low exposure transition, and ensure maximum predictability and reproducibility of treatment outcome. For example, treatments can be performed using the emitter 402, 403, 405 enclosed in the Faraday cage 400 with the patient lying on bed 404 as shown on figure 3. The patient may stay full time in this Faraday cage or may stay in this Faraday cage only for the purpose of being exposed to the medical electromagnetic wave, in which case he may stay in the Faraday cage of figure 1 when not being exposed to the medical electromagnetic wave.

Treatment of cancer using a medical electromagnetic wave is expected to often be efficient, but at least in difficult cases other known treatment methods should be used simultaneously. Old patients or cancers on an advanced stage may be more difficult or even impossible to cure.

In all cases, the patient is preferably relocated after treatment to a place which has very little power of artificial waves. If the patient stays in a Faraday cage full-time during treatment as recommended, he should preferably stay in the Faraday cage until complete disappearance of the neoplasm. After complete disappearance of the neoplasm, ceasing to use the Faraday cage has a pro-cancer effect which can yield to redevelopment of any remains of the neoplasm. This risk can be minimized by continuing to use the Faraday cage at least part-time during an interim period and/or by relocating the patient to a place which has very little artificial electromagnetic waves.

*treatment of cancer with a fixed bandwidth emitter*

For the treatment of cancer, the emitter can for example be programmed to generate a pseudo gaussian noise having constant spectral power in a frequency bandwidth from 600 MHz to 640 MHz with an overall power of 1 W/m2. Patients may for example be be exposed once each 4 days during 15 minutes, until some treatment results become detectable.

The overall power may be decreased. For example some anti-cancer effect is expected to still exist with an overall power of 10 $\mu$W/m2 and a 40 MHz bandwidth because this is still stronger than the power which caused deaths at DVB onset. However the corresponding level of anti-cancer effect may be quite low.

The overall power may also be increased insofar as it does not cause an excessive heating effect. Some moderate heating is permissible however since hyperthermia is a known anti-cancer therapy.

This treatment can be modified by using higher bandwidths whilst keeping the overall power constant. For example the bandwidth can extend from 50 MHz to 3 GHz, with a constant spectral power in this bandwidth and an overall power of 1 W/m2. This treatment can also be simplified by using a lower bandwidth. For example, a cw wave having a power of 1 W/m2 and a frequency between of about 600 MHz can be expected to perform well which is to a experimentally verified as certain extent by the fact that before inventing the Multiple-Waves Oscillator Lakhovsky used cw systems with some (though limited) success. However whilst a cw wave may perform well at high power values, a wave of the same overall power but having a larger bandwidth is generally expected to perform at least as good, and in most cases better.

The time duration between two exposures may be modified. For example weekly exposures may suffice. The duration of each exposure may be modified. Each exposure may for example last 10 minutes or 1 hour. If low power and/or low bandwidth is used, increasing exposure duration may to a certain extent compensate for insufficient power or bandwidth.

Treatment parameters will need to be optimized over time. In the interim period when treatment parameters have not been optimized, it is the physician's responsibility in view of the patient's particular situation to choose the most appropriate treatment and possibly to try more than one set of parameters. Generally, the benefits of 15 minutes exposures at 1W/m2 are believed to outweigh the corresponding risks in cancer patients; in particular, 1W/m2 is well below officially authorized power limits. However, in this interim period, the more cautious approach described in section "cautious procedure before treatment is optimized" may also be used if there is sufficient time available - or any other cautious approach based on attempting treatment first with low power and bandwidth and progressively increasing power and bandwidth.

*detailed explanation of cancer treatment efficiency*

During exposure of the patient to the medical electromagnetic wave, the thymus if still active (or otherwise, other organs capable of producing naive mature T cells) has a higher negative selection threshold, i.e. it will let T cells pass through which are more capable of binding antigens of the self or "near self". These cells when reaching lymph nodes are much more likely to be activated by antigens of cancerous cells than cells that have undergone negative selection in the thymus in the absence of the signal. The action of the immune system against cancerous cells is, in the short term, only delayed by the duration of the exposure. For example if a daily exposure of 15 minutes is used, the inaction of existing of T cells during 15 minutes is unlikely to cause cancer per se due to the short 15 minutes time. But the production by the thymus of new T cells which have a superior ability to bind and destroy cells of the "near self" is giving the patient a better chance of recovery.

Since exposure duration is much shorter than the thymus transit time, each T cell is expected to undergo only part of its negative selection process under the exposed conditions. Insofar as the negative selection process is not too highly repetitive, any single selection step which a T cell undergoes (with respect to a corresponding thymus-presented pMHC) in exposed conditions gives this T cell a chance of surviving the overall negative selection process despite the fact that in non-exposed conditions it binds this specific pMHC stronger than is normally allowed by thymus selection. Therefore each negative selection step which a T cell undergoes in exposed conditions participates to finally producing T cells that globally have a higher than normal capacity to eliminate cancerous cells.

*treatment of cancer with the Lakhovsky emitter*

An alternative treatment uses past therapeutic experience. The Lakhovsky multiple wavelengths oscillator used a wavelength range of 750 kHz to much more than 3 GHz, and used about 400 VA of power from the mains. The signal emitted by the "multiple wavelengths oscillator" was not a flat gaussian noise. It is not known how much the total emitted power was, but there was no thermal effect so that the power received by the patient was most likely below 10 W/m2. As some units of the Lakhovsky oscillator have been preserved, they may be used directly within the present invention. In this case the same therapeutic procedure used by Lakhovsky can be used, using for example 4 exposures of 15 minutes duration separated from each other by a few days. To ensure best reproducibility of Lakhovsky's results the patient should preferably remain full time inside a Faraday cage or in another little exposed area when not exposed, because the interactions between the treatment and the electromagnetic waves encountered in daily life could yield unpredictable results. Even so, the exact method of Lakhovsky is improved rather than replicated, because the anti-cancer effects of Lakhovsky's device are superimposed to the anti-cancer effects of the high to low exposure transition corresponding to the patient starting to use the Faraday cage.

During exposure, the Lakhovsky multiple wavelengths oscillator and the patient can preferably be enclosed in a Faraday cage so as to avoid causing auto-immune diseases to the operating physician and the neighbourhood.

*treatment of cancer by a medical electromagnetic wave of variable bandwidth*

The power of the medical electromagnetic wave used is limited in order to not cause any excessive direct heating of the patient. More generally, excessive powers may trigger unexpected side effects. For a constant power of the medical electromagnetic wave, the spectral power is lower if the bandwidth is higher, potentially reducing the effects on cancers when the $\Delta b$ of implied TCR-pMHCs is low. Thus in order to maximize the effect on all cancers, periods of exposure using different bandwidth can be used.

For example, the overall power of the medical electromagnetic wave is kept constant and each 20 minutes daily exposure period is subdivided in a 5 minutes exposure with a centre frequency of 100 MHz and a bandwidth of 40 MHz, a 5 minutes exposure with a centre frequency of 500 MHz and a bandwidth 200 MHz, a 5 minutes exposure period with a centre frequency of 1.1 GHz and a bandwidth of 2 GHz, and a 5 minutes exposure period with a centre frequency of 2.1 GHz and a bandwidth of 4 GHz.

*treatment of cancer by an electromagnetic wave which does not have a flat spectral power.*

Similar advantages can be achieved by using 20 minutes exposures to a medical electromagnetic wave which has a non-flat spectral power, comprising a higher spectral power in a small bandwidth and a lower spectral power in an extended bandwidth. For example a medical electromagnetic wave may be the sum of:

- a flat gaussian noise having a centre frequency of 100 MHz and a bandwidth of 40 MHz and an overall power of 0.3 W/m2;
- a flat gaussian noise having a centre frequency of 500 MHz and a bandwidth of 200 MHz and an overall power of 0.3 W/m2; and
- a flat gaussian noise having a centre frequency of 1100 MHz and a bandwidth of 2 GHz and an overall power of 0.3 W/m2;
- a flat gaussian noise having a centre frequency of 2100 MHz and a bandwidth of 4 GHz and an overall power of 0.3 W/m2;

The overall power of this medical electromagnetic wave can be further increased insofar as it does not cause excessive heating. This medical electromagnetic wave is believed to be particularly efficient due to it addressing simultaneously most of the TCR-pMHCs. However, for about the same reason it is also more likely to have undesirable side-effects including the triggering of auto-immune reactions. In the absence of treatment optimization results, this medical electromagnetic wave, possibly with increased power, is believed to be the best possible treatment for urgent and desperate cancer cases, where the possibility of undesirable side-effects is not a major concern.

*treatment of cancer by an electromagnetic wave scanning the frequency domain*

In case significant undesirable side effects would be found experimentally when using large bandwidth but not when using high frequencies at lower bandwidth, it is possible to reduce the bandwidth and scan the frequency domain with a signal which has a reduced bandwidth. For example the emitter can generate a medical electromagnetic wave which has a 40 MHz bandwidth signal and which has initially a centre frequency of 100 MHz which can be increased progressively to 3 GHz or more.

*treatment of cancer using short pulses*

The strength of the anti-cancer effect depends on exposure duration and on the power of the medical electromagnetic wave. Depending on the cancer being treated, it may be necessary to increase the power of the medical electromagnetic wave beyond the limit which can cause excessive heating. In such case a wave comprising a series of short pulses can be used: the increased power during the pulse is compensated by the lack of power outside the pulse so that the average power (which causes heating) remains low but the peak power (which may be limiting for the anti-cancer effect) is increased.

Whilst the use of a pulsed wave is useful to increase the peak power of the medical electromagnetic wave, it also causes the medical electromagnetic wave to be inefficient against T cells which form with cancerous cells a TCR-pMHC having a time constant $\tau_{rec}$ superior to the pulse length, and generally less efficient in "intermediate cases". Therefore, the pulse length must always remain reasonably long, superior to a "minimum pulse length". The minimum pulse length, is unknow. This minimum pulse length should preferably be obtained experimentally on the animal before being applied to man. Once the minimum pulse length is known, a pulsed wave may more securely be applied.

Typically the patient may be subject to an electromagnetic wave at 100 W/m2 during one pulse length and then un-exposed during 100 pulse lengths, this cycle being repeated to obtain a sufficient overall exposure time of about 15 minutes.

Short pulses may also trigger unpredictable effects. Short pulses should not be used unnecessarily and are not a preferred solution generally, except possibly for very difficult cases.

*discussion of treatment parameters*

Increasing exposure duration or number of exposures increases the number of negative selection steps in the thymus for which the selection criteria is modified. Therefore it increases the chances that a T cell line will emerge which can control a cancer, if such cancer is controllable by a T cell line having the modified selection criteria.

Increasing exposure power increases the aggressiveness of thymus-produced cells towards the self and thus towards cancer, and thus increases the proportion of cancers which are controllable and the typical size of controllable neoplasms. Therefore, more advanced neoplasms are likely to require higher power for treatment than neoplasms which can be treated early. Increasing power also increases the Rabi frequency and thus the Rabi integration bandwidth, which can compensate in whole or in part an insufficient bandwidth.

Increasing exposure bandwidth at constant spectral power increases the Δb range which is affected and the range of bandwidth conditions of affected TCR-pMHCs, and therefore is expected to increase the variety of cancers which can be cured.

Older patients are less responsive to treatment because they have lesser thymus output. It is expected that longer exposure periods and/or more numerous exposures should at least partly compensate for this drawback.

Patients having a well-advanced cancer with large neoplasm size or metastases are less easy to treat because even a T cell line relatively efficient against the cancer may not destruct cancerous cells fast enough to compensate for the natural growth of the neoplasm. It is expected that higher exposure power will be beneficial in this case, as compared to cancers which are less advanced.

*discussion of undesirable side effects*

Excessively increasing the proportion of time during which the patient undergoes exposures and the duration of the treatment may result in a pro-cancer effect because during exposure, no T cell line is efficient against cancer.

Increasing the exposure power (at constant bandwidth) increases the variety of auto-immune diseases that may be caused as a side effect of the treatment, because as more aggressive T cells are produced, auto-immune diseases requiring more aggressive T cell lines can develop.

Increasing the number or duration of exposures increases the likelihood of an auto-immune disease to be caused if such auto-immune disease can be caused by T cells produced under exposed conditions.

Increasing exposure bandwidth at constant spectral power is expected to increase the variety of auto-immune diseases which can be caused.

Inflammatory responses may also be disturbed. During exposure, certain inflammatory responses are expected to be stopped. If an inflammatory response is stopped for too long, it may be slow to restart, resulting in potentially damageable results if the inflammatory response was useful to maintain the patient alive.

Other undesireable side effects may appear. Inhibitory receptors of T cells may be rendered inefficient, which could cause a pro-cancer effect.

*reasons for treatment optimization*

Generally, the cancer treatments described in the above sections sections are expected to have reasonable efficiency on at least younger patients at an early stage of cancer development. Prognosis worsens with age and with delay between onset of cancer and start of treatment. When prognosis is bad, higher powers, bandwidths and exposure times may be necessary, including using pulsed exposure. But when prognosis is good, lower powers, bandwidths and exposure times may be preferable so as to avoid triggering an auto-immune disease uselessly. For example, heart attacks may in certain cases have an auto-immune cause and it is undesirable to cure a patient from cancer but cause him to die later by heart attack. This risk is not purely theoretical. For example, Lakhovsky believed that Guglielmo Marconi had died out of overexposure to short waves, particularly in relation to a shortwave emitter which Marconi had built for therapeutic purposes and experimented on himself. The death of Marconi by heart attacks may actually be attributable to an auto-immune problem caused by overexposure. Also, in certain cases, high powers and bandwidths may have unexpected side effects due to action on an inhibitory receptor.

Therefore, it is generally desirable to optimize the treatment. Treatment optimization essentially means not using exposure powers, exposure times and exposure bandwidths which are unnecessary in view of the patient's age and of the stage of his cancer. Treatment optimization may be conducted on animal models, refined by clinical testing, and further refined using the physician's experience generally, which will build up over time. The other considerations discussed above in sections "discussion of treatment parameters" and "discussion of undesirable side effects" are also useful in best adapting the treatment to any particular patient.

One possibility for treatment optimization is to observe any negative side effects of the treatment and determine maximum exposure power, duration and bandwidth on this basis. However this is likely to be insufficient in many cases because auto-immune diseases due to T cells selected during exposure periods may become detectable only years after treatment.

Another possibility for treatment optimization is based on finding the minimal exposure power and duration which yield good treatment outcomes, and the corresponding bandwidth.

In practice a mix of the above two methods may be used.

*exemplary optimization procedure on animals*

Different cancers may require different bandwidths or different powers. Different HLA systems may also require different bandwidths and/or center frequencies and/or powers. The treatment can be optimized by tests on the animal. For example it is possible to carry out initial optimization on the mouse, then refine it on apes. Preferably, in each animal species tested, a curve of thymus size as a function of age is first determined, and ages for that species are then normalized relative to the age of maximum thymus size (i.e. age of maximum thymus size = normalized age 1). Preferably, the entire procedure is followed in a Faraday cage to avoid any un-controlled artificial electromagnetic waves. An exemplary optimization procedure for the treatment, based on finding the minimum exposure power and duration which yields reasonable treatment outcomes, uses the following basic procedure comprising steps B1 to B4:

Step B1: Cancer is initiated in each animal, by chemical means or radiations for example.
Step B2: The specific cancer developed by each animal is identified and recorded, as much as feasible.
Step B3: Each animal is submitted to irradiation by an artificial electromagnetic waves in different conditions.
Step B4: the tumor reduction (+) or inefficiency of the treatment (-) is asserted and recorded for each animal.

The artificial wave is a white gaussian noise of constant spectral power between a lower frequency F1 and an upper frequency F2 and is zero for frequencies lower than F1 or higher than F2. A sufficient number of animals, preferably having all the same MHC type and similar age corresponding to maximum thymus size, is obtained. A full procedure is followed which comprises the following steps S1 to S4:

S1: 20 male animals are taken initially and the basic procedure is followed for these 20 animals using a 20 minutes daily exposure to the artificial waves with F1=100 MHz, a power of 0.1 W/m2, and a bandwidth BF=F2-F1 which varies from 50 MHz to 2000 MHz by steps of 100 MHz with one animal for each frequency step. If any animals is successfully treated, an average bandwidth Bav over all animals which were successfully treated is determined.
S2: 20 new animals are taken and the basic procedure is followed for these 20 animals using F1=100 MHz, F2=F1+Bav (if previously no animal was successfully treated, Bav=100 MHz is used), 20 minutes daily exposure, and power levels relative to 0.1 W/m2 of -3n dB for the animal number n. In W/m2 a power $P_n = 0.1 \cdot 10^{-\frac{3n}{10}}$ is applied to animal number n. The lowest index n for which treatment is successful for animals n and n+1 is recorded. The corresponding power level Pmin is recorded.
S3: 20 new animals are taken and the basic procedure is followed for these 20 animals using a 20 minutes daily exposure to the artificial waves with F1=100 MHz, a power of Pmin, and a bandwidth F2-F1 which varies from 100 MHz to 2000 MHz by steps of 100 MHz. If any animals is successfully treated, a new average frequency Bav over all animals which were successfully treated is determined.
S4: 20 new animals are taken and the basic procedure is followed for these 20 animals using F1=100 MHz, F2=Bav, 20 minutes daily exposure, and power levels relative to Pmin of -0.3n dB for the animal number n. In W/m2 a power $P_n = P_{min} \cdot 10^{-\frac{0.3n}{10}}$ is applied to animal number n. The lowest index n for which treatment is successful for animals n and n+1 is recorded. The corresponding new power level Pmin is recorded.

At this level, reasonably optimized treatment parameters for the MHC type and species tested should have been obtained.
Then the procedure is restarted but with animals having another MHC type and/or animals of a different species (different sorts of apes, ...), but of the same normalized age, using reduced initial range of frequencies and powers as can be reasonably deduced from the first test. The procedure is also restarted with females as these may yield to significantly different results.
The procedure can also be refined to obtain a more precise value of the bandwidth, and the minimum frequency F1 can also be optimized independently of bandwidth.
Optimized bandwidths and powers can thus be obtained which correspond to different MHC systems and animal species. All individual results (cancer type, whether tumor was reduced or not) obtained for each animal during these tests are recorded together with detailed exposure conditions, age, any side effect observed, any useful data generally. Complementary tests are carried out if needed. The lowest power and frequency range which allows good treatment results for most animals, MHC systems and cancer types is retained if this power and frequency range did not cause any observable side effects and is reasonable with regards to commonly accepted exposure standards; otherwise a compromise is made between treatment results and any side effects. Optimized bandwidth and power are thus obtained corresponding to a certain normalized age. More elaborate testing can be carried out in view of determining the minimum power which yields treatment results not statistically different from the results obtained for such power which yields the best treatment outcome, for example on the basis of a 90 % Student's T test with N=20.

Then the following procedure is used to optimize the daily duration of exposure as a function of normalized age: For example daily exposure durations of 5 minutes, 10 minutes, 20 minutes, 40 minutes, 80 minutes, 3 hours, 6 hours may be tested. For each daily exposure duration the Basic Procedure is carried out on a group of 20 animals using previously obtained power, bandwidth, and any other optimized parameter if available. The number of animals successfully treated is asserted. The minimal exposure duration which yields treatment results not statistically different from the results obtained for the exposure duration yielding maximal treatment success is asserted and retained. "not statistically different" means for example not statistically different on the basis of single sided 90 % Student's T test. This minimal exposure duration is retained as the optimized exposure duration for the normalized age being investigated. The same procedure is repeated for different species and/or MHC systems and the maximum value of the retained exposure duration over these different species and/or MHC systems is finally retained. An optimized exposure duration as a function of normalized age is thus obtained.

Details of the above procedures may be varied as is known in the art, insofar as the general target is maintained, which is to obtain treatment parameters, including characteristics of the artificial electromagnetic wave, which ensure good results on a large variety of species, MHC systems and cancer types without uselessly submitting the animals being treated to excessive power levels or frequency bandwidths which may result in autoimmune or other unintended side effects in the human. It is possible to use a wide range of animal species in the above procedures yet ideally it would be preferable to use different species of apes, if possible comparable to the human with regard to their lifetime expressed in normalized age. Further optimizations can be carried out to obtain fully optimized treatment parameters wherein exposure frequency, bandwidth and power may also depend on age.

The optimized treatment parameters obtained, which depend on normalized age, can be applied to the human taking into account the normalized age of the human.

*treatment optimization on humans*

Clinical experience will be essential in improving cancer treatment because optimization is generally necessary and because optimization on animals, although preferable to a non-optimized treatment, is far from perfect when applied to humans.

Therefore, a database should preferably be set up to record all details of treatments performed using the medical emitter of the present invention, the outcome of such treatments and a follow-up of patients. The follow-up is useful to detect possible post-treatment deaths which may occur years after treatment yet be related to an auto-immune disease contracted as a side effect of the treatment. This database should be properly analyzed from time to time to elaborate improved recommendations for treatment.

*cautious procedure before treatment is optimized*

If treatment must be applied before optimization results are available, the exact procedure to be used is a reasonable compromise between the risk of undesirable side-effects and the efficiency of the treatment. In difficult cases the procedure described in section "treatment of cancer by an electromagnetic wave which does not have a flat spectral power" may be used with a strong power of the medical electromagnetic wave. However, for treating slowly growing neoplasms which are not too advanced, the power and bandwidth used may be increased progressively from initially low values until some treatment results become observable. An example of such procedure is as follows:

(a) expose the patient 15 minutes to a white gaussian noise, centre frequency 600 MHz, bandwidth 40 MHz, overall power 0.1 mW/m2. Repeat this 4 times at 1 day interval. Wait 2 weeks to see if any positive results of the treatment appear.

(b) If no positive results of the treatment appear, repeat procedure (a) with a power increased by 10 dB each time, until a power of 10 W/m2 is reached or positive results are obtained.

(c) Repeat the procedure described in (a) and (b) with a bandwidth of 300 MHz, starting with an overall power of 1 mW/m2.

(d) Repeat the procedure described in (a) and (b) with a bandwidth of 3 GHz, starting with an overall power of 0.01 W/m2, centre frequency 1600 MHz.

The treatment is interrupted when positive treatment results appear. The appearance of positive treatment results is a sign that T cells able to control the cancer have been generated so that it is not necessary to continue the treatment.

In case the cancer worsens again after interruption of the treatment, the procedure may be restarted where it was before the interruption, as if no positive results had been detected, because either the cancer has somehow worsened or the improvement which led to interruption of the treatment was not actually due to the treatment. In case other treatments are conducted in parallel, it may be difficult to distinguish results from the medical electromagnetic wave treatment from results of other treatments, so that restarting the treatment after interruption may happen more often.

If no results are obtained, higher powers may be used, up to powers that cause a slight heating, no more heating however than is permissible for example in hyperthermia therapy.

**treatment of cancer without high exposure periods**

Cancer can be treated merely by a sufficiently long stay in a Faraday cage, for example the Faraday cage of figure 1. In this case, the transition period is anti-cancer according to Table 1 and is thus favorable to cancer recovery. If this treatment is not sufficient, the treatments using a medical electromagnetic wave may be used. If results are obtained with this treatment, the patient should stay in the Faraday cage until complete elimination of the neoplasm. Ceasing to use the Faraday cage after treatment will result in a pro-cancer effect possibly yielding to renewed growth of any remains of the neoplasm. This risk can be minimized by continuing to use the Faraday cage at least part-time during an adaptation period after end of the treatment and by relocating the patient to a place which has very little artificial electromagnetic waves.

**treatment of cancer using progressive field cancellation**

The Faraday cage of figure 3 can be used to treat cancer using progressive field cancellation. In this case, the emitter is used to generate the "outdoor simulation signal" mimicking the spectral power of the permanent electromagnetic waves present in the patient's normal environment. The power of the medical electromagnetic wave is reduced during the stay of the patient inside the Faraday cage, for example it may be reduced by 10 dB a week. The use of progressive field cancellation may make the treatment more efficient in the long term because it avoids triggering an excessive reaction of immune regulatory mechanisms and because the period of time during which new T cell lines become activated which are aggressive against the cancer is lengthened.

The above-described treatments by medical electromagnetic waves described in section can be combined with the treatment by progressive field cancellation. In this case the medical electromagnetic wave may comprise:

(a) a first medical electromagnetic wave which is emitted from time to time to cause production of more aggressive T cells, and
(b) a permanent medical electromagnetic wave which initially simulates spectral power of the electromagnetic waves present in patient's normal environment. The power of the permanent electromagnetic wave is progressively reduced as described in this section.

Progressive field cancellation is not recommended in difficult cancer cases, particularly when the cancer is at an advanced stage, because in such situations the strongest possible initial treatment is desirable to stop cancer progression.

**treatment of cancer by combined use of hyperthermia**

The treatment of cancer with or without high exposure periods is enhanced by the simultaneous use of hyperthermia. In this case, hyperthermia should preferably be performed inside the Faraday cage. A suitable hyperthermia "space suit" as described in Cancer Research 1984; 44; 4869s-4872s, using a hot circulating gas or liquid to heat the suit, should preferably be used to perform hyperthermia proper, although other hyperthermia techniques are also usable.

**treatment of autoimmune diseases**

*basic treatment*

Autoimmune diseases can be treated by submitting the patient to a permanent controlled exposure, for example in the Faraday cage of figure 4, preferably with the 6 antennas represented on figure 9. In today's electromagnetic environment people undergo daily changes in the power of the multiple electromagnetic fields to which they are submitted, which is a pro-autoimmune situation according to Table 1. In order to best overcome the autoimmune disease the patient should ideally be submitted to a constant electromagnetic environment. However if the patient is brutally installed in a very low exposure environment in a Faraday cage the autoimmune disease can worsen at least during a transition period, as described in Table 1 for the high exposure to low exposure transition. Submitting the patient to a constant controlled exposure suppresses daily exposure variations without creating a transient pro-autoimmune situation (which transient situation can last months or years, especially after thymus involution). A good clinical practice would be to measure fields in the places where the patient lives normally, and submit him permanently to the strongest of these fields on each frequency value. In this case the emitter or plurality of emitters can initially be programmed to generate the "outdoor simulation signal". This should guarantee that the binding conditions for T cells are generally equivalent to the "least binding" conditions which they have been submitted to in the thymus.

The outdoor simulation signal can be amplified within reasonable limits, insofar as it does not cause any heating effect. Such amplification may be useful to more fully interrupt an ongoing auto-immune disease initially triggered by artificial waves. The outdoor simulation signal may also be modified by replacing the maximum signal power which the patient could encounter in his normal life by the maximum spectral power of the artificial electromagnetic waves which the patient may have encountered in exceptional situations. The latter signal is useful to deal with auto-immune diseases which may have been triggered by exceptional exposures to artificial waves. Its power may also be further increased insofar as it does not cause any heating.

The "outdoor simulation signal" can optionally be superimposed to a second signal which is also pseudo gaussian but has a smoothly varying spectral power grossly proportional to the spectral power of the outdoor simulation signal averaged for example on a bandwidth of 5 MHz in the 50 - MHz range to 1 GHz-range, in order to smoothen the conditions generally.

It can optionally be further supplemented by a signal having a spectral power proportional to the thermal spectral power, for example over the 1 MHz to 3 GHz frequency range with a spectral power of at least 100x the thermal spectral power, possibly higher, up to an overall power of 1 W/m2. This latter signal is efficient in dealing with auto-immune diseases which are unrelated with artificial electromagnetic waves.

An auto-immune disease may have an underlying cause, possibly as simple as lack of exercise or excessive feeding, which if not eliminated may favour the return of the disease. It is thus desirable to suppress any underlying cause during and after the treatment.

The treatment may in certain cases worsen the situation rather than improve it. High frequencies like wifi do not penetrate as deeply into the human body as lower frequencies. For example if an auto-immune disease affects the innermost parts of the body, the presence of a permanent high frequency signal, for example at Wifi frequencies, may be damageable because the thymus will be overexposed relative to the areas where effector T cells take action, causing an auto-immune effect. If such problems do occur, it will be necessary to cut down the frequency bandwidth of the signal.

After recovery the patient should preferably be relocated to a relocation area having very low exposure to time-varying artificial waves and be confined within that area so as to not expose himself uselessly. Ideally the exposure conditions in the main Faraday cage should be progressively changed to simulate exposure conditions in the relocation area before the patient is allowed to leave. Ideally the relocation area should be protected by the jammers described in the corresponding section of this application if there are any GSM or other time-variable networks operating nearby.

*discussion of prognosis and risks*

The above treatment of autoimmune diseases is based on having a constant exposure. The exposure should preferably be homogeneous within the human body, otherwise if the thymus is more exposed than other parts of the body it may worsen the disease rather than cure it. The exposure should preferably be directionally homogeneous, with comparable E-field components in each direction, otherwise some disease-causing TCR-pMHC will be able to bind depending on their orientation, despite the presence of a sufficient field to inhibit such binding. Auto-immune diseases have varying mechanisms which are not as well-known as for cancer and it is often uncertain whether a disease is auto-immune or not. Therefore the treatment of auto-immune diseases is generally more difficult than the treatment of cancer and is likely to have a much lower success rate. Treatment of an auto-immune disease can also yield to a difficult situation in which the patient is unable to live a normal life without the permanent exposure to which he was submitted during treatment.

Unlike the treatment of cancer which can be limited to a few quarter-hour exposures, treatment of autoimmune diseases is also a long term treatment which may need continuous Faraday cage protection and exposure to the medical electromagnetic wave during an extended period of time. The treatment of auto-immune diseases is therefore costly and difficult for the patient.

Although stronger medical electromagnetic waves may often improve the short-term efficiency of the treatment on strictly auto-immune diseases, they also expose the patient to an increased risk of cancer and infectious diseases. When the disease has an infectious aspect as well as an auto-immune aspect, which may be the case of multiple sclerosis, they may actually worsen the disease. They also expose the patient to a higher long-term risk of the same or another auto-immune disease being triggered after the end of the treatment by T cells having been thymus-selected during the treatment. This results in an increased duration of the post-treatment adaptation period during which exposure conditions are progressively brought back to normal, and complicates the patient's return to normal life generally. For these reasons the increased short-term treatment efficiency expected from an increase of exposure power should generally be weighted against its numerous drawbacks. In many cases, it may be preferable to avoid any unnecessary increase of electromagnetic waves. The simplest treatment, which is a direct relocation of the patient to a Protected Area protected by jammers will not overcome all inflammatory diseases but it may often be the best compromise between long term and short term and between the need for a treatment and the patient's well-being.

*treatment optimization on animals*

Treatment of autoimmune diseases can be optimized on animals in a manner analogous to treatment of cancer. Instead of initiating cancer in the animals, an autoimmune disease can be artificially caused, for example by submitting the animal to a sequence of low exposure and high exposure which can trigger an autoimmune disease according to table 1 column E. Success of treatment is characterized by recovery from the autoimmune disease and survival. Apart from these points the procedure can be about the same like the procedure of optimizing cancer treatment on animals.

*treatment optimization by use of statistics*

It is also possible to use statistical results in order to optimize treatments. For example heart diseases (particularly acute myocardial infarction) may have an auto-immune aspect (see HAL- 00877298 version 2, section 9.3). The death rate by heart diseases was diminished in Paris in 2005 for men in the 35-44 age category. The affected range of $\Delta b$ values is expected to be essentially below 60 MHz. A typical centre frequency was 600 MHz. Power was less than 0.01 mW/m2 in most places in Paris. Thus it can be inferred that a treatment of heart diseases using a medical electromagnetic wave which is a gaussian noise having a bandwidth of 60 MHz and a power of 0.01 mW/m2 and a centre frequency of about 600 MHz will most likely have some degree of efficiency on men aged 35-44 living in low exposure areas. For men of the same age living in Paris, it may be necessary to use a higher power so as to overcome the effects of the already existing electromagnetic waves, and to superimpose an "outdoor simulation signal" to the 60 MHz flat gaussian signal, so as to avoid creating a pro-auto-immune effect at entering the Faraday cage.

The same treatment can apply to chronic diseases of the liver. In both cases it is unclear whether the disease is in essence auto-immune, inflammatory, or even anything else; but independent of the detailed interpretation of the causes, the possibility of the treatment is confirmed by the decrease of these diseases following the onset of DVB in Paris. Heart diseases and chronic diseases of the liver are wide categories. Detailed examination of the data shows that the specific diseases and age categories that reacted most to the onset of DVB and are thus expected to respond best to the treatment are cardiomyopathy, acute myocardial infarction and alcoholic disease of the liver in men aged 35-45 years, and all sorts of heart attacks and non-alcoholic fibrosis and cirrhosis of the liver in women aged 55-64.

It is expected that a higher power and/or bandwidth of the medical electromagnetic wave may increase efficacy of the treatment of heart diseases and chronic diseases of the liver, particularly on older age categories. Use of a Faraday cage is expected to increase the treatment's success rate but the medical electromagnetic wave can also successfully be applied without a Faraday cage, as was the case at the onset of DVB.

*treatment with a Faraday cage only*

GSM and Wifi increased the death rate by multiple sclerosis. Since the increase of death rate was most likely an acceleration of death in ongoing diseases, it results that in multiple sclerosis the implied T cell lines tend to change over time, i.e. new T cell lines appear that cause multiple sclerosis to continue, and most likely old T cell lines which also caused the disease tend to disappear. Thus a treatment option is to suppress all time-varying waves by using a permanent stay in a Faraday cage. This may initially worsen the disease due to the temporary pro-auto-immune transient period after entering the Faraday cage, but if the patient survives this initial period the disease may then progressively disappear due to the lack of a permanent pro-auto-immune effect. This is a less efficient solution than the use of an outdoor simulation signal, which avoids the dangers of the transient pro-auto-immune period after entering the Faraday cage.

*treatment with a Faraday cage which blocks high frequencies.*

The reason why using a Faraday cage part-time is not a recommended procedure in case of auto-immune problems (including multiple sclerosis) is that it causes a transient pro-autoimmune situation. A solution to this problem is to use the Faraday cage having walls as shown of figure 10. As this Faraday cage suppresses only time-varying fields, the transition period after entering the Faraday cage is less strongly pro-auto-immune than with a normal Faraday and provides a more stable environment than the outside environment, so that it contributes to the prevention or treatment of auto-immune problems.

The simplified treatment without an emitter of electromagnetic waves improves the situation relative to a full absence of control. However, for an efficient treatment of a readily installed auto-immune disease, the preferred treatments are those described herein that imply an increase of the electromagnetic fields inside the Faraday cage as compared to the outside. These treatments therefore cannot be performed without the presence of an emitter of medical electro-

magnetic waves.

*treatment by progressive increase of the medical electromagnetic wave*

When there is an underlying cause other than electromagnetic waves, which cannot be suppressed during treatment, then a treatment option is to progressively increase the power of the medical electromagnetic wave so as to obtain a permanent anti-auto-immune effect which lasts as long as the electromagnetic wave can be increased. For example the power of the electromagnetic wave can be increased by 3 dB a month until a power is reached which causes heating. This desperate solution may allow some extra months or years of survival depending on how fast power is increased. It can be attempted for example on Creutzfeldt-Jacob disease, which is in part auto-immune as discussed, but with a very bad prognosis.

The main limitation of this method is that power cannot be increased above certain limits. In order to diminish the overall power without affecting bandwidth, the center frequency of the medical electromagnetic wave can also be diminished. Diminishing the center frequency results in lower probability Wdest of destruction of the antigen-presenting cell at constant power, so that the medical electromagnetic wave can be efficient at a lower power value - provided the frequency conditions remain verified. If the treatment is started at a lower frequency and power, then it may last longer. However it may not always be possible to do so: for example Creutzfeldt Jacob disease responds well at 1 GHz centre frequency corresponding to GSM, but it is unknown whether it would respond equally well at centre frequencies below 100 MHz.

*treatment using thymus protection or local application*

The treatment can be improved in certain cases by using thymus protection as shown on figure 6. In this case the patient 800 is exposed to medical electromagnetic waves generated by an emitter 801. His thymus is protected by a metallic plate 802 appropriately placed to stop such part of the electromagnetic waves which would otherwise reach the thymus. The treatment can take place inside a Faraday cage (not represented) having appropriate absorbing walls as on figure 3. Details of the electromagnetic wave are unchanged as compared to the previous sections. The patient may be lying on a bed. The treatment is difficult because the exposure may sometimes last long, possibly until death, and the thymus protection should preferably remain in place during the whole of the treatment. Outcome is uncertain because the thymus protection does not affect any extra-thymic negative selection and because aspects of the auto-immune disease may develop in tissues surrounding the thymus without being affected by exposure. Use of thymus protection may improve prognosis, for example in Creutzfeldt-Jacob disease.

A comparable improvement may be possible by local application of a medical electromagnetic wave. For example in Creutzfeldt Jacob disease it is possible to use emitters fixed on the head and adapted to expose the head to a higher power than the thymus.

*treatment using jammers*

An auto-immune disease can be treated by using the jammers described herein to protect the place where the patient lives from the pro-auto-immune effect of time-variable waves, or by relocating the patient to a place which is protected from such pro-auto-immune effect by jammers.

In the case of a relocation, if the place where the patient is to be relocated is having little permanent electromagnetic waves, an adaptation period may be necessary in a Faraday cage as shown for example on figure 4 in which the electromagnetic conditions are modified progressively towards the predominating conditions in the place of relocation.

**basic treatment of infectious diseases**

A disease caused by a pathogen can be treated by placing the patient in a Faraday cage, typically at the onset of fever, and leaving him for a time which depends on the pathogen but which is typically about two weeks in the Faraday cage either full time or at night time (full time being ideally preferable). In case of a benign disease, no other treatment is necessary. In particular, children younger than 7 years old have a developing immune system and treatment by a Faraday cage can in many cases avoid the use of antibiotics and probably avoid the development of allergies associated with frequent antibiotic use and difficulties of the immune system generally. In case the Faraday cage treatment is insufficient or the disease potentially dangerous, additional treatments can be used in complement to the Faraday cage, including antibiotics.

**adjustment of a treatment of infectious disease.**

Use of the Faraday cage in the above conditions may occasionally cause autoimmune problems or worsen inflammatory reactions. In this case the emitter (if present in the Faraday cage) can be used to generate a medical electromagnetic wave in the Faraday cage, which electromagnetic wave may be an attenuated version of the outdoor simulation signal, in order to overcome the autoimmune problem or inflammatory problem. The treatment may also be interrupted by allowing the patient out of the Faraday cage. Dying bacterias sometimes release toxins which cause sufferings and may occasionally be dangerous. The treatment can be momentarily slowed down in case of excessive sufferings, by starting the emitter of electromagnetic waves, which in this case can be adjusted to simulate the spectral power of the waves outside the Faraday cage with a small coefficient of attenuation. Alternatively, if there is no emitter of medical electromagnetic waves in the Faraday cage, the treatment may be momentarily interrupted by letting the patient out of the Faraday cage.

An infectious disease treated by stays in a Faraday cage tends to re-appear when the Faraday cage ceases to be used because the T cells controlling the pathogen in the Faraday cage have a destruction probability Wdest of infected cells which will most likely pass below the control limit as soon as the Faraday cage ceases to be used. This problem can be solved:

- by lengthening the treatment (i.e. lengthening the time period during which the patient stays full-time or part-time in the Faraday cage) so as to fully eliminate the pathogen, and/or
- by repeating the treatment (i.e. re-starting a stay or a series of repeated stays in the Faraday cage) each time the disease reappears.

After a sufficient time during which the disease is globally controlled by repeated treatments, a new T cell line is likely to emerge which will be capable of controlling the pathogen outside the Faraday cage. The appearance of such T cell line is slowed down outside the Faraday cage because there is only a reduced number of naive mature T cells which can potentially control the disease outside the Faraday cage, and because due to both the reduced number of these naive T cells and their Wdest which is generally nearer to the control limit, the primary immune response is considerably slowed down outside the Faraday cage. On the other hands, most T cell lines selected by primary immune response in the Faraday cage are likely to be inefficient outside the Faraday cage. However these facts do not preclude the appearance of an appropriate line of T cells after a sufficient time of treatment repetitions with the patient staying outside the Faraday cage otherwise.

Although it is desireable that the patient stays full time in the Faraday cage when he is ill, he may also stay only at night in the Faraday cage when ill. If the disease is an infectious disease which is not self-mimicking, this is likely to only moderately affect the immune response because an immune system which is fully efficient for about half time can normally overcome a threat which otherwise (i.e. with an inefficient immune system) takes a few days to develop. This is likely to be the case with most bacterial diseases including bacterial pneumonias. But certain bacterial diseases like Lyme disease and certain viral diseases like influenza have self-mimicking aspects and in these cases the alternate stays in and out of the Faraday cage are likely to be more notably less efficient than a permanent stay, and even possibly damageable because they are pro-auto-immune, so that some care is needed when using the Faraday cage as treatment for an unknown infectious disease.

**treatment of self-mimicking pathogens and the like**

These pathogens include certain spirochaete and certain strains of influenza virus like the 1918 and probably the 2009-2010 influenza viruses. The treatment is similar to other pathogens but it is more important to avoid alternate stays in the Faraday cage and instead stay in permanently. Part-time, typically night-only stays in the Faraday cage can cause auto-immune problems which may favour the disease, and should preferably be avoided. At start-up of a stay in the Faraday cage the transient pro-auto-immune effect of the high to low transition may favour the pathogen, so that there is an uncertainty period during which the immune defense against the pathogen is improved by the direct anti-pathogen effect but weakened by the pro-auto-immune effect, resulting in an uncertain global effect. Later, the immune system comes to equilibrium with an improved anti-pathogen defense as per Table 1 column A and no more pro-auto-immune effect. If the patient survives the uncertainty period without a major degradation of his health, there is no specific problem. The advantages of the treatment by a stay in the Faraday cage are expected to globally overcome the risk associated to the uncertainty period, yet when a self-mimicking aspect is suspected the treatment should be used more carefully, including a monitoring of the patient at the beginning of the treatment, to interrupt the stay in the Faraday cage in case of a degradation of the patient's health.

**treatment of pathogens which are favoured by GSM base stations**

Certain infectious diseases, in particular infectious diseases causing sleep disturbances, tend to appear in the vicinity of GSM base stations, and possibly near Wifi emitters. These infectious diseases can be treated by use of a Faraday cage, like other infectious diseases. However treatment of these diseases using a Faraday cage can trigger auto-immune diseases or EHS syndrome. In order to minimize this risk, the low-pass Faraday cage described herein can be used. This Faraday cage attenuates the high frequency fields - which in the vicinity of GSM base stations are often the strongest fields - but leaves the permanent fields unmodified. If a patient living in a place which has strong exposure to a GSM base station but relatively low exposure to permanent artificial waves sleeps each night in that Faraday cage, his sleep disturbances or other symptoms of an infectious disease favoured by GSM will disappear - as in any Faraday cage - because they are mostly related to GSM in this case and the low-pass Faraday cage blocks the GSM signal. But use of a normal Faraday cage would additionally generate a pro-auto-immune effect due to the alternance between low exposure to permanent artificial waves and high exposure to permanent artificial waves. This pro-auto-immune effect can also trigger or worsen an EHS syndrome. This pro-auto-immune effect is avoided in the Faraday cage because the low-pass Faraday cage does not block permanent artificial waves.

**treatment of certain spirochaete**

Concerning cyst-forming, self-mimicking spirochaete that cause fever, a treatment may comprise the following steps:

(a) have a long preliminary period (at least a month, preferably several months) of staying uninterruptedly in a Faraday cage or another environment with very low artificial electromagnetic waves. This long period diminishes the pro-auto-immune effects that result from artificial electromagnetic waves and which favour self-mimicking spirochaetes.
(b) wait until fever appears.
(c) start using at least a cyst-inhibiting antibiotic, for example tetracycline. If a Faraday cage was not used earlier, start using a Faraday cage simultaneously, else continue to use the Faraday cage uninterruptedly.
(d) continue this treatment until well after disappearance of fever (preferably at least two weeks)

Preferably the patient should not leave the Faraday cage until the treatment is terminated.
As the long preliminary period is not always possible, it may be dispensed for. But this causes auto-immune effects due to the artificial time-varying waves present in the normal environment and due to the transition period after entering the Faraday cage, which is pro-autoimmune according to Table 1 column D. These pro-autoimmune effects favour the self-mimicking spirochaetes and diminish the efficacy of the treatment. The simultaneous use of a cyst-inhibiting product and the Faraday cage allows the immune system to properly eliminate the spirochaetes and avoids the formation of cysts that would allow the spirochaete to escape the immune system. Tetracycline may be replaced by any product (not necessarily an antibiotic) capable of inhibiting cyst formation.
As there is no certainty that all spirochaetes leave their cysts simultaneously, it may be necessary to reiterate the treatment a number of times. Preferably, the patient should stay in the Faraday cage or other environment with low artificial electromagnetic waves during the entire treatment including any repeats of the basic treatment. In this case phase (a) is only needed before the first administration of the cyst-inhibiting product.
If fever does not appear in phase (b) it may at least in certain cases be provoked by temporarily (for example during 12 hours) submitting the patient to a medical electromagnetic wave which is for example a flat gaussian noise having 80 MHz bandwidth, 1 GHz center frequency, 1 W/m2. Details, in particular the duration of exposure, may be validated and optimized by clinical trials. It is unclear whether fever will start on exposure or immediately after exposure. The medical electromagnetic wave is here to momentarily weaken the immune system so as encourage the undoing of cysts. What is attempted is to cause the undoing of cysts then as brutally as possible cause the immune reaction to restart and simultaneously use antibiotics.

**treatment of AIDS**

Treatment of AIDS is similar to treatment of other pathogens, with modifications due to the low control threshold and the dangerousness of this specific pathogen. A full-time stay in the Faraday cage can be started as soon as possible. A complementary anti-retroviral treatment, like tritherapy, can be used. Preferably the patient is left full time in the Faraday cage until well after full recovery. Tritherapy may be continued during the whole treatment or stopped during the treatment, in which case the virus load should preferably be monitored and tritherapy re-started if the virus load increases excessively after cessation of tritherapy. A patient who recovers from AIDS in a Faraday cage may develop the disease again when leaving it unless the virus has been entirely eliminated, because the T cells controlling the virus in the Faraday cage have a destruction probability $W_{dest}$ of infected cells which will most likely pass below the control limit as son as the Faraday cage ceases to be used. Therefore it is not recommended that AIDS patients entirely stop using a Faraday cage at the end of a treatment. Preferably they should continue to use it at least part time, for example at night. After a sufficient time of this intermittent use, a patient may have produced T cells capable of controlling the various mutated forms of the virus, outside the Faraday cage. But the number of such patients is not expected to be significantly higher than the number of patients who can control the virus after cessation of a successful tritherapy, and for other patients, continued intermittent or permanent use of the Faraday cage is necessary.

This method of treating AIDS can also be used for other dangerous pathogens, replacing tritherapy by a known therapy efficient against the pathogen in question.

The best chances for strong improvement to result from a treatment of AIDS by use of a Faraday cage are for the youngest patients, including children. Youngest patients have a developing immune system and starting a permanent stay in the Faraday cage early enough can allow this immune system to develop almost as if it had never been exposed to electromagnetic waves. Persons who developed their entire immune system under exposed conditions have irreversible weaknesses of their immune system and are less likely to fully recover. Persons who start the treatment at an advanced stage of the disease have a very weakened immune system with a strongly diminished T cell library, and are still less likely to fully recover. Best treatment of AIDS implies spending many years permanently in the Faraday cage, which is a difficult constraint.

Progressive field cancellation as described herein can also be applied to AIDS, particularly when the immune system is already dangerously weakened. In this case progressive field cancellation (which should preferably be done in combination with other treatments including thritherapies) may avoid brutal disturbances at the beginning of the treatment which could cause death of a weakened patient. It is also unknown whether or not AIDS has any self-mimicking aspects which could potentially result in it being favoured by the transient pro-auto-immune period after entering the Faraday cage. In case these self-mimicking aspects exist, progressive field cancellation may diminish the risk associated with the transition period after entering the Faraday cage. The field cancellation should preferably be as fast as permitted by the patient's health, and is a compromise between not causing death by having too fast a transition and not letting enough time for AIDS to cause death directly.

**treatment of inflammations**

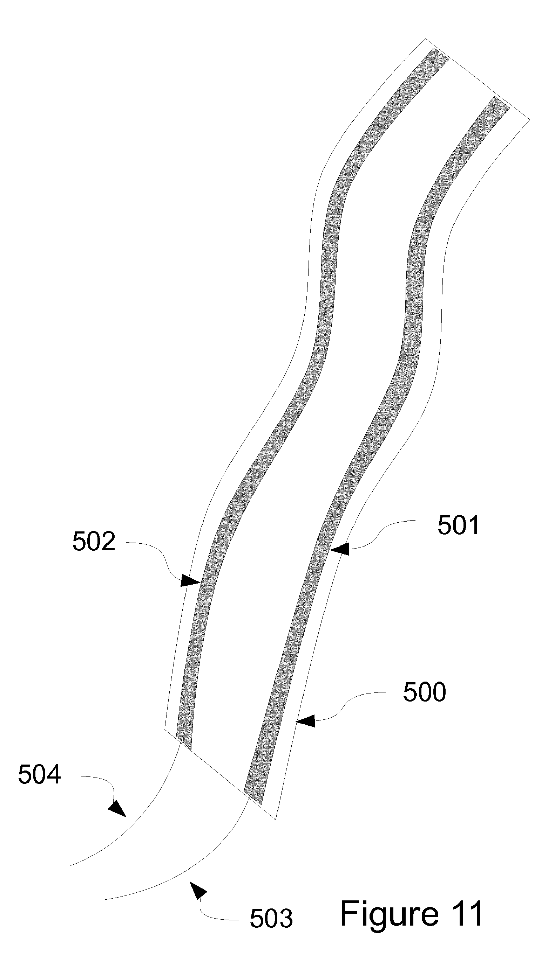

Localized Inflammations can be treated by submitting the corresponding area of the body to a localized electromagnetic wave. Figure 11 shows a device for the treatment of the vertebral column. It comprises a soft band 500 on which two conductive layers 501, 502 are deposited or glued. These conductive layers are connected via cables 503, 504 to a signal generator. The distance between the two conductive layers is for example 5 cm. For treatment, the band is applied on the affected zone of the vertebral column and the signal generator is activated to generate a large bandwidth pseudo-gaussian noise, which may for example occupy the full frequency band between 100 and 200 MHz. The power of the signal should preferably be limited to respect maximal exposure limits of about 1 W/m2 in the vertebral column but the treatment may be effective at much lower power levels and the minimal power levels should preferably be determined for each patient by successive attempts from the microwatt per square meter towards the watt per square meter. The device of figure 11 is constructed to maximize irradiation of the diseased area and minimize irradiation of the remaining parts of the body, so as to minimize the adverse health consequences of the diminished immune response.

In order to minimize effects on remaining parts of the body, it is possible to use lower frequencies, like 10 to 20 MHz for example, and to put the conductive layers 501,502 in direct electrical contact with the body via a conductive gel. In this case, electrical currents created inside the body do attenuate the field faster.

In order to increase the range of inflammations that will respond to the treatment it is also possible to increase the bandwidth of the medical electromagnetic wave, for example a pseudo gaussian signal between 100 MHz and 3000 MHz can be used.

Similar devices for local application can be designed specifically for other parts of the body.

Both localized and non-localized inflammations can also be treated by submitting the entire body to an electromagnetic wave. For example a white gaussian noise in the frequency range of 50 to 800 MHz may be used. This white gaussian noise comprises the FM and TV frequencies that participated in reducing the death rate by pneumoconiosis, emphysema and other inflammatory diseases in France in the 1981-1987 period and is therefore expected to be efficient against pneumoconiosis, and other inflammatory diseases. The effect on pneumoconiosis and emphysema is a long term effect and therefore, in this case, the patient should preferably be permanently submitted to the medical electromagnetic wave, ideally from onset of treatment until death.

These treatments of inflammations and inflammatory diseases can take place inside a Faraday cage using an emitter enclosed in the Faraday cage, so as to not submit the neighborhood to potentially dangerous waves. In the case of long-term treatments, the capabilities of the patient's immune system against pathogens are weakened, so it may be necessary to interrupt the treatment if the patient becomes ill with an infectious disease. Also, in the case of a long-term treatment, increasing the power of the medical electromagnetic wave progressively rather than brutally may minimize cancer risk at onset of the treatment.

Treatment of inflammations can be optimized on animals in a manner analogous to treatment of cancer. Instead of initiating cancer in the animals, mere inflammations are artificially caused. Success of treatment is characterized by less inflammation rather than survival. Animals may be re-used as they do not normally die in the experiment.

Inflammation can be useful to defend the body against pathogens and other external aggressions, so that treatment of inflammatory problems has to be done carefully and should preferably not be attempted without some analysis of the underlying cause of infection. I necessary a treatment of the underlying cause by known methods can take place simultaneously with the treatment of the inflammation by a medical electromagnetic wave. In some cases a treatment of the inflammatory problem may avoid short term sufferings but worsen the long-term prognosis by favouring a pathogen.

**organ transplantation**

The emitter of medical electromagnetic waves can be used in organ transplantation to prevent or treat transplant rejection. The emitter's antenna is preferably attached to the body of a patient near the transplanted organ so as to maximize exposure of the transplanted organ. The emitter's signal generator and other components may be carried by the patient, for example in a backpack, or they may be placed on a moveable table, for example, so as to allow the patient to move. Preferably, the patient (with the emitter) is staying in a Faraday cage, to avoid causing health problems to others due to the waves emitted by the emitter. The emitted signal will need to be optimized experimentally on animals and in clinical assays. Absent the optimization results, the emitter may for example be emitting a white noise from 100 MHz to 2 GHz at 10 W/m2, but whilst this is likely to somewhat diminish rejection mechanisms, an optimized signal may do much better. When it is difficult to apply the electromagnetic wave locally, the patient can also be exposed with thymus protection as shown on Figure 6. The patient may also be exposed entirely without any protection, but in this case there is an increased cancer risk (which may sometimes be negligible as compared to the risk of immediate death by transplant rejection). Electromagnetic wave power can be easily and instantaneously adjusted, which may be useful to overcome episodes of acute rejection, even in patient who can live without a permanently attached emitter of electromagnetic waves.

**treatment of Parkinson's disease**

Parkinson's disease was shown herein to have at least an infectious and possibly an auto-immune aspect. Parkinson's disease can be treated by a stay in a Faraday cage, to improve the immune system's efficacy against the pathogen. Using a Faraday cage only part-time implies a risk of auto-immune problems as per Table 1 column E since the artificial electromagnetic waves varies between the inside and the outside of the cage; therefore the patient should preferably stay full time in the Faraday cage. The initial period of the stay in the Faraday cage is pro-autoimmune according to Table 1 column D and there may be a worsening of the symptoms during this period. In order to avoid such transient worsening of the symptoms, the "outdoor simulation signal" can be used. Ideally, this "outdoor simulation signal" should be progressively decreased in order to improve the immune system's capabilities against pathogens without worsening the auto-immune problem.

At least in the short term the anti-pathogen efficacy of the treatment (which requires as little electromagnetic waves as possible) is not easily compatible with an anti-autoimmune aspect which implies maintaining a permanent medical electromagnetic wave. Therefore the compromise between these conflicting needs can be adjusted by clinical experience and this adjustment results in an optimized spectral power of the medical electromagnetic wave and an optimized change of this spectral power over time. Clinical experience may yield to the conclusion that the pro-auto-immune conditions do not particularly favour Parkinson's disease, in which case a treatment by a Faraday cage without particular precautions would be appropriate.

**reduction of body weight**

Body weight excess is in part related to GSM. However the time-varying aspect GSM.

The Faraday cage may be used so as to diminish body weight whilst avoiding the worsening or the first appearance of typical EHS symptoms like tingling and numbness, which are auto-immune symptoms which can be caused or worsened by the alternance between low electromagnetic fields inside the Faraday cage and high electromagnetic fields outside the Faraday cage. Alternatively a Faraday cage which fully blocks all frequencies can be used but with the risk of causing EHS symptoms. Alternatively a Faraday cage as shown on figure 3 can be used, which blocks all frequencies but has an enclosed emitter. In this case the enclosed emitter can be programmed to simulate the part of the signal present outside the Faraday cage which is permanent. The emitter will generate a gaussian noise which spectral power will be equal to the spectral power of the permanent artificial electromagnetic waves present in the outside environment of the Faraday cage, on the whole bandwidth occupied by these electromagnetic waves.

Ideally the patient willing to diminish his body weight should stay full time in the Faraday cage. As this is not always possible, nightly use of a Faraday cage may to a certain extent facilitate a reduction of body weight by improving nightly digestion.

**treatment of mental disease**

For treatment of depression or other mental problems certain patients (normally older patients) need a pro-auto-immune environment in order to diminish the thickness of their myelin sheaths. These patients can be submitted to a time varying electromagnetic wave inside or outside the Faraday cage. For example it is possible to use a white noise having a centre frequency of 600 MHz, a bandwidth of 6 MHz, a power of 0.01 W/m2, and to expose the patients for 4 hours once a week. Other patients (normally younger patients) need an anti-auto-immune environment in order to thicken their myelin sheaths. These patients can in principle stay permanently in a Faraday cage without other electromagnetic waves. However in order to facilitate the initial period immediately after entering the Faraday cage, the patient can be submitted to a permanent field mimicking the constant fields that are present in the outside environment. This avoid the transient pro-autoimmune effect immediately after entering the Faraday cage. Generally, patients aged more than 50 years are more likely to need of an auto-immune effect whilst patients aged less than 50 years are more likely to need an anti-auto-immune effect, but this is not systematic. This is an "average" result deduced from variations of suicide rates in France at the onset of GSM and at the onset of digital TV, but the needs of individual patients may differ and it may sometimes be necessary to try a treatment (pro or anti auto-immune) during a few months and try the opposite treatment if the first did worsen a situation rather than improve it. Patients needing an anti-auto-immune effect may also be treated by a stay in a Protected Area protected by the jammers as shown on figures 7 and 8, where the pro-auto-immune effect of communication networks is cancelled.

Whilst it is possible to treat depression, it is also possible to improve mental well-being in older persons who are not affected by depressive disorder, so as to make them happier than would naturally be the case. This can be done by submitting older persons to a pro-auto-immune effect (which would treat depression if present). Thus the mental well-being of older persons (preferably over 65 years old) can be improved by exposing these persons to the same time-varying wave which is used to cure depressive disorders.

## Industrial applications

The devices and methods described in this application can be used to cure diseases.

**Claims**

1. A Faraday cage for use in a treatment of a disease and/or a treatment for reducing body weight, the Faraday cage being adapted to receive a patient to be treated inside the Faraday cage, and the treatment comprising at least one stay of the patient to be treated in the Faraday cage.

2. A Faraday cage as claimed in claim 1, for use in a treatment for reducing body weight.

3. A Faraday cage as claimed in claim 1, for use in a treatment of disease.

4. A Faraday cage as claimed in claim 3, for use in a treatment of a disease which causes and/or requires a reaction of the immune system.

5. A Faraday cage as claimed in any of claims 3 to 4, for use in the treatment of a disease, the treatment further comprising oral or intravenous administration of a substance having a therapeutic effect against the disease, so as to complement the effects of the Faraday cage.

6. A Faraday cage as claimed in any of claims 3 to 5, the treatment further comprising exposure to a medical electro-magnetic wave having a favourable therapeutic effect on the disease, so as to complement the effects of the Faraday cage.

7. A Faraday cage as claimed in any of claims 3 to 6, for use in a treatment of cancer, autoimmune diseases or diseases caused by pathogens.

**8.** A Faraday cage as claimed in claim 7, for use in the treatment of cancer.

**9.** A Faraday cage according to claim 8, the treatment further comprising periods of exposure to electromagnetic waves.

**10.** A Faraday cage according to any of claims 8 to 9, the treatment comprising a phase of hyperthermia.

**11.** A Faraday cage according to claim 10, the hyperthermia being performed inside the Faraday cage.

**12.** A Faraday cage according to any of claims 3 to 7, for use in the treatment of fever and/or of an disease causing fever.

**13.** A Faraday cage as claimed in claim 12, the treatment further comprising measuring the body temperature from time to time and starting a stay in the Faraday cage when the body temperature increases.

**14.** A Faraday cage as claimed in claim 7, for use in the treatment of infectious diseases.

**15.** A Faraday cage as claimed in claim 14 for use in the treatment of infectious respiratory diseases.

**16.** A Faraday cage as claimed in claim 15 for use in the treatment of pneumonia.

**17.** A Faraday cage as claimed in claim 14 for use in the treatment of Acquired Immunodeficiency Syndrome (AIDS).

**18.** A Faraday cage as claimed in claim 14, for use in the treatment of diseases caused by cyst-forming spirochaetes, the treatment also comprising administering an antibiotic to the patient.

**19.** A Faraday cage as claimed in claim 18, the antibiotic being adapted to inhibit the formation of cysts.

**20.** A Faraday cage as claimed in claim 19, the antibiotic being tetracycline.

**21.** A Faraday cage as claimed in claim 19, the antibiotic being administered so as to be active when the patient is in the Faraday cage.

**22.** A Faraday cage as claimed in any of claims 1 to 21, the treatment comprising repeated overnight stays in the Faraday cage.

**23.** A Faraday cage as claimed in any of claims 1 to 21, the treatment comprising staying permanently in the Faraday cage during at least a week.

**24.** a Faraday cage according to any of claims 1 to 23, said Faraday cage enclosing a bed for allowing a patient to sleep inside the Faraday cage.

**25.** a Faraday cage according to any of claims 1 to 24, having an attenuation of at least 30 dB for the electric field in the 80 MHz to 1000 MHz frequency range.

**26.** a Faraday cage according to any of claims 1 to 24, having an attenuation of at least 40 dB for the electric field in the 80 MHz to 1000 MHz frequency range.

**27.** a Faraday cage according to any of claims 1 to 24, having an attenuation of at least 100 dB for the electric field in the 10 MHz to 2500 MHz frequency range.

**28.** a Faraday cage according to any of claims 1 to 27 having an attenuation of the electric field power superior to 120 dB in the 80 MHz to 110 MHz band and superior to 80 dB in the 110 MHz to 3 GHz band.

**29.** a Faraday cage according to any of claims 1 to 28 wherein the Faraday cage is adapted to attenuate the higher frequencies more strongly than the lower frequencies.

**30.** a Faraday cage according to claim 29, wherein a cut-off frequency between the more attenuated high frequencies and the less attenuated low frequencies is adapted to separate high frequency time-varying signals from lower frequency permanent signals.

**31.** a Faraday cage according to claim 30, for use in a treatment for reducing body weight.

**32.** a Faraday cage according to claim 30, for use in a treatment of sleep disturbances.

**33.** a Faraday cage as claimed in any of claims 3 to 30, for treating a disease, wherein the disease to be treated is not a sleep disturbance due to a cyst-forming spirochaete.

**34.** a Faraday cage as claimed in any of claims 3 to 30, for treating a disease, wherein the disease to be treated is not a sleep disturbance.

**35.** a building comprising a Faraday cage according to any of claims 1 to 24 the Faraday cage and the place where it is installed being adapted so that the spectral power of the electric field due to artificial electromagnetic waves inside the Faraday cage is less than 100 times the thermal spectral power, on at least the 10 MHz to 3 GHz frequency range.

**36.** a building comprising a Faraday cage according to any of claims 1 to 24 the Faraday cage and the place where it is installed being adapted so that the spectral power of the electric field due to artificial waves inside the Faraday cage is less than the thermal spectral power, on at least the 10 MHz to 3 GHz frequency range.

**37.** a building enclosed in a Faraday cage or comprising a plurality of Faraday cages enclosing a plurality of rooms, for use in a hospital for hosting patients.

**38.** a surgery room adapted to perform surgical acts and enclosed in a Faraday cage.

**39.** a surgery room as claimed in claim 38, comprising an absorber of electromagnetic waves, said absorber being enclosed in the Faraday cage and adapted to absorb electromagnetic waves emitted by medical apparatuses.

**40.** a low pass Faraday cage adapted to attenuate frequencies higher than a high limit more strongly than frequencies which are lower than a low limit.

**41.** a low pass Faraday cage as claimed in claim 40, the high limit being below the frequencies of GSM communication networks, Wi-fi and DECT.

**42.** a low pass Faraday cage as claimed in any of claims 40 to 41, the low limit being higher than the frequencies of Hertzian Television.

**43.** a low pass Faraday cage as claimed in any of claims 40 to 42, the low limit being 790 MHz.

**44.** a low pass Faraday cage as claimed in any of claims 40 to 43, the high limit being 900 MHz.

**45.** a low pass Faraday cage as claimed in any of claims 40 to 44, attenuating the electric field above the high limit by at least 20 dB more than the electric field below the low limit.

**46.** a low pass Faraday cage as claimed in any of claims 40 to 45 and as claimed in any of claims 1 to 24.

**47.** an emitter adapted to generate a medical electromagnetic wave, for use in a treatment of disease and/or a treatment for reducing body weight, the treatment comprising an exposure of a patient to be treated to the medical electromagnetic wave.

**48.** an emitter according to claim 47, for use in the treatment of a disease.

**49.** an emitter according to claim 48, the disease to be treated being a disease which causes and/or requires a reaction of the immune system.

**50.** An emitter according to claim 48 or 49, the disease to be treated being an in inflammatory state, an autoimmune disease, or a cancer.

**51.** An emitter according one of claims 48 to 50, the disease to be treated being an acute myocardial infarction, a cardiomyopathy, or an alcoholic liver disease.

**52.** An emitter according to claim 47, for use in a treatment against rejection of an organ transplant.

**53.** An emitter according to claim 50, the disease to be treated being cancer, the treatment comprising at least one exposure of the patient to the medical electromagnetic wave, said exposure lasting less than 24 hours.

**54.** an emitter according to claim 53, the treatment comprising a sequence of repeated exposures of the patient to be treated to the medical electromagnetic wave.

**55.** An emitter according to claim 50, the disease to be treated being an autoimmune disease, the treatment comprising a permanent exposure of the patient to be treated during at least one week to the medical electromagnetic wave.

**56.** An emitter according to claim 55, the power of the medical electromagnetic wave being increased during the treatment.

**57.** An emitter according to claim 50, the disease to be treated being an in inflammation, the treatment comprising at least one exposure of the patient to be treated which last until the symptoms of inflammation disappear and/or become acceptable by the patient.

**58.** an emitter according to any of claims 47 to 50, said medical electromagnetic wave being phase modulated according to a random or a pseudo random sequence of bits.

**59.** an emitter according to any of claims 47 to 58, said medical electromagnetic wave comprising a first part extending from a first lower frequency limit to a first upper frequency limit and having a spectral power superior to the thermal spectral power between the first lower and first upper frequency limits.

**60.** an emitter according to claim 59, a first bandwidth extending from the first lower frequency limit to the first upper frequency limit being at least 10 MHz.

**61.** an emitter according to claim 60, a first bandwidth extending from the first lower frequency limit to the first upper frequency limit being at least 20 MHz.

**62.** an emitter according to any of claims 59 to 61, having a substantially constant first spectral power between said first lower frequency limit and said upper frequency limit.

**63.** an emitter according to any of claims 59 to 62, said medical electromagnetic wave being substantially null for frequencies below the first lower frequency limit and above the first upper frequency limit.

**64.** an emitter according to claim 59, said electromagnetic wave comprising a second part extending from a second lower frequency limit to a second upper frequency limit and having a substantially constant second spectral power between the second lower frequency limit and the second upper frequency limit, said second spectral power being different from said first spectral power.

**65.** an emitter according to any of claims 59 to 63, said first part of the medical electromagnetic wave being a Gaussian or pseudo gaussian noise.

**66.** an emitter according to any of claims 59 to 65, said emitter being adapted to allow an operator to modify the first lower frequency limit and/or the first upper frequency limit.

**67.** an emitter according to any of claims 59 to 66, said emitter being adapted to allow an operator to modify the power of said first part of the medical electromagnetic wave.

**68.** an emitter according to any of claims 47 to 67, said emitter being adapted to generate at least a first medical electromagnetic wave having a low bandwidth and a second medical electromagnetic wave having a bandwidth larger than the low bandwidth.

**69.** an emitter according to claim 68, the treatment comprising at least an exposure to the low bandwidth medical electromagnetic wave and an exposure to the larger bandwidth electromagnetic wave.

70. an emitter according to any of claims 59 to 67, said emitter being programmable to turn on and off said first part of the medical electromagnetic wave according to a predefined time sequence.

71. an emitter according to claim 70, said predefined time sequence lasting for at least a week.

72. an emitter according to any of claims 59 to 71, the treatment comprising a step of determining the first lower frequency limit and/or first upper frequency limit and/or first spectral power and/or time sequence of the medical electromagnetic wave as a function of the age of the patient to be treated.

73. an emitter according to any of claims 59 to 72, the first lower frequency limit and/or first upper frequency limit and/or first spectral power and/or time sequence of the medical electromagnetic wave having been optimized by tests carried out on animals.

74. an emitter according to any of claims 59 to 73, the treatment comprising a diagnostics step to identify the disease to be treated (including cancer location and/or type of autoimmune disease) and/or the HLA system of the patient and/or the age of the patient, and a treatment step in which the medical electromagnetic wave is emitted towards the patient to be treated, wherein the first lower frequency limit and/or first upper frequency limit and/or first spectral power and/or time sequence depend on the identified disease and/or on the HLA system and/or the age of the patient.

75. an emitter according to any of claims 48 to 71, the emitter being adapted to concentrate said medical electromagnetic wave on a region of interest within the body of the patient.

76. an emitter according to claim 75 , the region of interest being a region specifically affected by the disease.

77. an emitter according to claim 112 in combination with claim 52, the region of interest being a transplanted organ.

78. an emitter according to any of claims 47 to 76, comprising an antenna for emitting the electromagnetic wave in open space.

79. an emitter according to claim 75, the emitter comprising an antenna for emitting said medical electromagnetic wave, said antenna being adapted to come into contact with the region of interest of the body of the patient.

80. an emitter according to claim 79, the antenna comprising an inner metallic emitting part surrounded by an isolating layer adapted to come into contact with said region of interest.

81. an emitter according to claim 75, the antenna comprising a conductor adapted to come into direct contact with said region of interest.

82. an emitter according to any of claims 48 to 75, comprising a cathode and an anode, adapted to circulate an electric current from the cathode to the anode through the body.

83. a set comprising an emitter according to claim 82 and a conducting gel, for applying the conducting gel between the body and the electrodes, for improving the contact reproducibility.

84. an emitter according to any of claims 75 to 83, comprising means for attaching the emitter to the body of a patient, to allow the patient to move without interrupting the treatment.

85. an emitter according to any of claims 47 to 113, adapted to generate an electric field having components on three mutually orthogonal directions which are are non-zero and are independent from each other.

86. an emitter according to claim 85, the components of the electric field on three mutually orthogonal directions being mutually independent gaussian noises.

87. an emitter according to any of claims 85 to 86 , the components of the electric field on three mutually orthogonal directions in a place where the patient is allowed to stay having the same time-averaged spectral power.

88. an emitter according to any of claims 85 to 87 , the components of the electric field on three mutually orthogonal directions being simultaneously turned on and off.

89. a jammer for use in reducing the incidence and/or seriousness of diseases caused by a time-varying artificial electromagnetic wave in a Protected Area.

90. a jammer as claimed in claim 89, for use in reducing the incidence and/or seriousness of mental diseases in the Protected Area.

91. a jammer as claimed in claim 89, for use in reducing the incidence and/or seriousness of mental diseases of patients younger than 45 years in the Protected Area.

92. a jammer as claimed in claim 94, for use in reducing the incidence and/or seriousness of depressive disorder of patients younger than 45 years in the Protected Area.

93. a jammer as claimed in claim 89, for use in reducing the incidence and/or seriousness of Creutzfeldt-Jacob disease, multiple sclerosis and/or other auto-immune diseases in the Protected Area.

94. a jammer as claimed in any of claims 89 to 93, adapted to generate a jamming signal having a power spectrum which is constant over time.

95. a jammer as claimed in any of claims 89 to 94, wherein the jammer emits a jamming signal occupying the same frequencies as the time-variable artificial electromagnetic wave.

96. a jammer as claimed in claim 95, the jamming signal having a power as measured on each frequency occupied by the time-variable artificial electromagnetic wave, in W/m2 in a Protected Area, which is to be protected from the pro-auto-immune effects of the time-varying signal, equal to at least 1/10th the peak power of the time-variable signal on the same frequency.

97. a jammer as claimed in claim 96, the power spectrum value of the jamming signal in the Protected Area on any frequency occupied by the time-variable artificial electromagnetic wave being at least equal to the peak power spectrum value of the time-variable signal on the same frequency.

98. a jammer as claimed in any of claims 89 to 97, arranged to generate signals which when reaching the Protected Area have substantially the same directions and/or polarizations as the time-variable artificial electromagnetic wave.

99. a jammer as claimed in any of claims 95 to 98, the time-variable artificial electromagnetic wave being GSM uplink and downlink emissions.

100. the jammer of claim 99, emitting a white noise covering all GSM uplink frequencies and a white noise covering all GSM downlink frequencies.

101. an installation for the treatment of depressive disorder and/or auto-immune diseases, comprising a building adapted to receive patients suffering from depressive disorders and/or auto-immune diseases and at least one jammer adapted to protect the building from the auto-immune effects of at least one time-variable artificial electromagnetic wave.

102. An installation for use in the treatment of disease and/or a treatment for reducing body weight, comprising a main Faraday cage according to any of claims 1 to 38 and an emitter according to any of claims 47 to 83.

103. An installation as claimed in claim 102, wherein:

the emitter and the Faraday cage are arranged so that the medical electromagnetic wave is present inside the Faraday cage,
the Faraday cage is adapted to shield the patient to be treated from at least certain artificial electromagnetic waves not emitted by the emitter, and/or to shield the outside of the Faraday cage from the medical electromagnetic wave.

104. an installation according to claim 103, comprising an absorber of the electromagnetic waves emitted by the emitter, said absorber being enclosed in the main Faraday cage, to avoid excessive build-up of electromagnetic fields inside the main Faraday cage.

**105.** an installation according to claim 104, wherein the emitter is enclosed into an absorbing cage, the absorbing cage being itself enclosed in the main Faraday cage, to best avoid field inhomogeneities inside the absorbing cage.

**106.** an installation according to any of claims 102 to 105, the emitter comprising an antenna and a signal generator assembly for generating a signal to be fed to the antenna, the signal generator assembly being enclosed in an intermediate Faraday cage having an opening through which a conductor passes to connect the signal generator to the antenna outside the intermediate Faraday cage, the intermediate Faraday cage and the main Faraday cage being arranged so that the inner part of the main Faraday cage remains electromagnetically isolated from the outside environment and, except through the antenna, from the devices inside the intermediate Faraday cage.

**107.** an installation according to claim 106, the signal generator assembly comprising a signal generator and a battery to power the signal generator.

**108.** an installation according to any of claims 106 to 107, comprising an absorber electromagnetic waves enclosed in the intermediate Faraday cage.

**109.** an installation according to any of claims 106 to 109, comprising a filter between the signal generator and the antenna, for avoiding leaks of unwanted frequencies into the main Faraday cage through the antenna.

**110.** an installation according to any of claims 103 to 109, further comprising a secondary Faraday cage separate from the main Faraday cage, for allowing patients to stay in the secondary Faraday cage whilst coming into the main Faraday cage from time to time to undergo exposure sequences.

**111.** an installation according to any of claims 102 to 110, adapted to obtain a substantially constant power of irradiationby said electromagnetic wave in a treatment area where a patient can stay.

**112.** an installation according to claim 111, comprising means for preventing a patient to leave the treatment area and reach areas of higher exposure.

**113.** an installation according to claim 112 the means for preventing being a physical barrier separating an area where a patient can stay from an antenna adapted to emit the medical electromagnetic wave.

**114.** an installation according to claim 113 the means for preventing being a wall separating an area where a patient can stay from an antenna adapted to emit the medical electromagnetic wave, said wall being adapted to let the electromagnetic waves emitted from the antenna pass through the wall.

**115.** an installation according to any of claims 102 to 114, adapted to concentrate irradiation power on an area of interest in the body of a patient.

**116.** an installation according to any of claims 102 to 114, wherein the emitter is used to generate an outdoor simulation signal which mimicks the time-averaged spectral power of the artificial electromagnetic waves present in the normal environment of a patient, but which is kept substantially constant over time.

**117.** an installation as claimed in claim 116, for use in the treatment of depressive disorder in patients aged less than 45 years.

**118.** an installation as claimed in claim 116, for use in the treatment of an auto-immune disease.

**119.** an installation according to any of claims 116 to 118, the outdoor simulation signal being a gaussian noise and the spectral power of this gaussian noise being equal to the time-averaged power of the artificial electromagnetic waves present in the patient's environment.

**120.** a device comprising a Faraday cage and an emitter of electromagnetic waves, wherein:

the Faraday cage is adapted to receive a patient to be treated inside the Faraday cage,
the emitter is adapted to emit a medical electromagnetic wave,
the emitter and the Faraday cage are arranged to that the medical electromagnetic wave is present inside the Faraday cage,

the Faraday cage is adapted to shield the patient to be treated from at least certain artificial electromagnetic waves not emitted by the emitter, and/or to shield the outside of the Faraday cage from the medical electromagnetic wave.

121. a device as claimed in claim 120, the medical electromagnetic wave being adapted to interact with the immune system.

122. a device as claimed in any of claims 120 to 121, the Faraday cage being adapted to shield the outside of the Faraday cage from the medical electromagnetic wave.

123. a device as claimed in claim 122, wherein the power of the medical electromagnetic wave is attenuated by at least 20 dB outside the Faraday cage as compared to a place adapted to receive the patient to be treated inside the Faraday cage.

124. a device as claimed in any of claims 120 to 123, further comprising an absorber of electromagnetic waves enclosed inside the Faraday cage.

125. a device as claimed in claim 124, the emitter, the Faraday cage and the absorber being arranged so that the power of the medical electromagnetic wave is substantially spatially constant in a volume adapted to receive the patient to be treated.

126. The device claimed in any of claims 120 to 125, the medical electromagnetic wave being adapted to treat a disease.

127. The device claimed in any of claims 120 to 126, the medical electromagnetic wave being adapted to treat cancer.

128. The device claimed in any of claims 120 to 126, the medical electromagnetic wave being adapted to treat an auto-immune disease.

129. The device claimed in any of claims 120 to 126, the medical electromagnetic wave being adapted to prevent the occurrence of an auto-immune disease during a stay in the Faraday cage.

130. a device as claimed in any of claims 120 to 125, wherein the emitter of electromagnetic waves and the medical electromagnetic wave are as claimed in any of claims 47 to 83.

131. a device as claimed in any of claims 120 to 125, the Faraday cage being a Faraday cage as claimed in any of claims 1 to 38.

132. A method for telecommunicating comprising a step of emitting an artificial electromagnetic wave reaching an exposed area exposed to said artificial electromagnetic wave, wherein a person living in said exposed area suffers from an exposure-related disease which is one of the following:

(a) an infectious disease which is not a sleep disturbance caused by cyst-forming spirochaetes, and/ or
(b) a cancer, and/or
(c) asthma,

wherein said artificial electromagnetic wave is an electromagnetic wave which causes and/or worsens the exposure-related disease and/or wherein the suppression of said artificial electromagnetic wave improves the ability of the immune system to fight said exposure-related disease, and
wherein said method is **characterized by** the fact that it comprises administering to said person a stay in a Faraday cage.

133. The method of claim 1, wherein said artificial electromagnetic wave is an electromagnetic wave which causes and/or worsens the exposure-related disease.

134. The method as claimed in any of claims 1 to 133, for telecommunicating by FM radio broadcasting and/or analog or digital video broadcasting and/or cellular telephony communications and/or Wi-fi.

135. The method as claimed in any of claims 1 to 133, for telecommunicating by FM radio broadcasting and/or analog or digital video broadcasting and/or GSM and/or UMTS and/or Wifi.

136. The method as claimed in any of claims 132 to 135, wherein said exposure-related disease is asthma.

137. The method as claimed in any of claims 132 to 135, wherein said exposure-related disease is pneumonia.

138. The method as claimed in claim any of claims 132 to 135, wherein said exposure-related disease is Acquired Immunodeficiency Syndrome (AIDS).

139. The method as claimed in any of claims 132 to 135, wherein said exposure-related disease is caused by a cyst-forming spirochaete.

140. The method as claimed in any of claims 132 to 139 wherein said person is not Electro-Hyper-Sensitive.

141. The method as claimed in any of claims 132 to 139 wherein said person does not suffer from a sleep disturbance.

142. A method for telecommunicating comprising a step of emitting an artificial electromagnetic wave reaching an exposed area exposed to said artificial electromagnetic wave, wherein a person living in said exposed area is at risk of nosocomial infection, wherein said artificial electromagnetic wave is an electromagnetic wave that increases the risk of a nosocomial infection, and
Wherein said method is **characterized by** the fact that it comprises administering to said person a stay in a Faraday cage.

143. A method for telecommunicating comprising emitting an artificial electromagnetic wave having a frequency between 800 and 1000 MHz reaching an exposed area exposed to said artificial electromagnetic wave, wherein a person living in said exposed area is overweight, Wherein said method is **characterized by** the fact that it comprises administering to said person a stay in a Faraday cage.

144. The method of any of claims 132 to 143, comprising administering to said person a plurality of overnight stays in the Faraday cage.

145. The method of any of claims 132 to 143, comprising administering to said person a permanent stay of at least one week in the Faraday cage.

146. The method of any of claims 132 to 145, the Faraday cage having an attenuation of at least 30 dB for the electric field in the 80 MHz to 1000 MHz frequency range.

147. A method for telecommunications comprising emitting a time-varying artificial electromagnetic wave reaching a Protected Area where at least one person lives who suffers from an auto-immune disease and/or a multiple sclerosis and/or Creutzfeldt-Jacob disease, and/or who is younger than 45 years and suffers from a depressive disorder, **characterized by** the fact that said method comprises emitting a jamming signal reaching the Protected Area, wherein the power spectrum of the jamming signal is substantially constant over time, and wherein the jamming signal occupies the same frequencies as the time-variable artificial electromagnetic wave.

148. The method of claim 147, the jamming signal having a power as measured on each frequency occupied by the time-variable artificial electromagnetic wave, in W/m2 in the Protected Area, which is equal to at least 1/10th the peak power of the time-variable signal on the same frequency.

149. The method of any of claims 147 to 148, the jamming signal being provided by a jammer.

150. The method of any of claims 147 to 149, the artificial electromagnetic wave being GSM.

151. The method of any of claims 132 to 150, wherein said person is a hospital patient.

152. A non-therapeutic method for inducing weight loss in a subject, comprising administering the subject a stay in a Faraday cage.

153. The method of any of claims 132 to 152, wherein the Faraday cage has an attenuation of at least 30 dB for the electric field in the 80 MHz to 1000 MHz frequency range.

**154.** A low-pass Faraday cage adapted to attenuate the higher frequencies of the electric field more strongly than the lower frequencies of the electric field.

**155.** The low-pass Faraday cage of claim 154, enclosing a bed and/or adapted to enclose an adult human being.

**156.** The low-pass Faraday cage of any of claims 154, wherein the Faraday cage comprises at least a first and a second conducting elements, wherein a first surface of the first conducting element is situated in front of a second surface of the second conductive element to form a capacitance between the first and a second surface, to allow some low frequency electric field to enter the Faraday cage.

The low-pass Faraday cage of claim 154, adapted so that the resistance measured between any two points of the first conductive element and the resistance measured between any two points of the second conducting element are lower than the resistance measured between any point of the first conducting element and any point of the second conductive element.

**157.** The low-pass Faraday cage of claim 154, wherein a cut-off frequency between the more attenuated high frequencies and the less attenuated low frequencies is adapted to separate high frequency time-varying signals from lower frequency permanent signals.

**158.** The low-pass Faraday cage of claim 157, wherein the cut-off frequency is between a high limit and a low limit, wherein the Faraday cage attenuates frequencies higher than the high limit more strongly than frequencies lower than the low limit.

**159.** The low-pass Faraday cage of claim 158, wherein the high limit is below the frequencies of GSM communication networks, Wifi and DECT.

**160.** The low-pass Faraday cage of any of claims 157 to 159, wherein the low limit is higher than the frequencies of Hertzian Television.

**161.** A low-pass Faraday cage according to any of claims 158 to 160 wherein the low limit is 790 MHz

**162.** A low-pass Faraday cage according to any of claims 158 to 161, wherein the high limit is 900 MHz.

**163.** A low-pass Faraday cage according to any of claims 158 to 162, attenuating the electric field above the high limit by at least 20 dB more than the electric field below the low limit.

**164.** The method according to any of claims 132 to 139 or 152, wherein the Faraday cage is a low-pass Faraday cage according to any of claims 154 to 163 , and wherein the radiofrequency electromagnetic waves comprise cellular telephony electromagnetic waves.

**165.** A surgery room adapted to perform surgical acts and enclosed in a Faraday cage.

**166.** The surgery room of claim 165, comprising an absorber of electromagnetic waves, said absorber being enclosed in the Faraday cage and adapted to absorb electromagnetic waves emitted by medical apparatuses.

**167.** The method of any of claims 132 to 139 , wherein the Faraday cage encloses a surgery room as per claim 165 or 166.

**168.** a device comprising an emitter adapted to emit a first medical electromagnetic wave adapted to cure a disease, wherein said first medical electromagnetic wave comprises a first part extending from a first lower frequency limit to a first upper frequency limit and having a spectral power superior to the thermal spectral power between the first lower and first upper frequency limits, and the first bandwidth extending between the first lower frequency limit and the first upper frequency limit is larger than 10 MHz,
**characterized by** the fact that it comprises a Faraday cage, wherein:

the Faraday cage is adapted to receive a person to be treated inside the Faraday cage,
the emitter and the Faraday cage are arranged to that the medical electromagnetic wave is present inside the Faraday cage, and
the Faraday cage is adapted to shield the outside of the Faraday cage from the medical electromagnetic wave.

**169.** A device as claimed in claim 168, comprising an absorber of electromagnetic waves enclosed inside the Faraday cage.

**170.** A device as claimed in any of claims 168 to 169, the first medical electromagnetic wave having a substantially constant first spectral power between said first lower frequency limit and said upper frequency limit.

**171.** A device as claimed in any of claims 168 to 170, wherein the emitter is enclosed into an absorbing cage, the absorbing cage being itself enclosed in the main Faraday cage.

**172.** A device as claimed in claim 171, wherein the absorbing cage is adapted to receive the person to be treated inside the absorbing cage, and the emitter, the absorbing cage and the Faraday cage are arranged so that the first medical electromagnetic wave is present inside the absorbing cage.

**173.** A device as claimed in any of claims 171 to 172 wherein the absorbing cage forms an anechoic chamber.

**174.** A device as claimed in any of claims 168 to 173, wherein the emitter is further adapted to emit a second electromagnetic wave, wherein the emitter is adapted so that the first and second electromagnetic waves have different propagation directions when reaching the person to be treated and wherein the first and second electromagnetic waves are adapted to not form a stable interference pattern.

**175.** A device as claimed in claim 174, wherein the emitter comprises a first sub-emitter emitting the first electromagnetic wave and a second sub-emitter emitting the second electromagnetic wave.

**176.** A device as claimed in any of claims 174 to 175, wherein the first and second electromagnetic waves are mutually non-correlated noises.

**177.** A device as claimed in any of claims 168 to 173, wherein the emitter is further adapted to emit a third electromagnetic wave, wherein the emitter is adapted so that the first, second and third electromagnetic waves have different propagation directions when reaching the person to be treated and wherein the first, second and third electromagnetic waves are adapted to not form a stable interference pattern.

Figure 1

Figure 2

Figure 3

Figure 4

Figure 5

801

802

800

## Figure 6

Protected
Area

□ :GSM base station

△ :Jammer

## Figure 7

Protected Area

□ :GSM base station

Δ :Jammer

Figure 8

701

□

710

721

730

720

711

700

Figure 9

Figure 10

Figure 11

Europäisches
Patentamt

European
Patent Office

Office européen
des brevets

## EUROPEAN SEARCH REPORT

Application Number

EP 14 16 7813

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/008447 A2 (UNIV CALIFORNIA [US]; COHEN MARK [US]) 17 January 2008 (2008-01-17) * abstract; figures 1-6 * * page 6, line 17 - page 11, line 16 * | 1-15 | INV. A61N1/16 A61N1/06 A61N2/02 |
| X | Gary W Arendash ET AL: "Electromagnetic field treatment protects against and reverses cognitive impairment in Alzheimer's disease mice", Journal of Alzheimer's disease : JAD, 1 January 2010 (2010-01-01), pages 191-210, XP055175638, Netherlands DOI: 10.3233/JAD-2010-1228 Retrieved from the Internet: URL:http://www.ncbi.nlm.nih.gov/pubmed/20061638 * abstract * * paragraphs [Introduction], [EMF_exposure_protocol] * | 1-15 | |
| X | HOMBACH V ET AL: "Noninvasive beat-by-beat registration of ventricular late potentials using high resolution electrocardiography", INTERNATIONAL JOURNAL OF CARDIOLOGY, ELSEVIER SCIENCE PUBLISHERS, AMSTERDAM, NL, vol. 6, no. 2, 1 August 1984 (1984-08-01), pages 167-183, XP026258327, ISSN: 0167-5273, DOI: 10.1016/0167-5273(84)90351-6 [retrieved on 1984-08-01] * abstract; figure 1 * * paragraph [Methods] * | 1-15 | TECHNICAL FIELDS SEARCHED (IPC) A61N |

The present search report has been drawn up for all claims

| Place of search | Date of completion of the search | Examiner |
|---|---|---|
| Munich | 11 March 2015 | Lahorte, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 14 16 7813

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2015

| Patent document cited in search report | Publication date | Patent family member(s) | Publication date |
|---|---|---|---|
| WO 2008008447 A2 | 17-01-2008 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 732276
- US 1962565 A
- US 61889065 B
- WO 732276 A

### Non-patent literature cited in the description

- **G. LAKHOVSKY.** Radiations and Waves, Sources of Our Life. 1941
- **VINCENT LAUER.** *A Quantum Theory of the Biological Effects of Radio-frequencies and its application to Cancer,* November 2013, http://hal.archives-ouvertes.fr/hal-00877298
- **VINCENT LAUER.** A model of the interaction of T lymphocytes with electromagnetic waves. *HAL-009755963,* 09 April 2014, http://hal.archives-ouvertes.fr/hal-00975963
- **R WOLF ; D WOLF.** Increased Incidence of Cancer near a Cell-Phone Transmitter Station. *International Journal of Cancer Prevention,* 2004, vol. 1 (2
- **A M SOMMER ; J STRECKERT ; A K BITZ ; V W HANSEN ; A LERCHL.** No effects of GSM-modulated 900 MHz electromagnetic fields on survival rates and spontaneous development of lymphoma in female AKR/J mice. *BMJ cancer,* 2004
- **A. LERCHL.** Effects of mobile phone electromagnetic fields at nonthermal SAR values on melatonin and body weight of Djungarian hamsters. *Journal of Pineal Research,* 2008
- Einfluss hochfrequenter Felder des Mobilfunks auf die metabolische Umsatzrate in Tiermodell (Labornager). Jacobs-University Bremen
- **A PELLETIER ; S DELANAUD ; P DECIMA ; G THUROCZY ; R DE SEZE ; M CERRI ; V BACH ; J P LIBERT ; N LOOS.** effects of chronic exposure to radiofrequency electromagnetic fields on energy balance in developing rats. *Environmental Science and Pollution Research,* 2013
- **J. KASZUBA-ZWOINSKA.** loss of interstitial cells of Cajal after pulsating electromagnetic fields (pemf) in gastrointestinal tract of the rats. *Journal of Physiology and pharmacology,* vol. 56 (3), 421-432, www.kpp.krakow.pl
- Study of health effects of the shortwave transmitter station of Schwarzenburg. *Technical Report, university of Berne, Switzerland,* 1995
- **LEITGEB.** EMF protection sleep study near mobile phone base stations. *Somnologie,* 2008, vol. 12, 234-243